# EUROPEAN PATENT APPLICATION

(11) **EP 3 323 422 A1**
(43) Date of publication of application: **23.05.2018**
(21) Application number: 16306539.4
(22) Date of filing: 22.11.2016
(51) Int. Cl.: A61K 38/00, C07K 7/08

(54) **NEW D-CONFIGURED CATESLYTIN PEPTIDE**

(71) Applicant: Université de Strasbourg, 67000 Strasbourg (FR); Institut National de la Santé et de la Recherche Médicale, 75013 Paris (FR)
(72) Inventor: MARBAN, Céline, 68000 COLMAR (FR); METZ-BOUTIGUE, Marie-Hélène, 67000 STRASBOURG (FR); LAVALLE, Philippe, 67370 WINTZENHEIM KOCHERSBERG (FR); SCHAAF, Pierre, 67120 MOLSHEIM (FR); HAIKEL, Youssef, 67000 STRASBOURG (FR)
(74) Representative: Cabinet Becker et Associés

(57) **Abstract**

The present invention relates to a cateslytin peptide having an amino acid sequence consisting or consisting essentially in the sequence of SEQ ID NO: 1, wherein at least 80%, preferably at least 90%, of the amino acids residues of said cateslytin are D-configured. The invention also relates to the use of said cateslytin peptide as a drug, especially in the treatment of an infection in a patient in needs thereof.

SEQUENCE LISTING

<110>  Université de Strasbourg
       INSERM

<120>  New D-configured Cateslytin peptide

<130>  B2368EP

<160>  8

<170>  PatentIn version 3.5

<210>  1
<211>  15
<212>  PRT
<213>  Bos taurus

<400>  1

Arg Ser Met Arg Leu Ser Phe Arg Ala Arg Gly Tyr Gly Phe Arg
1               5                   10                  15


<210>  2
<211>  15
<212>  PRT
<213>  Homo sapiens

<400>  2

Ser Ser Met Lys Leu Ser Phe Arg Ala Arg Gly Tyr Gly Phe Arg
1               5                   10                  15


<210>  3
<211>  15
<212>  PRT
<213>  Artificial Sequence

<220>
<223>  Peptide

<400>  3

Ser Ser Met Lys Leu Ser Phe Arg Ala Arg Ala Tyr Gly Phe Arg
1               5                   10                  15


<210>  4
<211>  15
<212>  PRT
<213>  Artificial Sequence

<220>
<223>  Peptide

<400>  4

Arg Ser Met Lys Leu Ser Phe Arg Ala Arg Ala Tyr Gly Phe Arg
1               5                   10                  15

<210>  5
<211>  15
<212>  PRT
<213>  Artificial Sequence

<220>
<223>  Peptide

<400>  5

Arg Ser Met Lys Leu Ser Phe Arg Thr Arg Ala Tyr Gly Phe Arg
1               5                   10                  15


<210>  6
<211>  15
<212>  PRT
<213>  Artificial Sequence

<220>
<223>  Peptide

<400>  6

Arg Ser Met Lys Leu Ser Phe Arg Ala Pro Ala Tyr Gly Phe Arg
1               5                   10                  15


<210>  7
<211>  15
<212>  PRT
<213>  Artificial Sequence

<220>
<223>  Peptide


<220>
<221>  R or S
<222>  (1)..(1)

<220>
<221>  positively charged amino acid, preferably R or K
<222>  (4)..(4)

<220>
<221>  T, S or A
<222>  (9)..(9)

<220>
<221>  P, K or R
<222>  (10)..(10)

<220>
<221>  very small amino acid, preferably G or A
<222>  (11)..(11)

<400>  7

Xaa Ser Met Xaa Leu Ser Phe Arg Xaa Xaa Xaa Tyr Gly Phe Arg
1               5                   10                  15

<210>  8
<211>  15
<212>  PRT
<213>  Artificial Sequence

<220>
<223>  Peptide



<220>
<221>  R or S
<222>  (1)..(1)

<220>
<221>  positively charged amino acid, preferably R or K
<222>  (4)..(4)

<220>
<221>  very small amino acid, preferably G or A
<222>  (11)..(11)

<400>  8

Xaa Ser Met Xaa Leu Ser Phe Arg Ala Arg Xaa Tyr Gly Phe Arg
1               5                   10                  15

## Description

### Field of the Invention

The present invention relates to the field of medicine, in particular of infections. It provides new treatments against infections, in particular against bacterial and fungal infections.

### Background of the Invention

Infections represent one of the main healthcare challenge of the 21st century as they resulted in 9.2 million deaths in 2013 (about 17% of all deaths). Infections consist in the invasion of an organism's body tissues by disease-causing agents, their multiplication, and the reaction of host tissues to these organisms and the toxins they produce. These disease-causing agents can be as diverse as bacteria, viruses, fungi and parasites.

In the domain of bacterial infections, the discovery of antibiotics to treat infections is one of the greatest achievements of modern medicine. However, excessive and inappropriate use of antibiotics fosters the emergence and spread of antibiotic-resistant microorganisms. Indeed, infections caused by antibiotic-resistant microorganisms also known as "superbugs" often no longer respond to conventional treatments, thereby extending the duration of the disease related to infection and even lead to patient death. In addition, the discovery of fluoroquinolones in the 1970s brought to an end the portfolio of antibiotics against Gram-negative bacteria. Over the past 25 years, no new classes of antibiotics have been discovered. Specifically due to this antibiotic resistance phenomenon and the lack of discovery of new antibiotic classes, humanity is now facing the possibility of a future without effective antibiotics for treating bacterial infections. The problem is so serious that it threatens the achievements of modern medicine. A post-antibiotic era in which common infections and minor injuries can kill is a very real possibility for the 21st century.

The situation is very similar in the domain of fungal infections. Indeed, the excessive use of antifungal agents, compounded by the shortage of new drugs put on the market, is causing the accumulation of multi-resistance phenotypes in many fungal strains. Infections caused by these resistant microorganisms often no longer respond to conventional treatments, therefore lengthening the duration of illness related to the infection. Moreover, the widespread use of antifungal agents in clinics and hospitals promotes the development and spread of antifungal-resistant strains and thus the occurrence of nosocomial infections.

Over the last decade, host defense peptides (HDPs) have emerged as a promising alternative for treatment of bacterial and other microbial infections. Naturally occurring HDPs, also named antimicrobial peptides, constitute an exciting class of drug candidates. HDPs are usually rather small peptides (10-40 amino acids), cationic and amphiphilic with a broad diversity in their secondary structure and well preserved during evolution. They are naturally present in tissues frequently exposed to pathogens, such as the skin, lungs, and gastrointestinal tract.

They display an unusually broad spectrum of activity against pathogens including bacteria, viruses, fungi and parasites (Hancock RE et al, 2006, Nat Biotechnol., 24(12): 1551-1557). Mammalian HDPs represent an important component of the innate immune system as they can trigger both direct microbe killing by disrupting the pathogens membranes and rapid immune response modulation (Metz-Boutigue MH et al, 2003, Trends Microbiol, 11(12): 585-592; Zasloff M, 2002, Nature, 415(6870): 389-395). Moreover, HDPs act quickly and in a non-specific manner, therefore bacteria are not prone to develop high-level resistance towards these compounds in the same extent as towards conventional antibiotics.

Despite the great threat that constitute the increasing development of antibiotic and antifungal resistances, there is still nowadays no real alternative treatment to conventional antibiotics or antifungal agents on the market. Thus, there is a persisting and urgent medical need to develop new antibacterial agents and new antifungal agents capable to efficiently overcome pathogens resistance. The present invention seeks to meet these and other needs.

### Summary of the Invention

Among all isolated and characterized HDPs, peptides generated from the endogenous processing of chromogranin A are of particular interest. Chromogranin A (CGA) is an acidic glyco-phosphoprotein stored in the secretory vesicles of numerous nervous, neuroendocrine and immune cells and released upon stress in most of the body fluids (Taupenot L, 2003, N. Engl. J. Med., 348: 1134-1149). CgA is known to be a precursor of several biological active peptides. Among these peptides several correspond to short linear HDPs (less than 25 residues) and are therefore very easy to synthesize for a minimal cost. Moreover, they are stable in a wide range of temperature and pH (Helle KB et al, 2007, Cell. Mol. Life Sci., 64: 2863-2886) and are not toxic for host cells. Among all isolated and characterized HDPs, cateslytin (CTL) constitutes an interesting candidate as it is very small (15 amino acids) and therefore very easy to synthesize for a minimal cost. In addition, cateslytin is also a potent antibacterial and antifungal agent (Aslam R et al, 2013, PLoS One, 8(7):e68993 ; Briolat J et al, 2005, Cell Mol Life Sci, 62(3):377-85).

The inventors have surprisingly discovered that a cateslytin peptide with D-configured amino acids residues (D-CTL) is a much potent antibiotic and antifungal agent than its natural counterpart (L-CTL). Moreover, D-CTL is not cytotoxic or immunogenic and is very stable at high temperature and acidic pH. Altogether, the inventors have thus discovered a new molecule for the development of a very promising new class of antibiotics and antifungal drugs.

Accordingly, in a first aspect, the present invention concerns a cateslytin peptide having an amino acid sequence consisting or consisting essentially in the sequence of SEQ ID NO: 1, wherein at least 60%, preferably at least 80%, of the amino acids residues of said cateslytin are D-configured.

Preferably, all the amino acids residues of said cateslytin are D-configured.

In a second aspect, the invention also concerns the cateslytin peptide according to the invention for use as a drug.

The invention also concerns, in a third aspect, a pharmaceutical composition comprising or consisting essentially in a cateslytin peptide according to the invention. Preferably, the pharmaceutical composition according to the invention further comprises an additional active ingredient selected from the group consisting in an antibiotic, an antifungal, an antiviral, an antiparasitic and a combination thereof.

In a fourth aspect, the invention still concerns a product or kit comprising a) a cateslytin peptide according to the invention and b) an active ingredient selected from the group consisting in an antibiotic, an antifungal, an antiviral, an antiparasitic and a combination thereof, as a combined preparation for simultaneous, separate or sequential use.

The invention yet concerns, in a fifth aspect, the cateslytin peptide according to the invention, the pharmaceutical composition according to the invention, or the product or kit according to the invention for use in the treatment of an infection, preferably an infection selected from the group consisting in bacterial, viral, parasite and fungal infections, even more preferably a bacterial infection.

In a preferred embodiment, the infection is selected from the group consisting in fibrosis, meningitis, skin infections such as acne, intestinal infections such as esophagitis, gastritis, enteritis, colitis, sigmoiditis, rectitis, and peritonitis, urinary tract infections, vaginal infections, female upper genital tract infections such as salpingitis, endometritis, oophoritis, myometritis, parametritis and infection in the pelvic peritoneum, respiratory tract infections such as pneumonia, intra-amniotic infections, odontogenic infections, endodontic infections, bloodstream infections, or a combination thereof.

Preferably, the infection is a nosocomial infection, more preferably a nosocomial infection selected from the group consisting in catheter-related infections, hospital acquired pneumonia such as ventilator associated pneumonia, post-partum infection, hospital acquired gastroenteritis, hospital acquired urinary tract infections, or a combination thereof, even more preferably the nosocomial infection is a catheter-related infection.

In another preferred embodiment, the infection is a fungal infection, more preferably a fungal infection selected from the group consisting in aspergillosis, blastomycosis, candidiasis, coccidioidomycosis, cryptococcosis, histoplasmosis, ucormycosis, paracoccidioidomycosis, sporotrichosis, pneumocystis, and a mixture thereof, still more preferably the fungal infection is a candidiasis, even more preferably an oral candidiasis.

Preferably, the fungal infection is caused by a pathogen selected from the group consisting in *Candida* species such as *Candida albicans, Candida parapsilosis, Candida tropicalis, Candida krusei, Candida guillermondii, Candida rugosa, Candida dubliniensis, Candida auris, Candida glabrata, Candida lusitaniae, Candida kefyr, Candida famata, Candida inconspicua,* and *Candida norvegensis*; *Aspergillus species* such as *Aspergillus fumigatus, Aspergillus clavatus,* and *Aspergillus flavus; Cryptococcus* species such as *Cryptococcus neoformans* and *Cryptococcus gattii; Histoplasma* species such as *Histoplasma capsulatum*; *Pneumocystis* species such as *Pneumocystis jirovecii* and *Pneumocystis carinii*; *Stachybotrys* species such as *Stachybotrys chartarum*; *Blastomyces* species such as *Blastomyces dermatitidis; Coccidioides* species such as *Coccidioides immitis and Coccidioides posadasii; Mucorales* species such as *Rhizopus oryzae, Rhizomucor, Absidia, Lichtheimia corymbifera, Syncephalastrum racemosum, Apophysomyces variabilis* and *Mucor indicus; Paracoccidioides* species such as *Paracoccidioides brasiliensis; Sporothrix* species such as *Sporothrix schenckii*; *Epidermophyton* species such as *Epidermophyton floccosum; Microsporum* species such as *Microsporum canis* and *Microsporum audouinii*; *Trichophyton* species such as *Trichophyton interdigitale* (or *mentagrophytes*), *Trichophyton tonsurans, Trichophyton schoenleini, Trichophyton rubrum,* and *Trichophyton verrucosum*; *Hortaea* species such as *Hortaea werneckii*; *Penicillium* species such as *Penicillium marneffei*; *Piedraia* species such as *Penicillium hortae*; *Malassezia* species such as *Malassezia furfur; Lacazia* species such as *Lacazia loboi*; *Exophiala* species such as *Exophiala jeanselmei*; *Fonsecaea* species such as *Fonsecaea pedrosoi* and *Fonsecaea compacta*; *Phialophora* species such as *Phialophora verrucosa; Basidiobolus* species such as *Basidiobolus ranarum*; *Conidiobolus* species such as *Conidiobolus coronatus* and *Conidiobolus incongruus; Enterocytozoon* species such as *Enterocytozoon bieneusi* and *Encephalitozoon intestinalis*; *Rhinosporidium* species such as *Rhinosporidium seeberi; Geotrichum* species such as *Geotrichum candidum ; Pseudallescheria* species such as *Pseudallescheria boydii; Trichosporon* species; *Torulopsis glabrata,* or a mixture thereof.

More preferably the fungal infection is caused by a *Candida species* selected from the group consisting in *Candida albicans, Candida parapsilosis, Candida tropicalis, Candida krusei, Candida guillermondii, Candida rugosa, Candida dubliniensis, Candida auris, Candida glabrata, Candida lusitaniae, Candida kefyr, Candida famata, Candida inconspicua,* and *Candida norvegensis.*

Even more preferably the fungal infection is caused by *Candida albicans.*

In yet another preferred embodiment, the infection is a bacterial infection. Preferably, the bacterial infection is caused by a bacteria selected from the group consisting in *Escherichia coli, Escherichia coli Amp^{R} Kan^{R} Chlo^{R}, Escherichia coli AmpC, Escherichia coli ESBL, Escherichia coli OXA48, Staphylococcus aureus MSSA, Staphylococcus aureus MRSA, Klebsiella pneumoniae, Klebsiella pneumoniae ESBL, Klebsiella pneumoniae KPC, Enterobacter cloacae, Enterobacter cloacae ESBL, Enterobacter cloacae AmpC, Enterobacter cloacae OXA48, Enterobacter aerogenes, Enterobacter aerogenes ESBL, Enterobacter aerogenes AmpC, Serratia marcescens, Serratia marcescens AmpC, Morganella morganii, Morganella morganii AmpC, Citrobacter freundii, Citrobacter freundii AmpC, Pseudomonas aerigunosa, Pseudomonas aerigunosa AmpC, Pseudomonas aerigunosa VIM, Parvimonas micra, Prevotella intermedia, Fusobacterium nucleatum, Enterococcus faecalis, Prevotella nigrescens, Actinomyces israelii, Porphyromonas endodontalis, Porphyromonas gingivalis Micrococcus luteus, Bacillus megaterium, Actinomyces israelii, Aeromonas hydrophila, Aeromonas caviae, Bacillus anthracis, Bacillus cereus, Bacteroides fragilis, Bartonella henselae, Bartonella Quintana, Bordetella pertussis, Borrelia burgdorferi, Borrelia garinii, Borrelia afzelii, Borrelia recurrentis, Brucella abortus, Brucella canis, Brucella melitensis, Brucella suis, Campylobacter jejuni, Campylobacter coli, Campylobacter fetus, Chlamydia pneumoniae, Chlamydia trachomatis, Chlamydophila psittaci, Clostridium botulinum, Clostridium difficile, Clostridium perfringens, Clostridium tetani, Corynebacterium diphtheria, Ehrlichia canis, Ehrlichia chaffeensis, Enterococcus faecium, Francisella tularensis, Haemophilus influenza, Helicobacter pylori, Legionella pneumophila, Leptospira interrogans, Leptospira santarosai, Leptospira weilii, Leptospira noguchii, Listeria monocytogenes, Mycobacterium leprae, Mycobacterium tuberculosis, Mycobacterium ulcerans, Mycoplasma pneumonia, Neisseria gonorrhoeae, Neisseria meningitides, Nocardia asteroids, Rickettsia rickettsia, Salmonella enteritidis, Salmonella typhi, Salmonella paratyphi, Salmonella typhimurium, Shigella sonnei, Shigella flexnerii, Shigella dysenteriae, Staphylococcus epidermidis, Staphylococcus saprophyticus, Streptococcus agalactiae, Streptococcus pneumoniae, Streptococcus pyogenes, Streptococcus viridans, Treponema pallidum, Ureaplasma urealyticum, Vibrio cholera, Vibrio parahaemolyticus, Yersinia pestis, Yersinia enterocolitica, Yersinia pseudotuberculosis,* and combination thereof.

More preferably the bacterial infection is caused by a bacteria selected from the group consisting in *Escherichia coli, Escherichia coli Amp^{R} Kan^{R} Chlo^{R}, Escherichia coli AmpC, Escherichia coli ESBL, Escherichia coli OXA48, Staphylococcus aureus MSSA, Staphylococcus aureus MRSA, Klebsiella pneumoniae, Klebsiella pneumoniae ESBL, Klebsiella pneumoniae KPC, Enterobacter cloacae, Enterobacter cloacae ESBL, Enterobacter cloacae AmpC, Enterobacter cloacae OXA48, Enterobacter aerogenes, Enterobacter aerogenes ESBL, Enterobacter aerogenes AmpC, Serratia marcescens, Serratia marcescens AmpC, Morganella morganii, Morganella morganii AmpC, Citrobacter freundii, Citrobacter freundii AmpC, Pseudomonas aerigunosa, Pseudomonas aerigunosa AmpC, Pseudomonas aerigunosa VIM, Parvimonas micra, Prevotella intermedia, Fusobacterium nucleatum, Enterococcus faecalis* and combination thereof.

Even more preferably the bacterial infection is caused by a bacteria selected from the group consisting in *Escherichia coli, Escherichia coli Amp^{R} Kan^{R} Chlo^{R}, MSSA Staphylococcus aureus, MRSA Staphylococcus aureus, Parvimonas micra, Prevotella intermedia, Fusobacterium nucleatum, Enterococcus faecalis,* and combination thereof.

In a most preffered embodiment, the bacterial infection is caused by the bacteria *Escherichia coli or Escherichia coli Amp^{R} Kan^{R} Chlo^{R}.*

Preferably, the antifungal according to the invention is selected from the group consisting in polyenes such as amphotericin B, candicidin, filipin, hamycin, natamycin, nystatin and rimocidin; imidazoles such as bifonazole, butoconazole, clotrimazole, econazole, fenticonazole, isoconazole, ketoconazole, luliconazole, miconazole, omoconazole, oxiconazole, sertaconazole, sulconazole, tioconazole, and spectrazole; triazolles such as albaconazole, efinaconazole, epoxiconazole, fluconazole, isavuconazole, itraconazole, posaconazole, propiconazole, ravuconazole, terconazole, and voriconazole; thiazoles such as abafungin; allylamines such as amorolfin, butenafine, naftifine, and terbinafine; echinocandins such as anidulafungin, caspofungin, and micafungin; benzoic acid, ciclopirox, flucytosine, 5-fluorocytosine, griseofulvin, haloprogin, tolnaftate, undecylenic acid, derivatives and combinations thereof.

More preferably the antifungal according to the invention is selected from the group consisting in triazolles such as albaconazole, efinaconazole, epoxiconazole, fluconazole, isavuconazole, itraconazole, posaconazole, propiconazole, ravuconazole, terconazole, and voriconazole.

Still preferably the antifungal is the voriconazole or the fluconazole. Even more preferably the antifungal is the voriconazole.

Preferably, the antibiotic according to the invention is selected from the group consisting in penicillins such as penicillin G, penicillin K, penicillin N, penicillin O, penicillin V, methicillin, benzylpenicillin, nafcillin, oxacillin, cloxacillin, dicloxacillin, ampicillin, amoxicillin, pivampicillin, hetacillin, bacampicillin, metampicillin, talampicillin, epicillin, carbenicillin, ticarcillin, temocillin, mezlocillin, and piperacillin; cephalosporins such as cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefonicid, cefprozil, cefuroxime, cefuzonam, cefmetazole, cefotetan, cefoxitin, loracarbef, cefbuperazone, cefminox, cefotetan, cefoxitin, cefotiam, cefcapene, cefdaloxime, cefdinir, cefditoren, cefetamet, cefixime, cefmenoxime, cefodizime, cefotaxime, cefovecin, cefpimizole, cefpodoxime, cefteram, ceftamere, ceftibuten, ceftiofur, ceftiolene, ceftizoxime, ceftriaxone, cefoperazone, ceftazidime, latamoxef, cefclidine, cefepime, cefluprenam, cefoselis, cefozopran, cefpirome, cefquinome, flomoxef, ceftobiprole, ceftaroline, ceftolozane, cefaloram, cefaparole, cefcanel, cefedrolor, cefempidone, cefetrizole, cefivitril, cefmatilen, cefmepidium, cefoxazole, cefrotil, cefsumide, ceftioxide, cefuracetime, and nitrocefin; polymyxins such as polysporin, Neosporin,polymyxin B, and polymyxin E, rifampicins such as rifampicin, rifapentine, and rifaximin; Fidaxomicin; quinolones such as cinoxacin, nalidixic acid, oxolinic acid, piromidic acid, pipemidic acid, rosoxacin, ciprofloxacin, enoxacin, fleroxacin, lomefloxacin, nadifloxacin, norfloxacin, ofloxacin, pefloxacin, rufloxacin, balofloxacin, grepafloxacin, levofloxacin, pazufloxacin, temafloxacin, tosufloxacin, clinafloxacin, gatifloxacin, gemifloxacin, moxifloxacin, sitafloxacin, trovafloxacin, prulifloxacin, delafloxacin, nemonoxacin, and zabofloxacin; sulfonamides such as sulfafurazole, sulfacetamide, sulfadiazine, sulfadimidine, sulfafurazole, sulfisomidine, sulfadoxine, sulfamethoxazole, sulfamoxole, sulfanitran, sulfadimethoxine, sulfametho-xypyridazine, sulfametoxydiazine, sulfadoxine, sulfametopyrazine, and terephtyl; macrolides such as azithromycin, clarithromycin, erythromycin, fidaxomicin, telithromycin, carbomycin A, josamycin, kitasamycin, midecamycin, oleandomycin, solithromycin, spiramycin, troleandomycin, tylosin, and roxithromycin; ketolides such as telithromycin, and cethromycin; lluoroketolides such as solithromycin; lincosamides such as lincomycin, clindamycin, and pirlimycin; tetracyclines such as demeclocycline, doxycycline, minocycline, oxytetracycline, and tetracycline; aminoglycosides such as amikacin, dibekacin, gentamicin, kanamycin, neomycin, netilmicin, sisomicin, tobramycin, paromomycin, and streptomycin; ansamycins such as geldanamycin, herbimycin, and rifaximin; carbacephems such as loracarbef; carbapenems such as ertapenem, doripenem, imipenem (or cilastatin), and meropenem; glycopeptides such as teicoplanin, vancomycin, telavancin, dalbavancin, and oritavancin; lincosamides such as clindamycin and lincomycin; lipopeptides such as daptomycin; monobactams such as aztreonam; nitrofurans such as furazolidone, and nitrofurantoin; oxazolidinones such as linezolid, posizolid, radezolid, and torezolid; teixobactin, clofazimine, dapsone, capreomycin, cycloserine, ethambutol, ethionamide, isoniazid, pyrazinamide, rifabutin, arsphenamine, chloramphenicol, fosfomycin, fusidic acid, metronidazole, mupirocin, platensimycin, quinupristin (or dalfopristin), thiamphenicol, tigecycline, tinidazole, trimethoprim, alatrofloxacin, fidaxomycin, nalidixice acide, rifampin, derivatives and combination thereof.

More preferably, the antibiotic according to the invention is selected from the group consisting in cefotaxim, methicillin, vancomycin, and amoxicillin.

### Brief Description of the Drawings

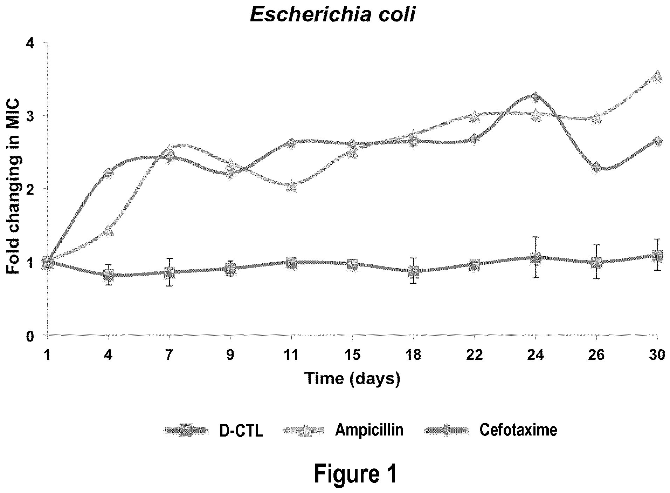

**Figure 1****: Resistance acquisition assay of *E. coli* in the presence of D-CTL compared to ampicillin and cefotaxim.** Bacteria were cultured in the presence of ½ MIC of the antibacterial agent for 30 days. The fold change in MIC was evaluated at the indicated days. The graph is representative of two experiments done independently.

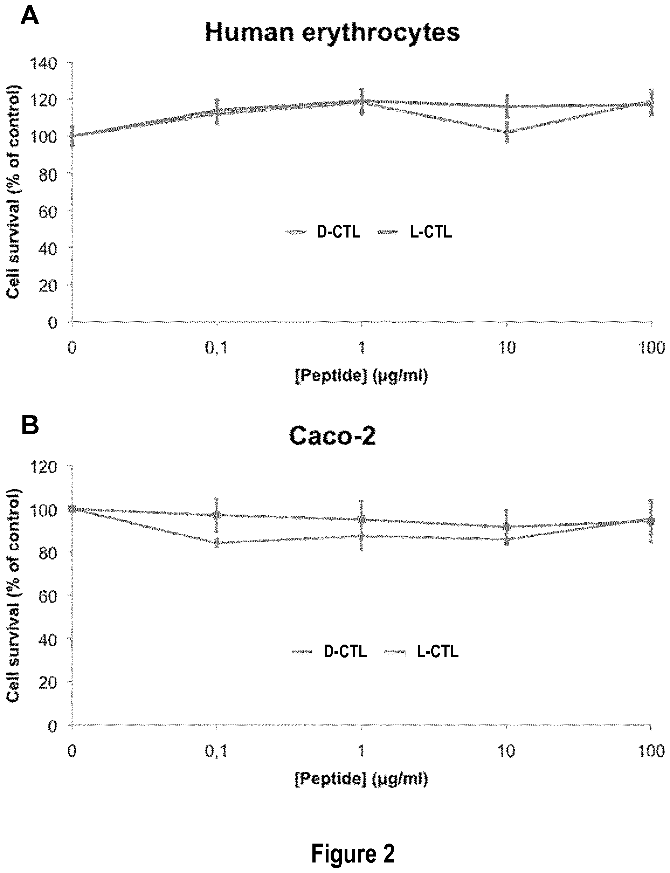

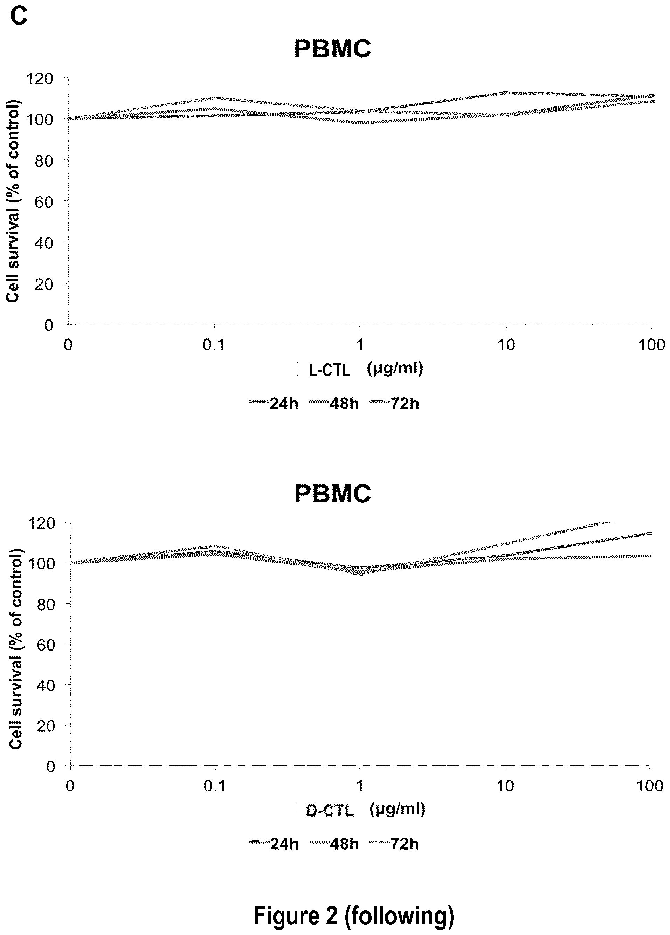

**Figure 2****: Cytotoxicity assays of D-CTL and L-CTL towards human erythocytes (A), human epithelial cells of the intestine (Caco-2 cells) (B) and PBMCs (C).** Erythrocytes hemolysis was evaluated on human erythrocytes treated with the indicated concentrations of D-CTL for one hour. The cytotoxicity of Caco-2 cells and PMBCs was assessed at the indicated concentrations for 72 hours. Each figure corresponds to a mean of at least two independant experiments.

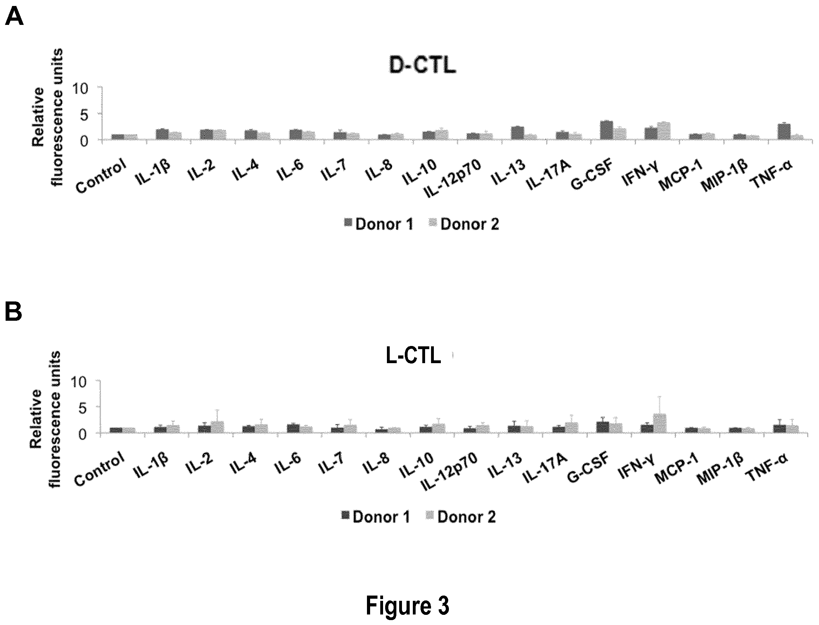

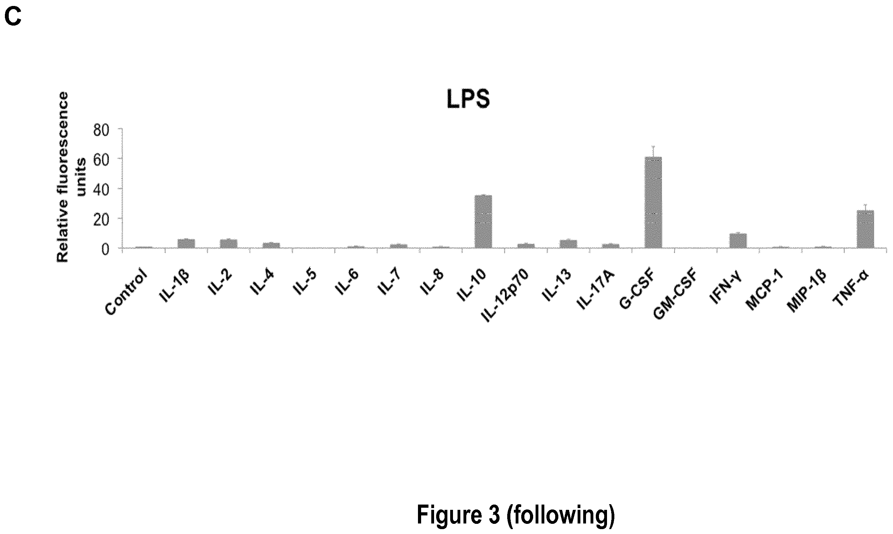

**Figure 3****: Cytokine release assay following treatment of PBMCs with D-CTL.** Cells from two donors were treated with D-CTL (A), L-CTL (B) for 24 hours and the indicated cytokines levels were evaluated in the cell supernatant using the Bio-Plex® technology. LPS was also used as a positive control for one of the two donors.

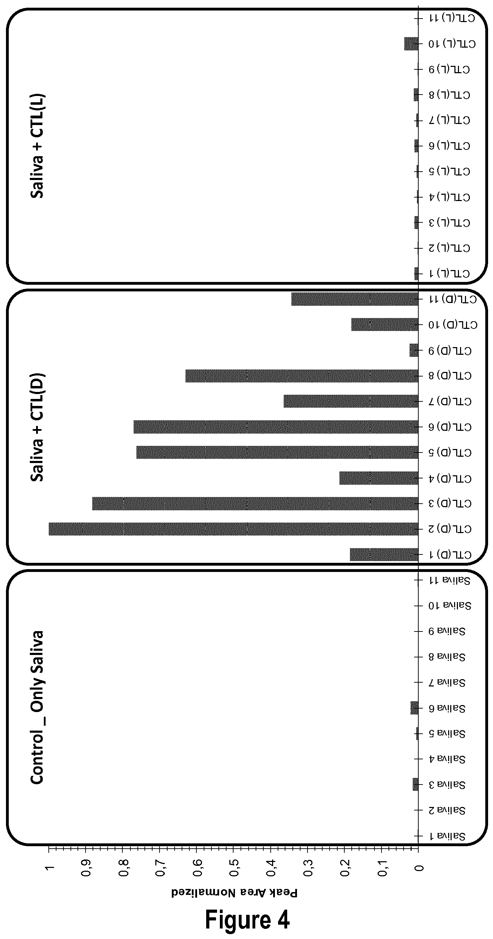

**Figure 4****: Stability of D-CTL and L-CTL in saliva.** Saliva from ten healthy volunteers was incubated with D-CTL or L-CTL for 24 hours and peptide stability was then assessed by LC-SRM. The analyses were performed on an Agilent 1100 Series HPLC system hyphenated to a QQQ-6490 triple quadrupole (Agilent Technologies). A total of 48 transitions (6 precursors) were monitored in an unscheduled mode within a total cycle time of 3 000 ms.

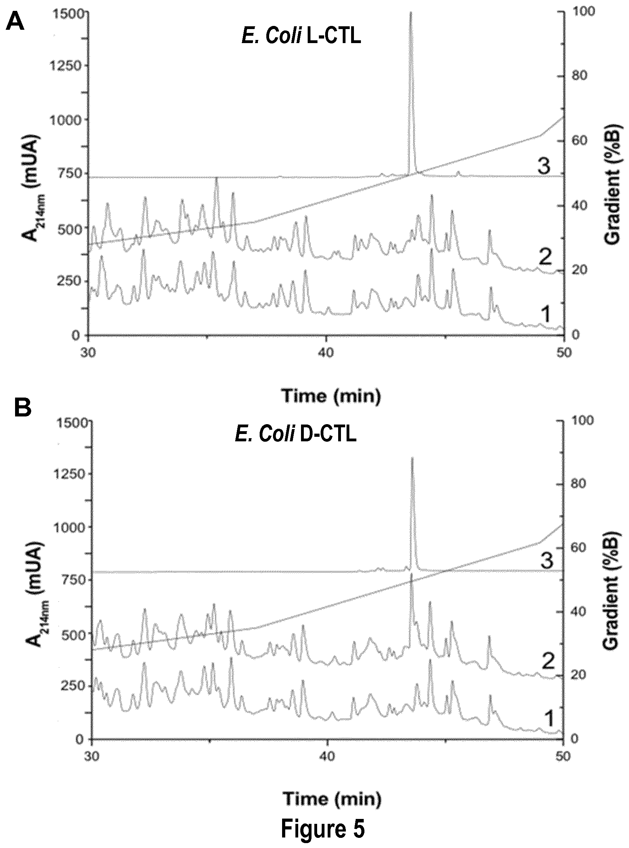

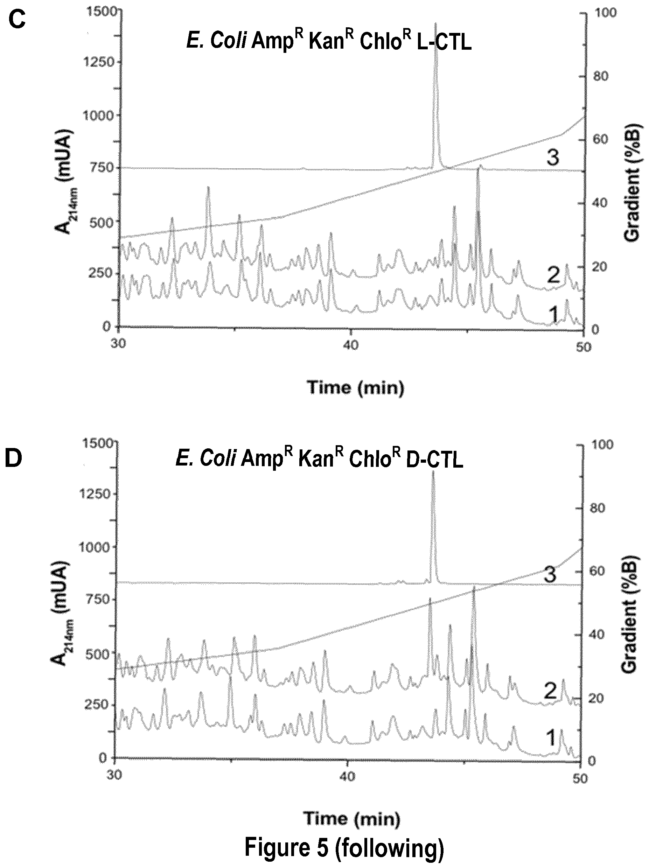

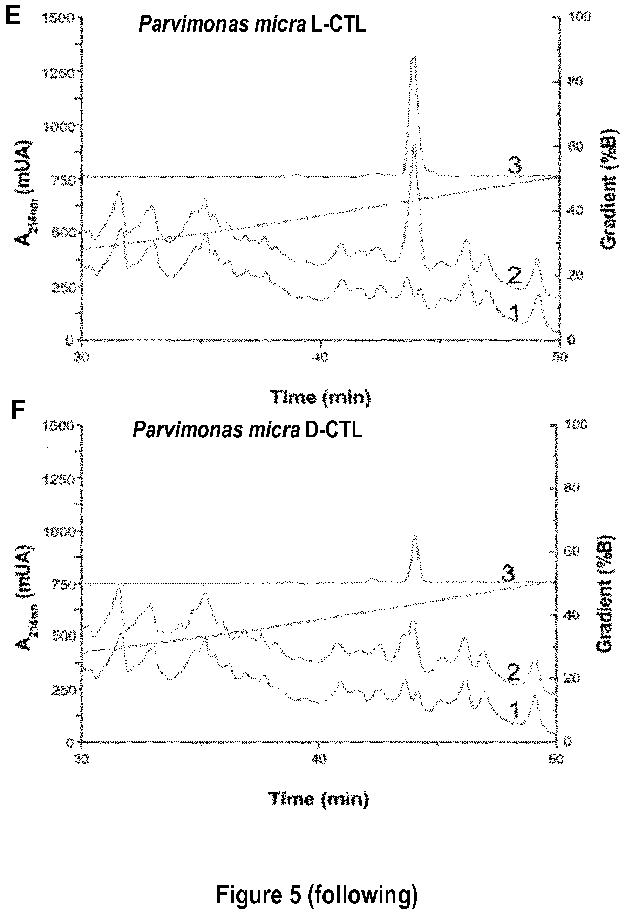

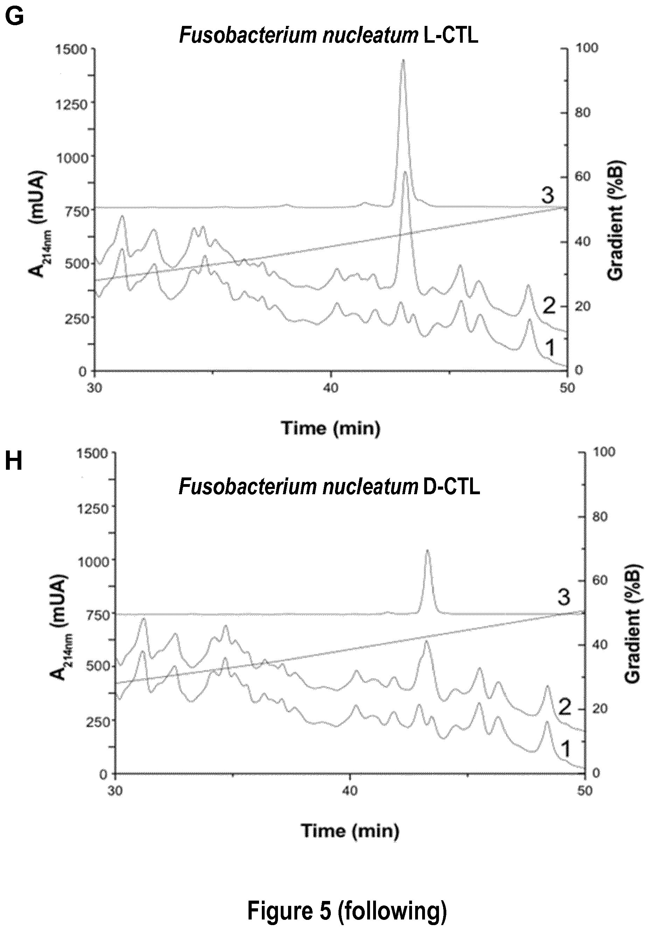

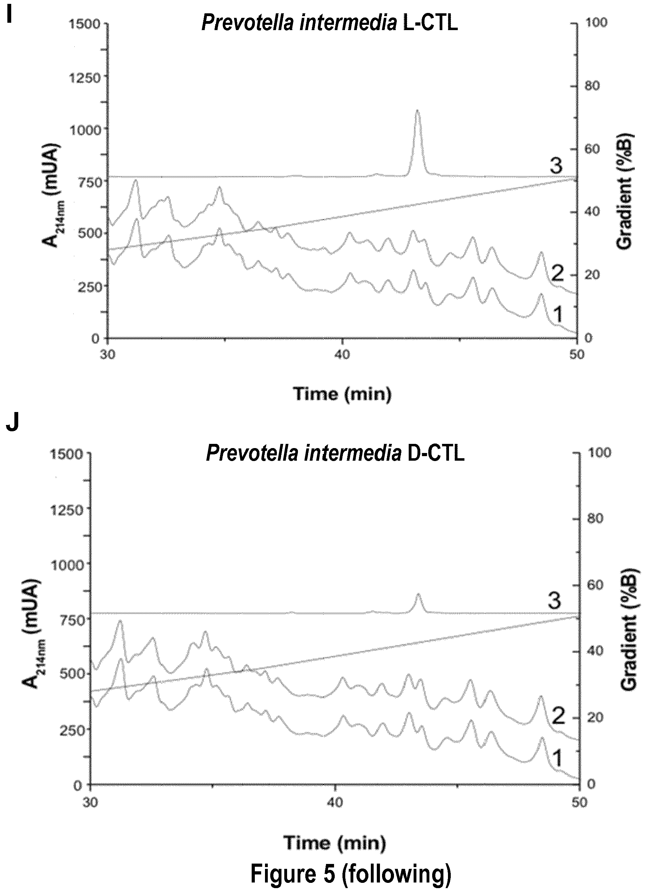

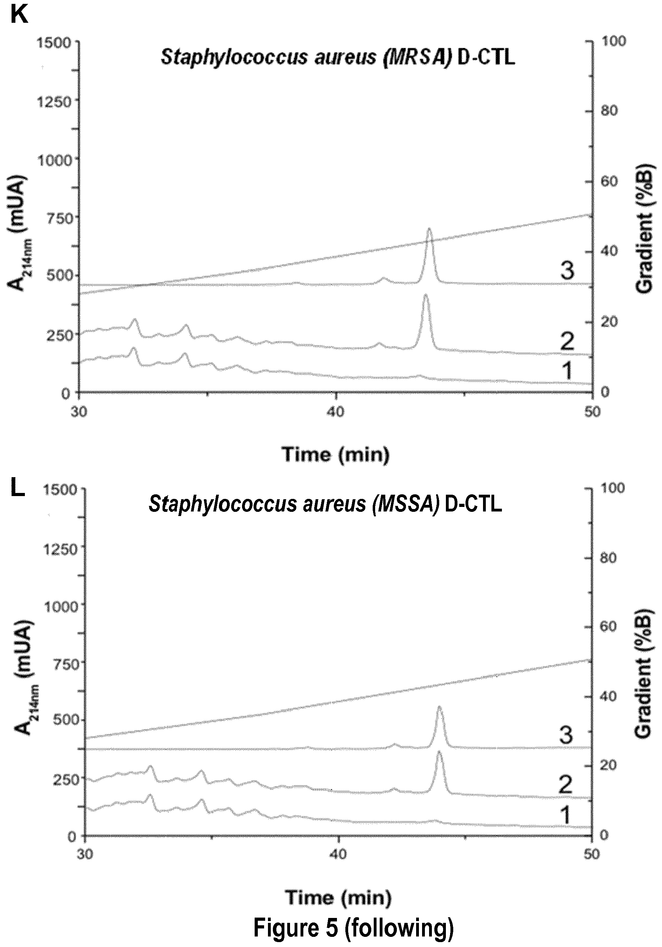

**Figure 5****: Stability of D-CTL and L-CTL towards the virulence factors of different bacterial strains.** Bacterial supernatants were incubated with D-CTL or L-CTL for 24 hours and peptide stability was then assessed by HPLC. Chromatograms 1 correspond to supernantant only, chromatograms 2 correspond to supernatant and peptide and chromatograms 3 corresponds to peptide only.

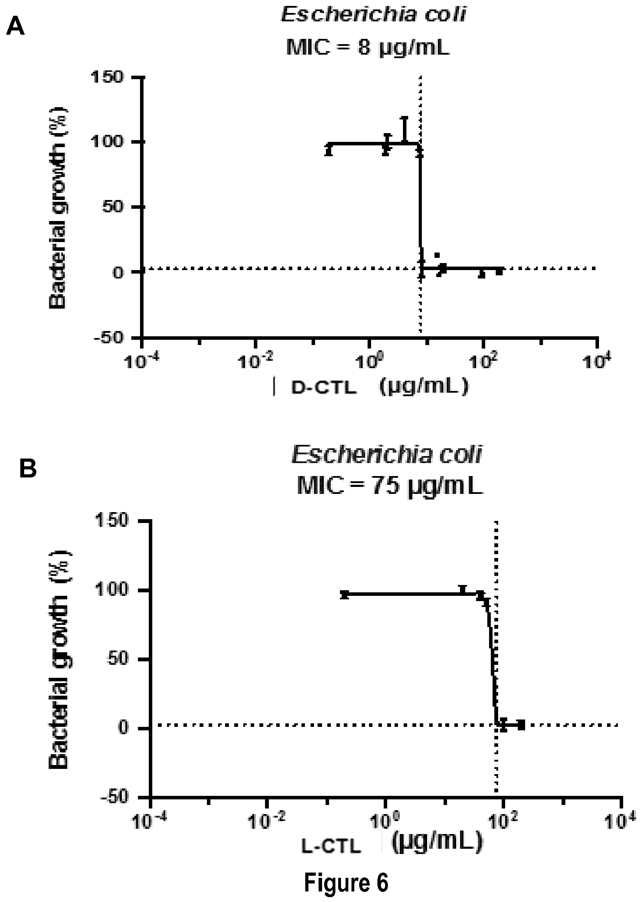

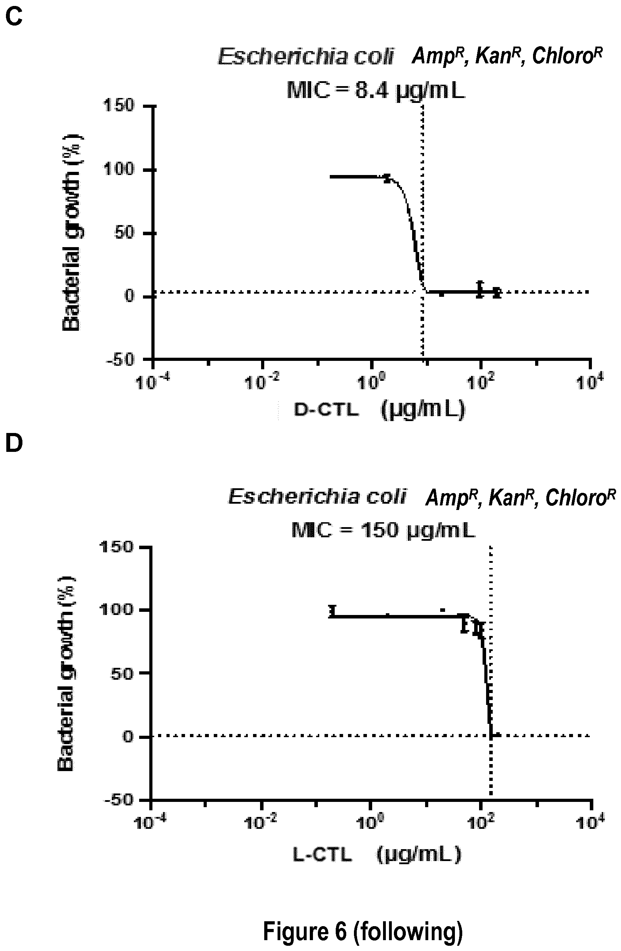

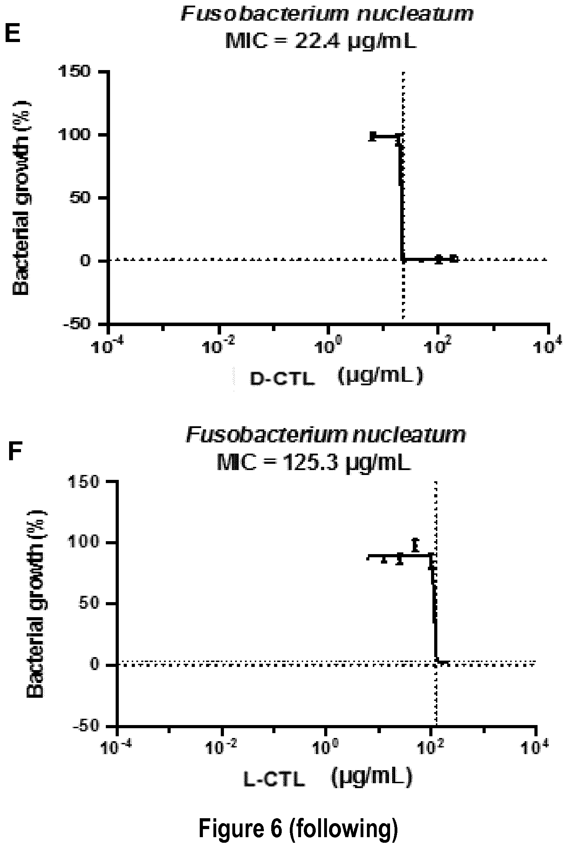

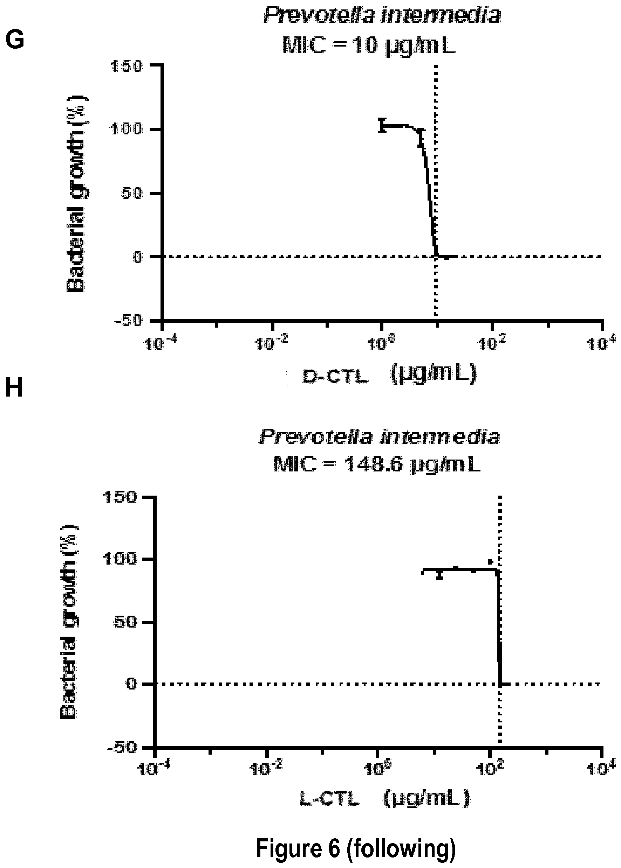

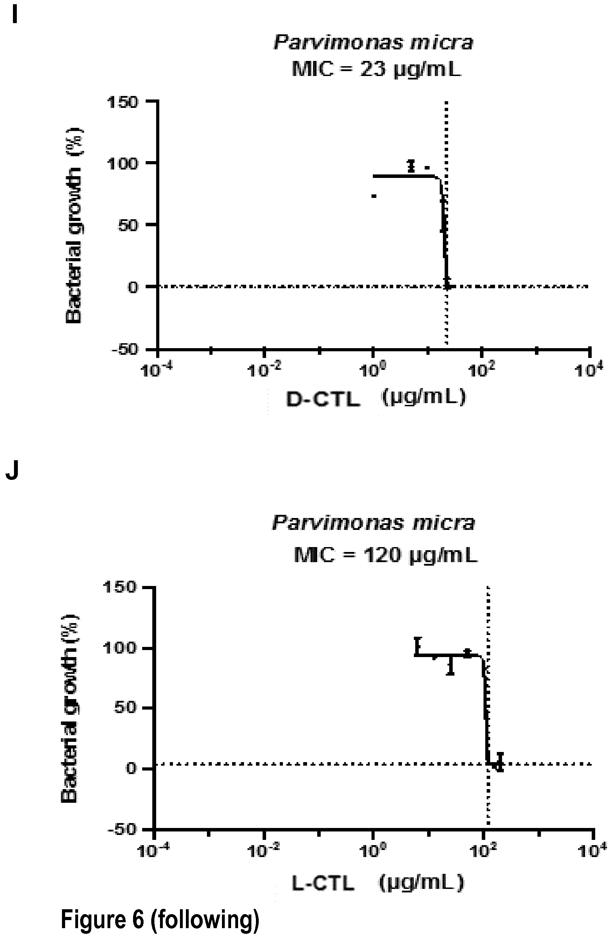

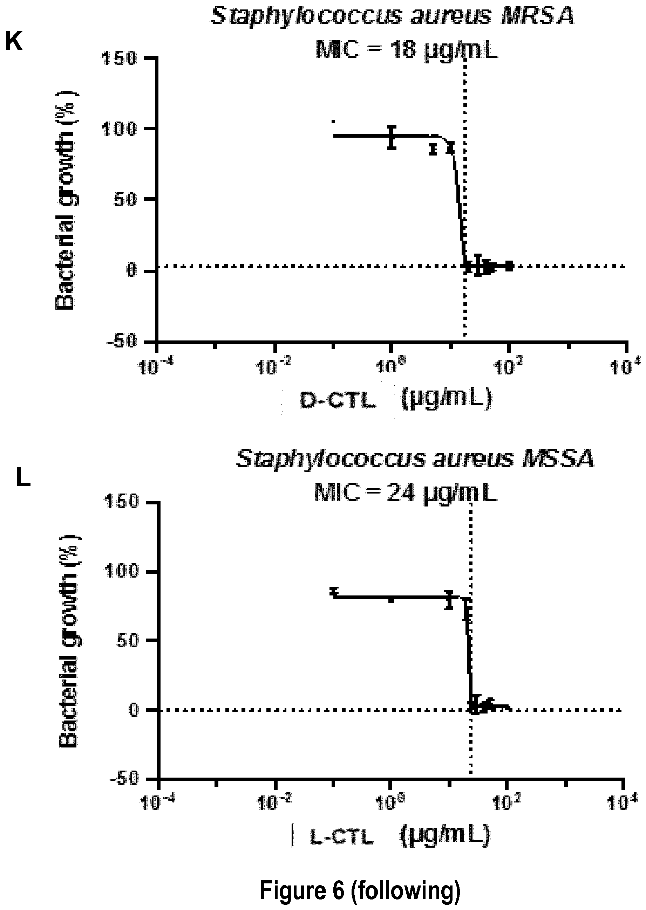

**Figure 6****: Antibacterial activity of CTL-D and CTL-L against pathogenic microorganisms.** The percentage of growth inhibition of the indicated pathogens in the presence of different concentrations of CTL-D or CTL-L was determined by broth microdilution assays. The MIC was determined using a modified Gompertz function as described in Lambert et al., 2000. Experiments were performed with biological replicates.

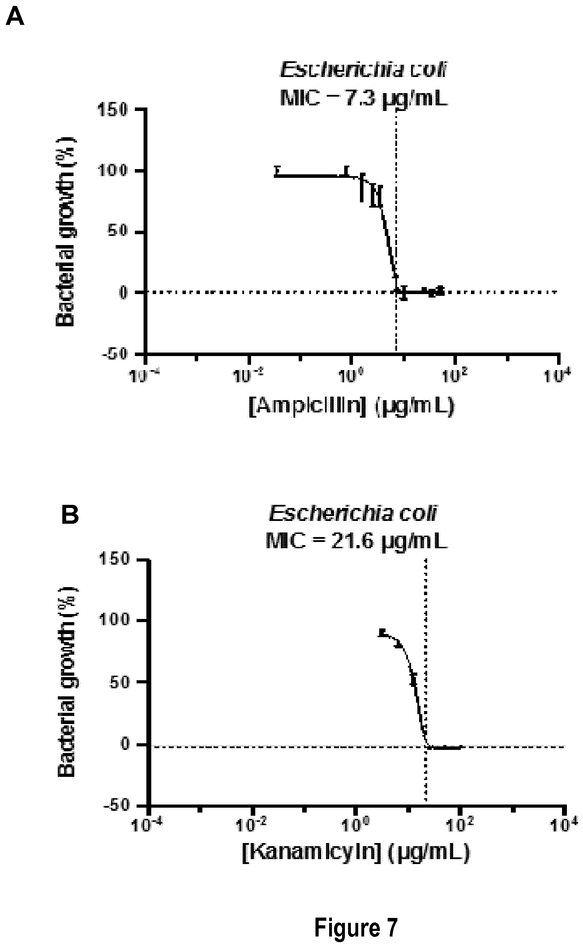

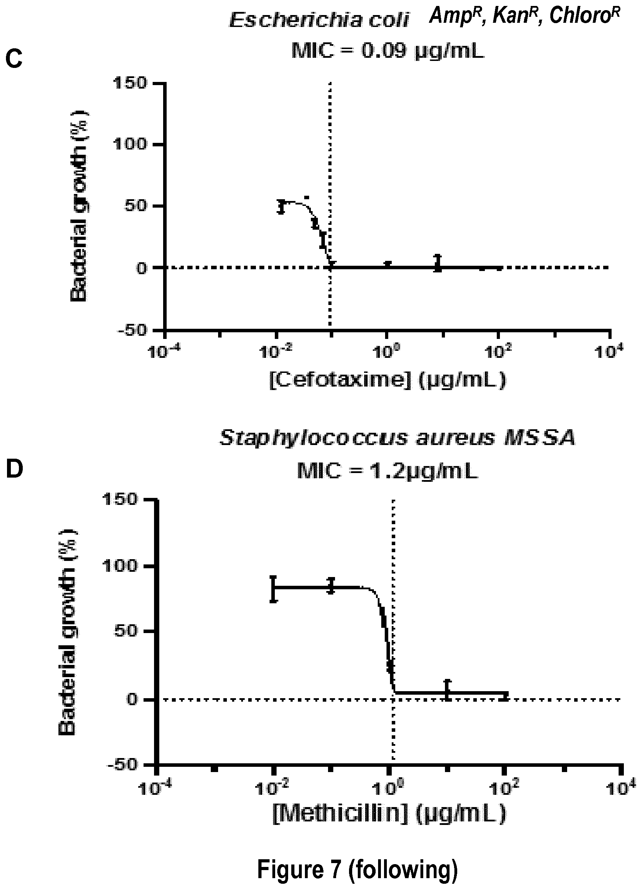

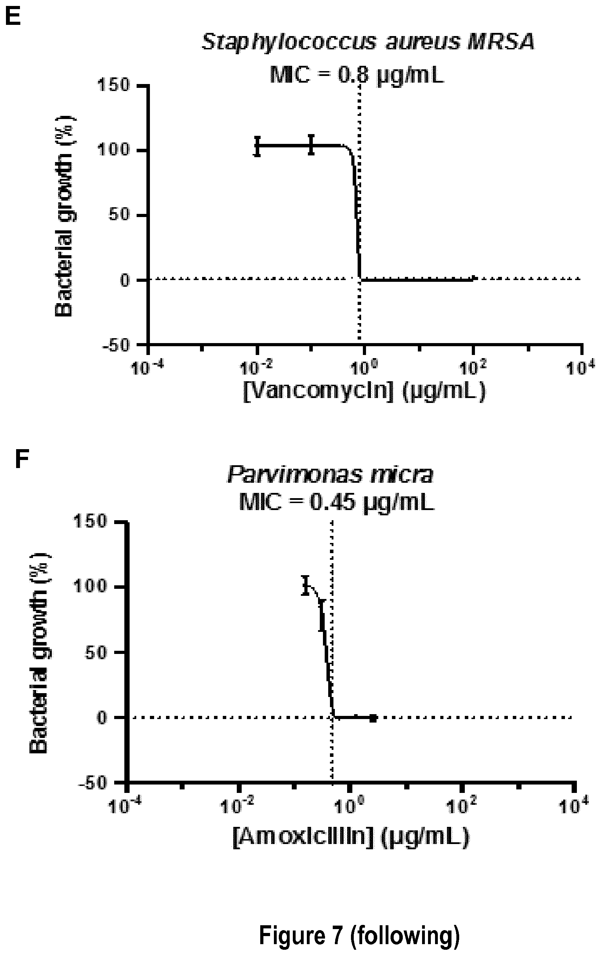

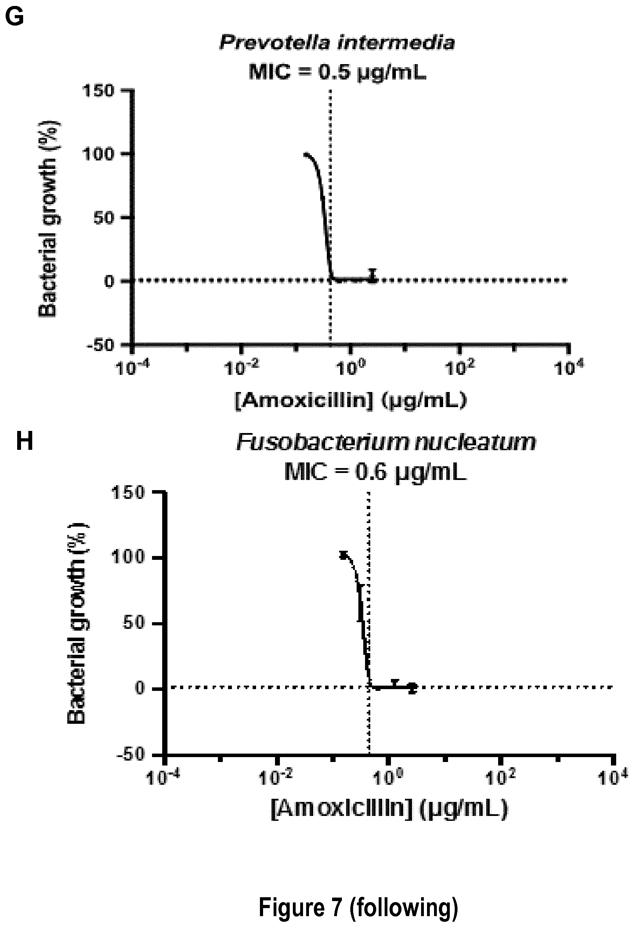

**Figure 7****:** The percentage of growth inhibition of the indicated pathogens in the presence of different concentrations of antibiotics was determined by broth microdilution assays. The MIC was calculated using a modified Gompertz function as described in Lambert et al., 2000. Experiments were performed with biological replicates.

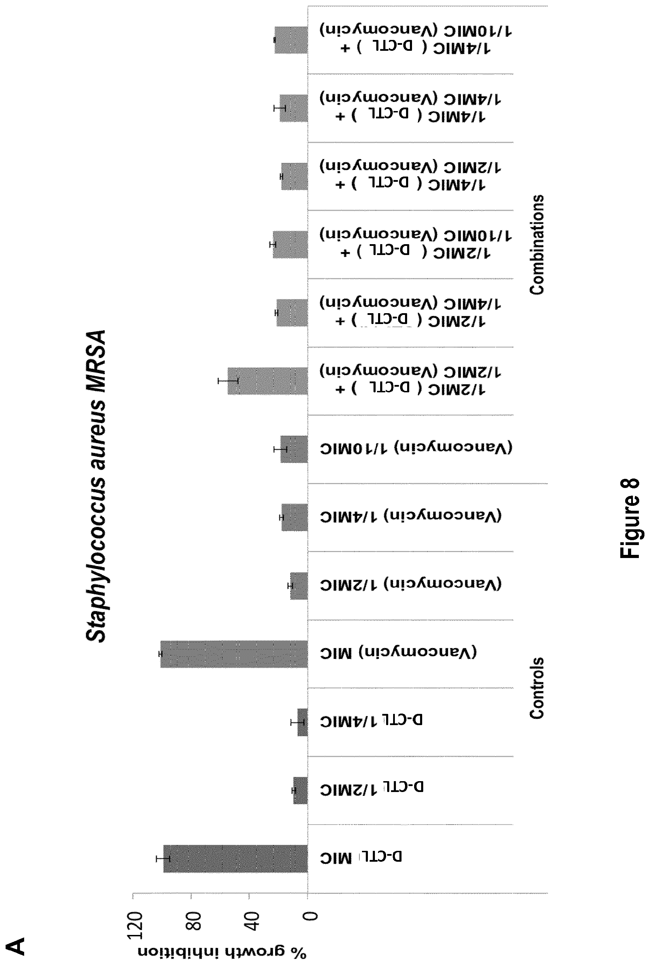

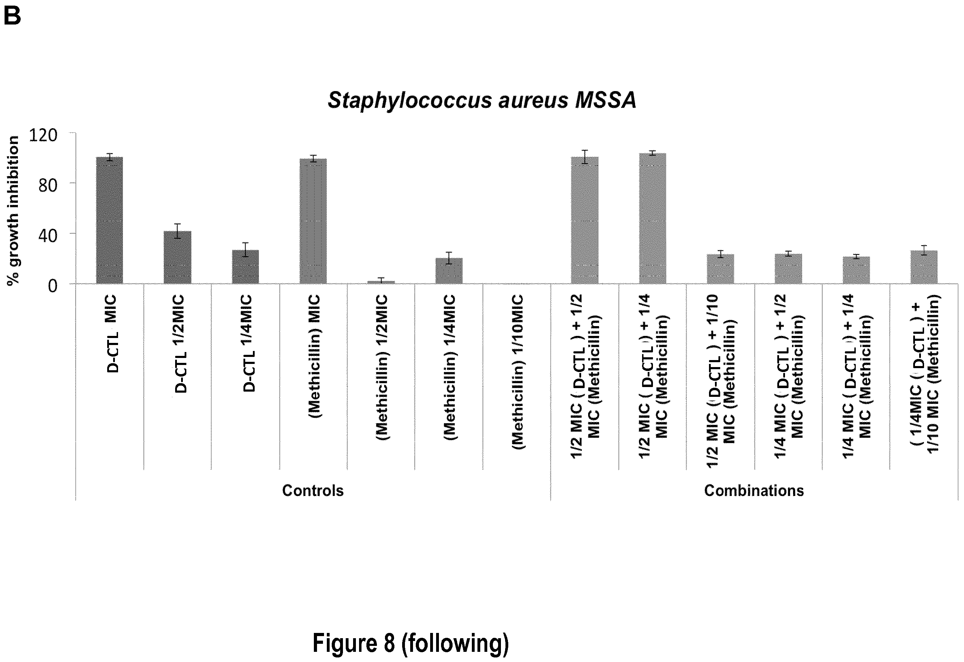

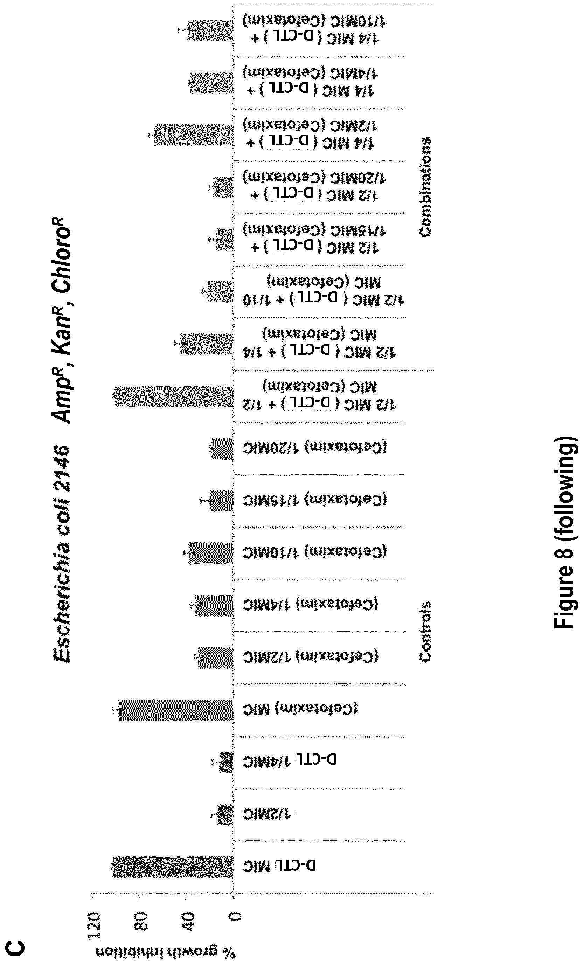

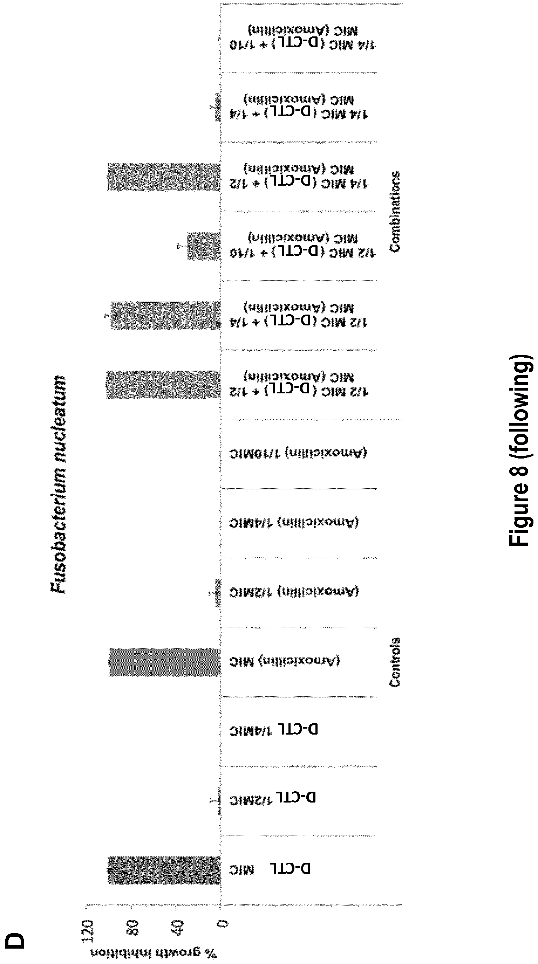

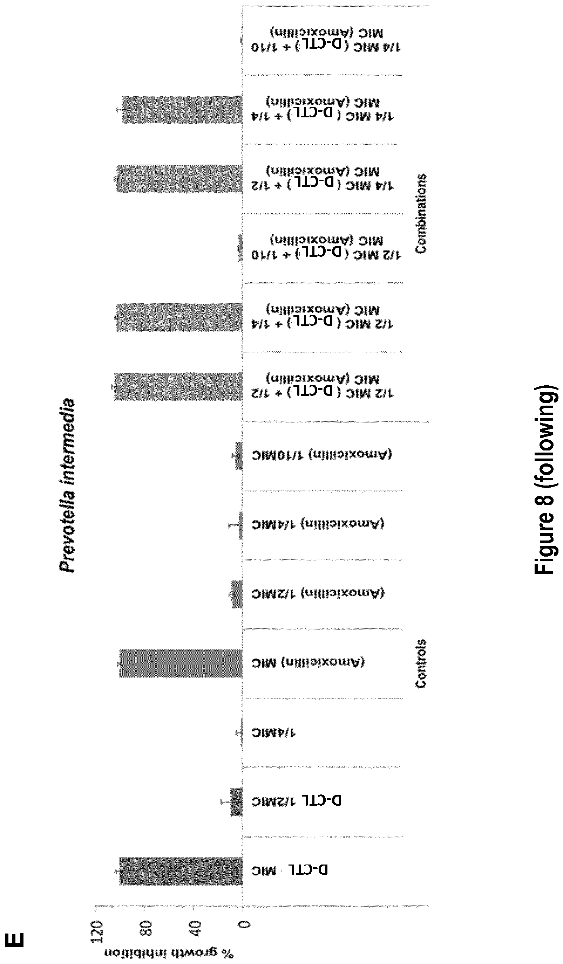

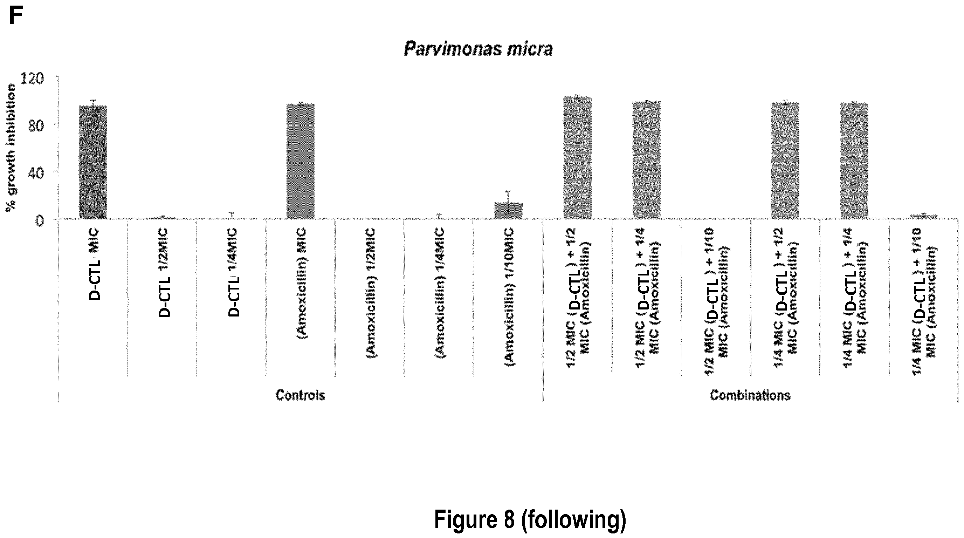

**Figure 8****: Antibacterial activity of CTL-D in combination with conventional antibiotics against pathogenic microorganisms.** The percentage of growth inhibition of the indicated pathogens in the presence of different concentrations of antibiotics was determined by broth microdilution assays. The MICs, defined as the lowest concentrations of each drug or the combo able to inhibit 100% of the inoculum, were used to calculate the FIC index of each combination. Each experiment was performed at least in duplicate.

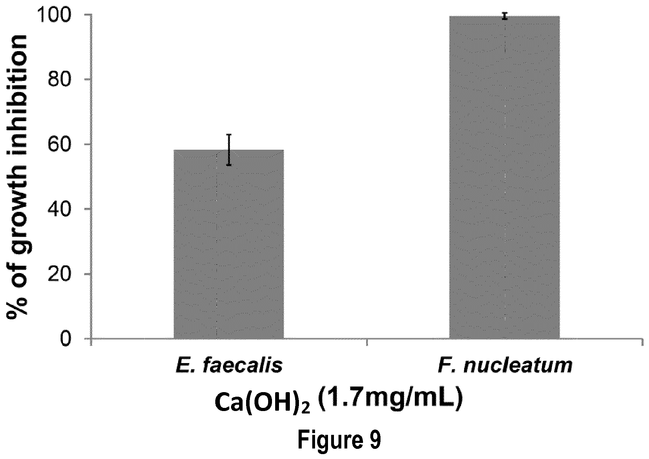

**Figure 9****: Inhibition of E. faecalis and E. nucleatum growth by a saturated solution of Ca(OH)2**
   Broth dilution assays were performed on E. faecalis and F. nucleatum in the presence of a saturated solution of Ca(OH)2. Results are expressed in percentage of growth inhibition and represent a mean of at least three independent experiments. For each set of assay, the standard deviation was determined.

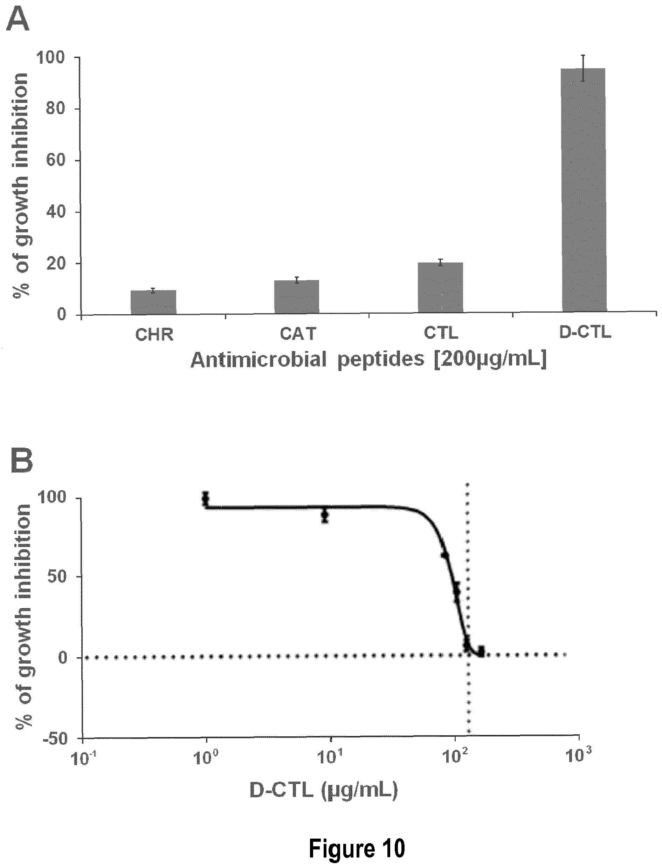

**Figure 10****: Activity of antimicrobial peptides against *E. faecalis***
   **A)** Broth dilution assays were performed on *E. faecalis* in the presence of the indicated peptides at a final concentration of 200 µg/mL. Results are expressed in percentage of growth inhibition and correspond to a mean of at least three experiments done independently. For each set of assay, the standard deviation was determined.
   **B)** The MIC of D-CTL on *E. faecalis* was determined by broth dilution assays in the presence of increasing amounts of D-CTL and calculated using a modified Gompertz model. For each set of assay, the standard deviation was determined.

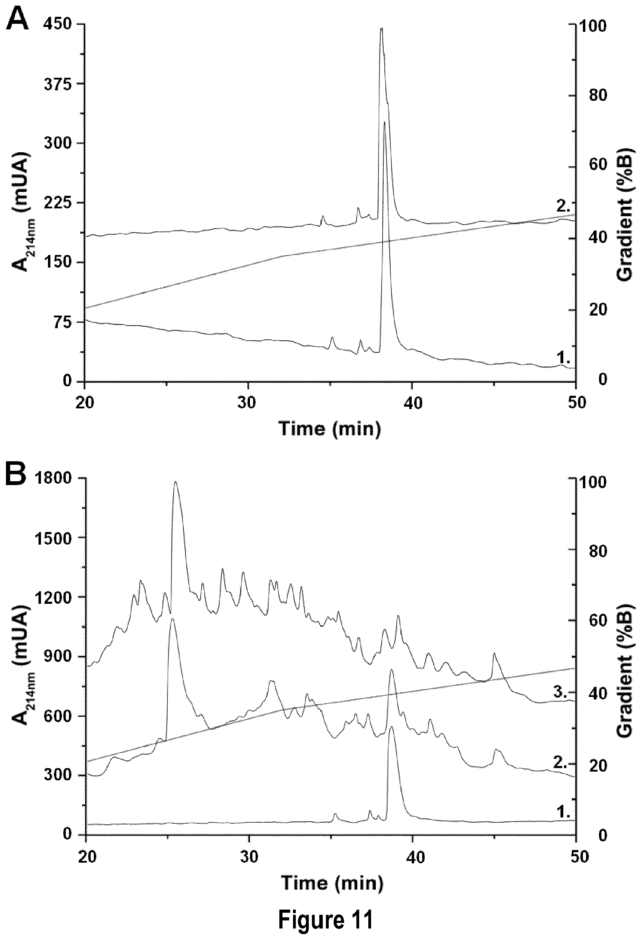

**Figure 11****: Stability of D-CTL in a saturated solution of Ca(OH)₂ and in bacterial supernatant**
   A) D-CTL at the MIC was incubated in a saturated solution (1.7 g/L) of Ca(OH)₂ diluted in milli-Q water (pH9) for 24h. The samples were then analyzed by HPLC. Chromatogram 1 corresponds to D-CTL, chromatogram 2 corresponds to D-CTL diluted in a saturated solution of Ca(OH)₂ (pH 9). Absorbance is expressed in Unity of Milli-Absorbance (UmA) depending on the time in minutes (min).
   **B)** The supernatant of *E. faecalis* was incubated with or without D-CTL at 37°C for 24h and the samples were analyzed by HPLC. Chromatogram 1 corresponds to D-CTL, chromatogram 2 corresponds to D-CTL diluted in the supernatant of *E. faecalis* and chromatogram 3 corresponds to the supernatant of *E. faecalis* alone. Results are expressed in Unity of Milli Absorbance (UmA) depending on the time in minutes (min).

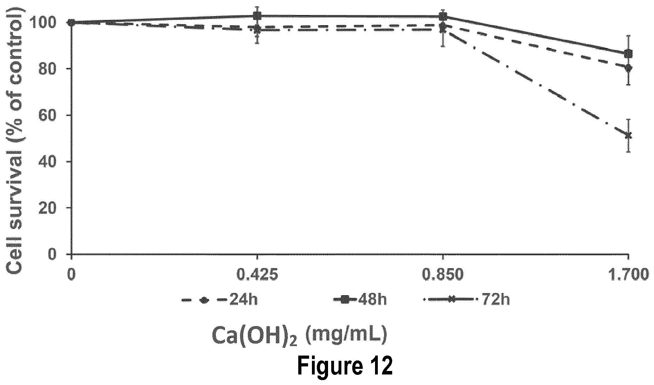

**Figure 12****: Cytotoxicity of Ca(OH)₂ on human gingival fibroblasts cells**
   HGF-1 cells were incubated for 24h with Ca(OH)₂ at 1,7mg/mL, 0,85 mg/mL and 0.425mg/mL. MTT assays were performed after an incubation of 24h, 48h and 72h. Results are expressed in percentage of cell viability in comparison to the control and represent a mean of at least three independent experiments. For each set of assay, the standard deviation was determined.

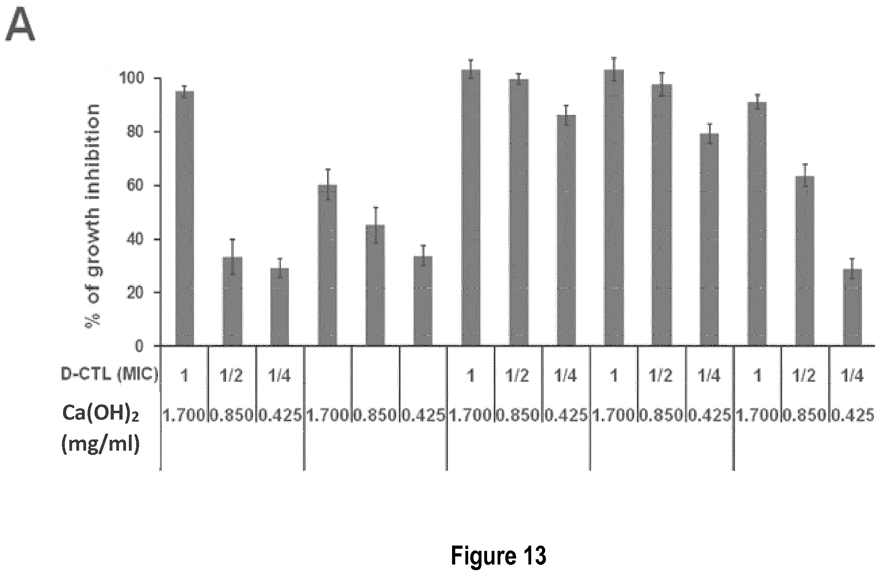

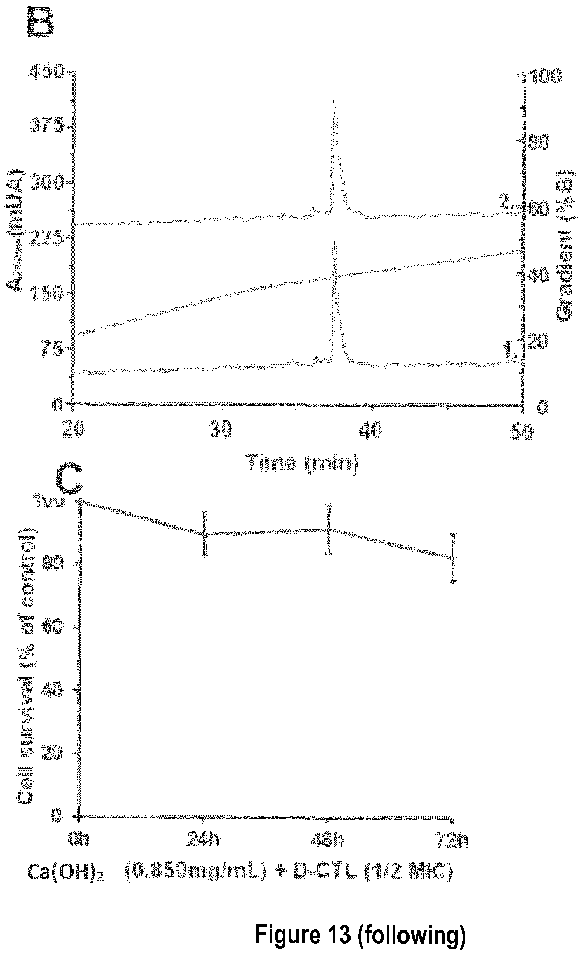

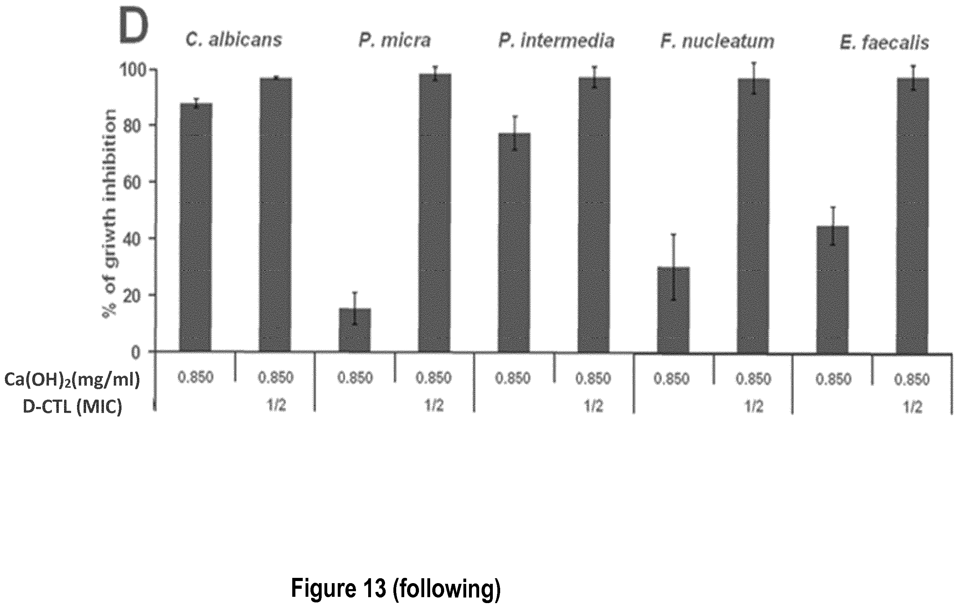

**Figure 13****: Antimicrobial activity, stability and cytotoxicity of the combination between D-CTL and Ca(OH)₂**
   A) The most efficient combination of Ca(OH)₂ and D-CTL was determined by broth dilution assays. The different combinations tested are indicated in the graph. Results correspond to a mean of at least three experiments done in triplicate and are expressed in percentage of *E. faecalis* growth inhibition. For each set of assay, the standard deviation was determined.
   **B)** The stability of the combination was determined by HPLC. Chromatogram 1 corresponds to D-CTL and Chromatogram 2 corresponds to the combination (Ca(OH)₂ 0,85g/L and ½ MIC of D-CTL) diluted mili-Q water (pH 8,5). Absorbance is expressed in Unity of Milli Absorbance (UmA) depending on the time in minutes (min).
   **C)** The cytotoxicity of the combination (Ca(OH)₂ 1.7mg/mL and ½ MIC of D-CTL) on HGF-1 cells was assessed by MTT assays. Data are a mean of at least three independent experiments and are expressed in percentage of viability. For each set of assay, the standard deviation was determined.
   **D)** The efficiency of the combination (Ca(OH)₂ 1.7mg/mL and ½ MIC of D-CTL) in comparison with Ca(OH)₂ 0.85mg/mL was tested on four other main endodontic pathogens: *Fusobacterium nucleatum, Parvimonas micra, Prevotella intermedia* and *Candida albicans.* Results are expressed in percentage of growth inhibition and correspond to a mean of at least three independent experiments, each performed in triplicate. For each set of assay, the standard deviation was determined.

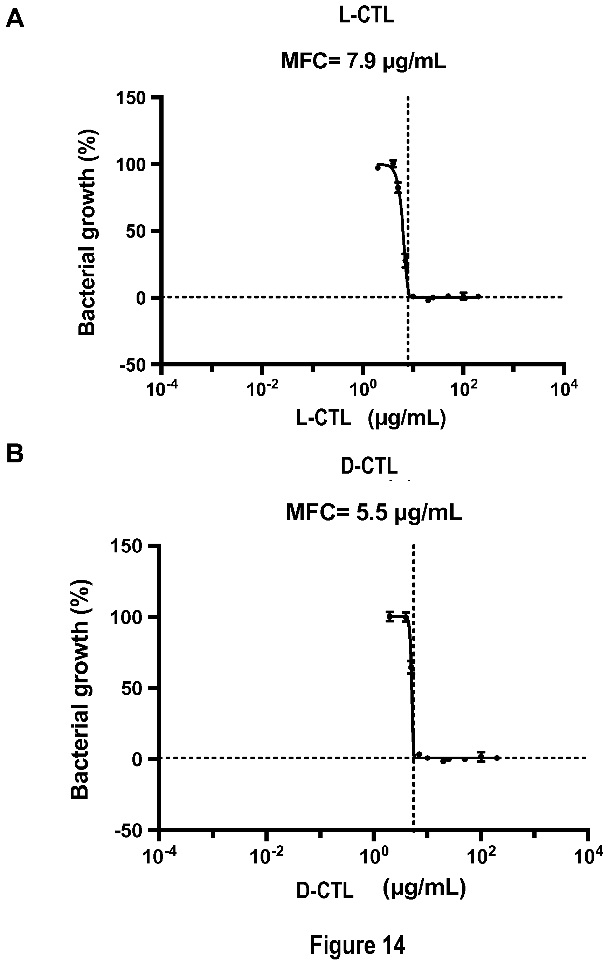

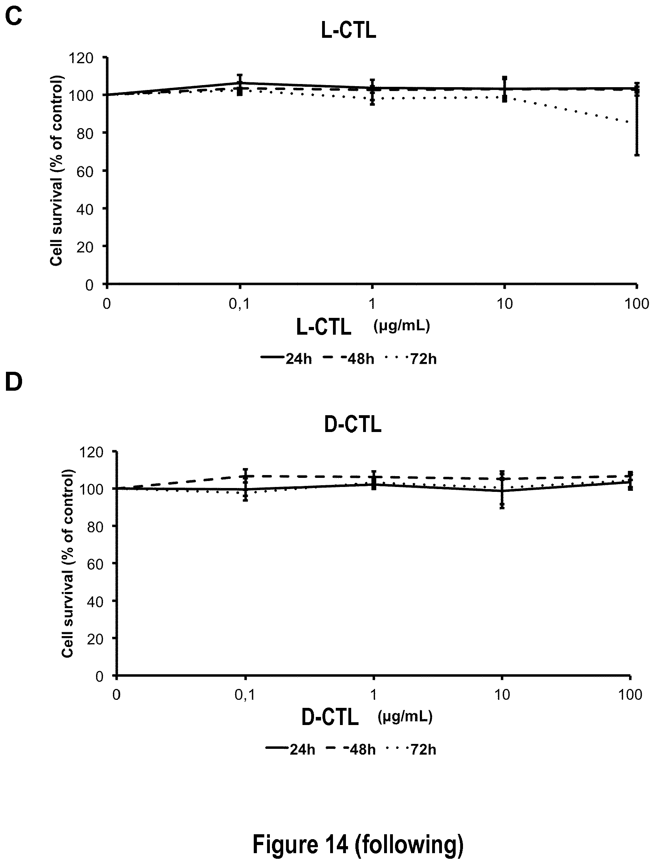

**Figure 14****: Comparison of L-CTL and D-CTL therapeutic index** The antifungal activity of L-CTL (A) and D-CTL (B) against *Candida albicans* was determined by antifungal tests performed in the presence of the peptide of interest at different concentrations. The positive control for each test is voriconazole (VCZ), a conventional antifungal agent. The minimal fungicidal concentration (MFC), determined using a modified Gompertz model, is 7.9 µg/mL for L-CTL (A), and 5.5 µg/mL for D-CTL (B). The peptides cytotoxicity was determined by MMT assays using a human gingival fibroblasts cell line (HGF-1) as a model (C-D). Each peptide (L-CTL (C) and D-CTL (D)) was incubated with the cells at different concentrations (0 µg/mL, 0.1 µg/mL, 1 µg/mL, 10 µg/mL, 100 µg/mL) for 24 h, 48 h and 72 h. The results obtained are expressed as percentage of cell survival. For each panel, the average of at least three separate experiments is shown.

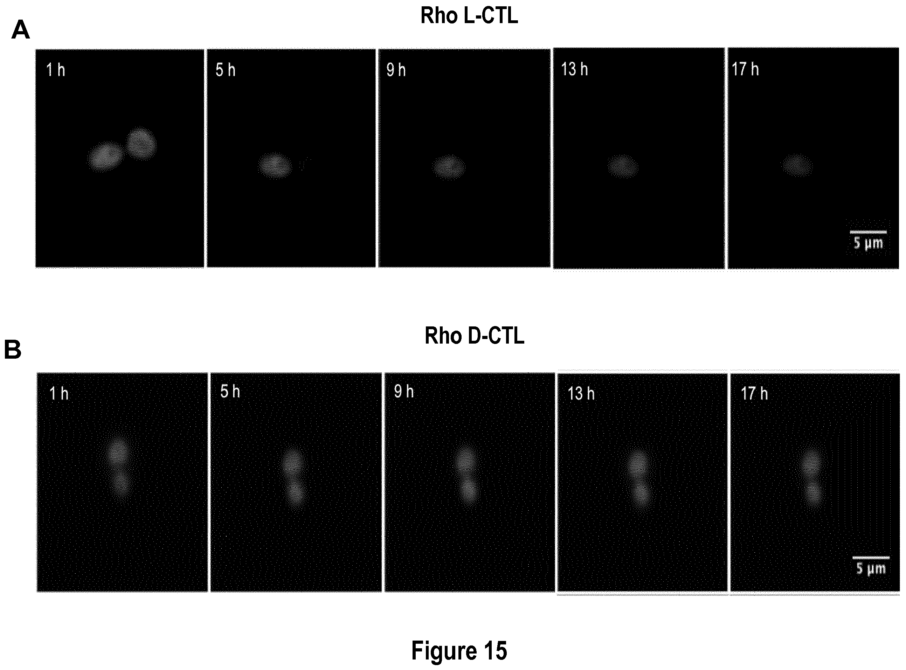

**Figure 15****: Time-lapse videomicroscopy analysis of colonies of *Candida albicans* treated by the rhodamined peptide (Rho-L-CTL or Rho-D-CTL)**
   The rhodamined peptides were used at a concentration of 10×MFC (10×7.9 µg/mL = 79 µg/mL for Rho-L-CTL, 10x5.5 µg/mL = 55 µg/mL for Rho-D-CTL), and incubated for 30 min with *Candida albicans.* Images (fluorescence and phase contrast) were captured with a 60x objective. The time elapsed between two frames is 4 h.

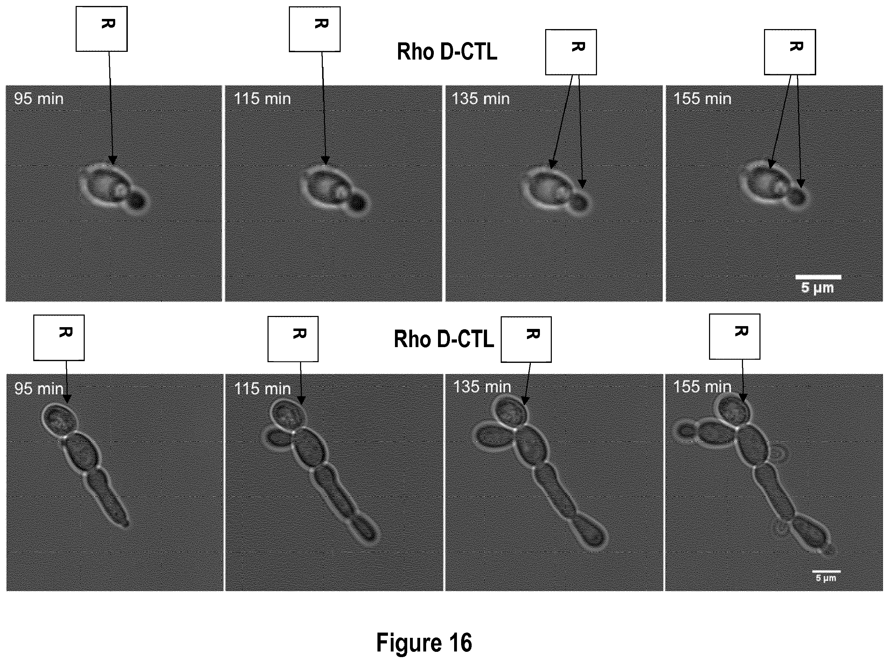

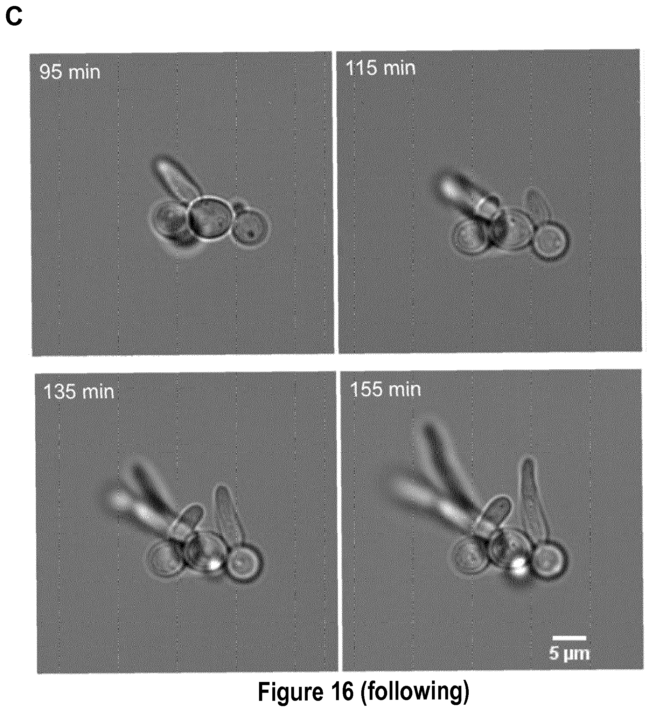

**Figure 16****: Time-lapse videomicroscopy analysis of colonies of *Candida albicans* partially invaded by Rho-D-CTL, depending on the cellular division**
   Rho-D-CTL was used at a concentration of 10×MFC (10x5.5 µg/mL = 55 µg/mL), and incubated for 30 min. Images (fluorescence and phase contrast) were captured with a 60x objective. The time elapsed between two frames is 20 min. In total, 16 colonies partially invaded by Rho-D-CTL were followed. 6 of them revealed that the division of an invaded yeast induce the passage of the rhodamined peptide in the nascent yeast (A). The other 10 colonies partially invaded grew by division of the non invaded yeast (B). "R" means that the cell is emmiting red fluorescence.
**Figure 16C****: Time-lapse videomicroscopy analysis of a colony of *Candida albicans* alone, without any previous treatment**
   Images were captured with a 60x objective. The time elapsed between two frames is 20 min. In total, 11 colonies were followed. In each case, a growth progressing from every yeast of the colony was observed.
**Figure 17****: Analysis by HPLC of L-CTL and D-CTL in the presence of the supernatant of *Candida albicans***
   100 µL of supernatant was directly incubated without (chromatogram 1) or with (chromatogram 2) L-CTL (A) or D-CTL (B) (19 µg; 10 µL) at 37°C for 24 h. As a control, each peptide (19 µg; 10 µL) was incubated in water (100 µL) at 37°C for 24 h prior being analysed by HPLC (chromatogram 3).
   Absorbance was monitored at 214 nm and the solvent system consisted of 0.1% (v/v) TFA in water (solvent A) and 0.09% (v/v) TFA in 70% (v/v) acetonitrile-water (solvent B). Elution was performed at a flow rate of 700 µL/min with a gradient of solvent B as indicated on the chromatograms. Each experiment was repeated three times.

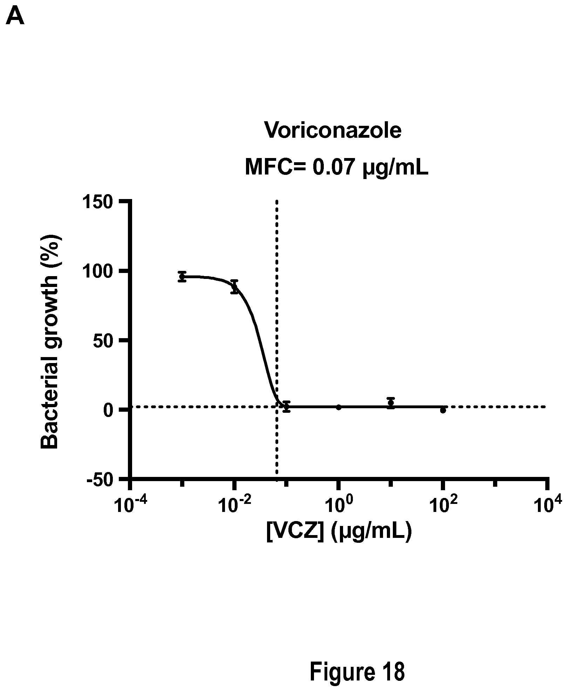

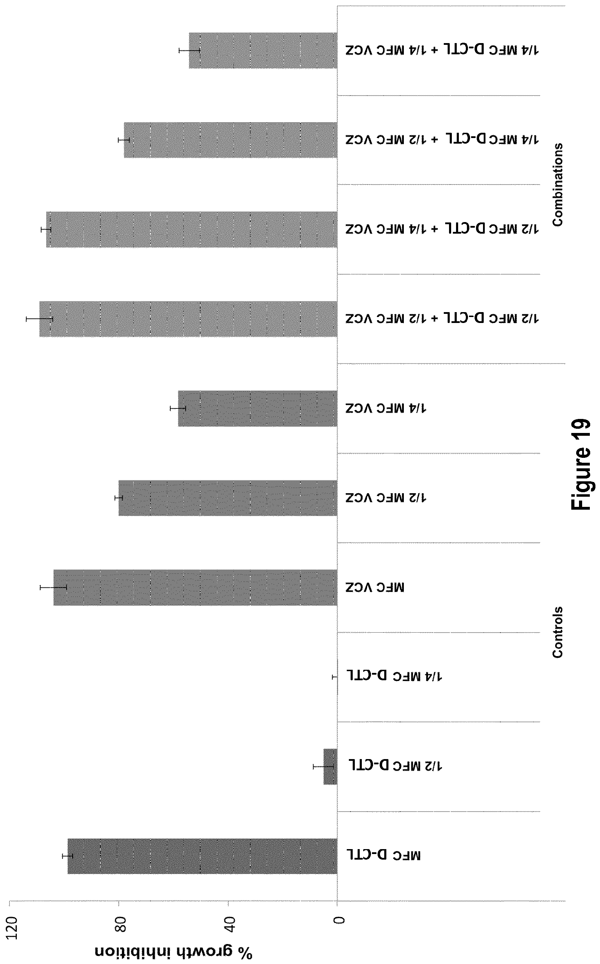

**Figure 18****: Evaluation of the combination of different concentrations of voriconazole (VCZ) and D-CTL against *Candida albicans***
   The antifungal activity of voriconazole (VCZ) against *Candida albicans* was determined by antifungal assays with decreasing concentrations of VCZ. The MFC of VCZ is 0.07 µg/mL (A). 4 combinations of D-CTL and VCZ on *Candida albicans* were tested (B): 1/2 MFC_{D-CTL} combined with 1/2 MFC_{VCZ,} 1/2 MFC_{D-CTL(d)} combined with 1/4 MFC_{VCZ}, 1/4 MFC_{D-CTL} combined with 1/2 MFC_{VCZ}, 1/4 MFC_{D-CTL} combined with 1/4 MFC_{VCZ}.

### Detailed description of the Invention

The inventors have surprisingly discovered that a cateslytin peptide wherein all the amino acids residues are D-configured (D-CTL) is a potent, broad spectrum antibiotic, even effective against antibiotic-resistant bacteria. Interestingly, D-CTL effectiveness is much greater than its natural L-configured counterpart (L-CTL). The inventors have also discovered that D-CTL is highly potent against fungus such as *Candida albicans.* Moreover, D-CTL present several other interesting properties. Indeed, D-CTL is not cytotoxic or immunogenic. D-CTL is very stable at high temperature or acidic pH. D-CTL has a synergetic action with several classic antibiotics. The inventors have thus discovered a new molecule for the development of a very promising new class of antibiotics and antifungal drugs.

### Definitions

As used herein, the terms "Chromogranin A" or "CGA" are equivalent and refer to an acidic glyco-phosphoprotein stored in the secretory vesicles of numerous nervous, neuroendocrine and immune cells and released upon stress in most of the body fluids (Taupenot L, 2003, N. Engl. J. Med., 348: 1134-1149). CGA is a precursor of several biological active peptides.

As used herein, the terms "Catestatin" or "CAT" are equivalent and refer to a biological natural active peptide derived from CGA that exhibit antimicrobial activity against a wide array of pathogens.

The terms "Cateslytin" or "CTL", as used herein, are equivalent and refer to an arginine rich fragment of the catestatin located at its N-terminal extremity. The cateslytin is the active domain of the catestatin. Preferably, the cateslytin peptide of the invention derived from the bovine catestatin and has the sequence of SEQ ID NO: 1.

Every amino acid, except glycine, can occur in two isomeric forms, because of the possibility of forming two different enantiomers (stereoisomers) around their central carbon atom. By convention, these two different enantiomers are called "L-" and "D-forms" or are considered as "L-" and "D-configured" or are corresponding to "right" and "left-handed configurations". As used herein, the terms "D-form", "D-configured" and "right-handed configuration" are equivalent and refer to amino acids that when drawn in the Fischer projection in which the carboxylic acid group is on top and the side chain on bottom have their amine group on the right of the carbon chain.

In the peptide or protein sequences described in this document, amino-acids are represented by their one letter code according to the following nomenclature: A: Alanine; C: cysteine; D: aspartic acid; E: glutamic acid; F: phenylalanine; G: glycine; H: histidine; I: isoleucine; K: lysine; L: leucine; M: methionine; N: asparagine; P: proline; Q: glutamine; R: arginine; S: serine; T: threonine; V: valine; W: tryptophan and Y: tyrosine.

As used herein, the terms "sequence identity" or "identity" are used interchangeably and refer to an exact amino acid to amino acid correspondence of two amino acid sequences. Percent of identity between two amino acid sequences (A) and (B) is determined by comparing the two sequences aligned in an optimal manner, through a window of comparison. Said alignment of sequences can be carried out by well-known methods, for example, using the algorithm for global alignment of Needleman-Wunsch. Protein analysis software matches similar sequences using measures of similarity assigned to various substitutions, deletions and other modifications, including conservative amino acid substitutions. Once the total alignment is obtained, the percentage of identity can be obtained by dividing the full number of identical amino acid residues aligned by the full number of residues contained in the longest sequence between the sequence (A) and (B). Sequence identity is typically determined using sequence analysis software. For comparing two amino acid sequences, one can use, for example, the tool "Emboss needle" for pairwise sequence alignment of proteins providing by EMBL-EBI and available on http://www.ebi.ac.uk/Tools/services/web/toolform.ebi?tool=emboss_needle&context=prot ein, using default settings: (I) Matrix: BLOSUM62, (ii) Gap open: 10, (iii) gap extend: 0.5, (iv) output format: pair, (v) end gap penalty: false, (vi) end gap open: 10, (vii) end gap extend: 0.5.

As used herein, the terms "Amino acid modification", "amino acid change", and "mutation" are used interchangeably and refer to a change in an amino acid sequence such as a substitution, an insertion, and/or a deletion.

By "amino acid substitution" or "substitution" herein is meant the replacement of an amino acid at a particular position in a parent amino acid sequence with another amino acid.

By "amino acid insertion" or "insertion" is meant the addition of an amino acid at a particular position in a parent amino acid sequence.

By "amino acid deletion" or "deletion" is meant the removal of an amino acid at a particular position in a parent amino acid sequence.

The amino acid substitutions may be conservative. A conservative substitution is the replacement of a given amino acid residue by another residue having a side chain ("R-group") with similar physico-chemical properties (e.g., charge, bulk and/or hydrophobicity). In general, a conservative amino acid substitution will not substantially change the functional properties of a protein. Conservative substitutions and the corresponding rules are well-described in the state of the art. For instance, conservative substitutions can be defined by substitutions within the groups of amino acids reflected in the following tables:

**Table 1 - Amino Acid Residue**

| Amino Acid groups | Amino Acid Residues |
|---|---|
| Acidic Residues | D and E |
| Basic Residues | K, R, and H |
| Hydrophilic Uncharged Residues | S, T, N, and Q |
| Aliphatic Uncharged Residues | G, A, V, L, and I |
| Non-polar Uncharged Residues | C, M, and P |
| Aromatic Residues | F, Y, and W |

**Table 2 - Alternative Conservative Amino Acid Residue Substitution Groups**

| | | | |
|---|---|---|---|
| 1 | Alanine (A) | Serine (S) | Threonine (T) |
| 2 | Aspartic acid (D) | Glutamic acid (E) | |
| 3 | Asparagine (N) | Glutamine (Q) | |
| 4 | Arginine (R) | Lysine (K) | |
| 5 | Isoleucine (I) | Leucine (L) | Methionine (M) |
| 6 | Phenylalanine (F) | Tyrosine (Y) | Tryptophan (W) |

**Table 3 - Further Alternative Physical and Functional Classifications of Amino Acid Residues**

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Positively charged residues | K, R and H |
| Polar residues | C, D, E, H, K, N, Q, R, S, and T |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | E, Q, T, K, S, G, P, D, E, and R |

Additional groups for conservative substitutions are: valine-leucine-isoleucine, phenylalanine-tyrosine, lysine-arginine, alanine-valine, and asparagine-glutamine.

As used herein, the terms "parent amino acid sequence" or "parent polypeptide" are equivalent and refer to an unmodified amino acid sequence that is subsequently modified to generate a variant.

As used herein, the terms "variant amino acid sequence", "variant polypeptide" or "variant" are equivalent and refer to an amino acid sequence that differs from that of a parent amino acid sequence by virtue of at least one amino acid modification. Typically, a variant comprises from 1 to 6 amino acid modifications, preferably from 1 to 4 amino acid modifications. In particular, the variant may have 1, 2, 3, 4, 5, 6 amino acid changes as compared to its parent amino acid sequence. In a specific aspect, the variant may have from 1 to 3 amino acid changes, e.g. 1, 2, or 3 amino acid changes as compared to its parent amino acid sequence. The variants may comprise one or several amino acid substitutions, and/or, one or several amino acid insertions, and/or one or several amino acid deletions. In some embodiments, the variant may comprise one or several conservative substitutions, e.g. as shown here above.

As used herein, the term "consists essentially in" is intended to refer to an amino acid sequence that differs from that of a parent amino acid sequence by virtue of 1, 2, 3, 4, or 5 substitutions, additions, deletions or combination thereof, preferably by virtue of 1, 2, or 3 substitutions, additions, deletions or combination thereof. In one embodiment, it refers to an amino acid sequence that differs from that of a parent amino acid sequence by virtue of 1, 2 or 3 additions or deletions, and/or 1, 2 or 3 substitutions. In one embodiment, it refers to an amino acid sequence that differs from that of a parent amino acid sequence by virtue of 1, 2, 3, 4, or 5 substitutions.

The terms "kit", "product" or "combined preparation", as used herein, defines especially a "kit of parts" in the sense that the combination partners (a) and (b), as defined in the present application can be dosed independently or by use of different fixed combinations with distinguished amounts of the combination partners (a) and (b), i.e. simultaneously or at different time points. The parts of the kit of parts can then be administered simultaneously or chronologically staggered, that is at different time points for any part of the kit of parts. The ratio of the total amounts of the combination partner (a) to the combination partner (b) to be administered in the combined preparation can be varied. The combination partners (a) and (b) can be administered by the same route or by different routes.

As used herein, the term "treatment", "treat" or "treating" refers to any act intended to ameliorate the health status of patients such as therapy, prevention, prophylaxis and retardation of the infection. In certain embodiments, such term refers to the amelioration or eradication of the infection or symptoms associated with it. In other embodiments, this term refers to minimizing the spread or worsening of the infection resulting from the administration of one or more therapeutic agents to a subject with such a disease.

As used herein, the terms "subject", "individual" or "patient" are interchangeable and refer to an animal, preferably to a mammal, even more preferably to a human, including adult, child, new-borns and human at the prenatal stage. However, the term "subject" can also refer to non-human animals, in particular mammals such as dogs, cats, horses, cows, pigs, sheep and non-human primates, among others.

The terms "quantity," "amount," and "dose" are used interchangeably herein and may refer to an absolute quantification of a molecule.

As used herein, the terms "active principle", "active ingredient" and "active pharmaceutical ingredient" are equivalent and refers to a component of a pharmaceutical composition having a therapeutic effect.

As used herein, the term "therapeutic effect" refers to an effect induced by an active ingredient, a pharmaceutical composition, a kit, a product or a combined preparation according to the invention, capable to prevent or to delay the appearance of an infection, or to cure or to attenuate the effects of an infection.

As used herein, the term "effective amount" refers to a quantity of an active ingredient or of a pharmaceutical composition which prevents, removes or reduces the deleterious effects of the infection. It is obvious that the quantity to be administered can be adapted by the man skilled in the art according to the subject to be treated, to the nature of the infection, etc. In particular, doses and regimen of administration may be function of the nature, of the stage and of the severity of the infection to be treated, as well as of the weight, the age and the global health of the subject to be treated, as well as of the judgment of the doctor.

The term "synergic effect", as used herein, refers to a pharmaceutical composition according to the invention or to a kit, product or combined preparation according to the invention having a therapeutic effect superior to the sum of the therapeutic effects of all the active ingredients present in said pharmaceutical composition, kit, product, or combined preparation, when individually taken. Such an effect can be evaluated on the basis of the fraction of inhibitory concentrations (FIC-index) as illustrated in the examples.

As used herein, the terms "sub-therapeutic amount" or "sub-therapeutic dose" are equivalent and refer to an amount of active ingredient which is not sufficient to induce a therapeutic effect by itself. In particular, the term "subtherapeutic dose" refers to an amount or dose of an active ingredient lower than the conventional dose administered to a subject for the same indication and the same administration route when it is used alone.

As used herein, the term "excipient or pharmaceutically acceptable carrier" refers to any ingredient except active ingredients that is present in a pharmaceutical composition. Its addition may be aimed to confer a particular consistency or other physical or gustative properties to the final product. An excipient or pharmaceutically acceptable carrier must be devoid of any interaction, in particular chemical, with the actives ingredients.

As used herein, the term "simultaneous" refers to a pharmaceutical composition, a kit, a product or a combined preparation according to the invention in which the active ingredients are used or administered simultaneously, i.e. at the same time.

As used herein, the term "sequential" refers to a pharmaceutical composition, a kit, a product or a combined preparation according to the invention in which the active ingredients are used or administered sequentially, i.e. one after the other. Preferably, when the administration is sequential, all the active ingredients are administered in less than about an hour, preferably less than about 10 minutes, even more preferably in less than about a minute.

As used herein, the term "separate" refers to a pharmaceutical composition, a kit, a product or a combined preparation according to the invention in which the active ingredients are used or administered at distinct time of the day. Preferably, when the administration is separate, the active ingredients are administered with an interval of about 1 hour to about 24 hours, preferably with an interval of about 1 hour and 15 hours, more preferably with an interval of about 1 hour and 8 hours, even more preferably with an interval of about 1 hour and 4 hours.

As used herein, the term "infection" refers to the invasion of an organism's body tissues by disease-causing microorganisms, their multiplication, and the reaction of host tissues to these microorganisms and eventually the toxins they produce.

The terms "infectious agent", "microbial agent", "pathogen" and "disease-causing microorganism", as used herein, are equivalent and refer to microorganisms that cause infection. Preferably, the infectious agent according to the invention is selected from virus, bacteria, fungus, including yeasts, or parasites.

As used herein, the terms "antibiotic" and "antibacterial" are equivalent and refer to a type of antimicrobial active ingredient used in the treatment and prevention of bacterial infections.

As used herein, the term "antiviral" refers to type of antimicrobial active ingredient used in the treatment and prevention of viral infections.

As used herein, the term "antifungal" refers to type of antimicrobial active ingredient used in the treatment and prevention of fungal infections.

As used herein, the term "antiparasitic" refers to type of antimicrobial active ingredient used in the treatment and prevention of parasitic infections.

As used herein, the terms "hospital-acquired infection", "HAI" or "nosocomial infection" are equivalent and refer to an infection that is contracted from the environment or staff of a healthcare facility. It can be spread in the hospital environment, nursing home environment, rehabilitation facility, clinic, or other clinical settings. Infection is spread to the susceptible patient in the clinical setting by a number of means including health care staff that can spread infection, contaminated equipment, bed linens, or air droplets, the outside environment, another infected patient, or even the patient itself, such as the patient's own skin microbiota which can become opportunistic after surgery or other procedures that compromise the protective skin barrier.

As used herein, the terms "catheter-related infection" or "catheter-related bloodstream infection" are equivalent and refer to a type of hospital-acquired infection originating from an intravenous catheter.

The terms "antimicrobial resistance" or "AMR", as used herein, are equivalent and refer to the ability of a microbial agent to resist the effects of medication previously used to treat them. Antimicrobial resistance encompasses antifungal resistance, antiviral resistance, antiparasitic resistance, and antibiotic resistance.

As used herein, the term "Amp^{R}" refers to bacteria that are resistant to the antibiotic ampicillin.

As used herein, the term "Kan^{R}" refers to bacteria that are resistant to the antibiotic kanamycin.

As used herein, the term "Chlor^{R}" refers to bacteria that are resistant to the antibiotic chloramphenicol.

β-lactamases are enzymes (EC 3.5.2.6) produced by bacteria that provide multi-resistance to β-lactam antibiotics such as penicillins, cephamycins, and carbapenems (or ertapenem).

As used herein, the term "AmpC" refers to bacteria that produce the β-lactamase of the C type that hydrolyze broad and extended-spectrum cephalosporins.

As used herein, the terms "Extended-spectrum β-lactamases" or "ESBL" are equivalent and refer to bacteria that produce beta-lactamases that can hydrolyze extended-spectrum cephalosporins with an oxyimino side chain but not broad cephalosporins.

As used herein, the terms "VIM" and "Verona integron-encoded metallo-β-lactamase" are equivalent and refer to bacteria that produce a Metallo-β-lactamase of the VIM type that hydrolyze carbapenems.

As used herein, the terms "OXA" and "oxacillinase" are equivalent and refer to bacteria that produce a type of β-lactamase capable to hydrolyze carbapenems.

As used herein, the terms "KPC" and "K. pneumoniae carbapenemase" are equivalent and refer to bacteria that produce a type of β-lactamase capable to hydrolyze carbapenems.

As used herein, the terms "MSSA" or "Methicillin-sensitive *Staphylococcus aureus"* refer to a *Staphylococcus aureus* bacteria which is sensitive to the antibiotic methicillin.

As used herein, the terms "MRSA" or "Methicillin-resistant *Staphylococcus aureus"* refer to a *Staphylococcus aureus* bacteria which is resistant to the antibiotic methicillin.

As used herein, the term "wound" refers to a type of injury in which the skin is torn, cut, or punctured.

As used herein, the term of "cicatrization" refers to the process the process of wound healing.

In the present document, the term « about » refers to a range of values of ± 10% of the specified value. For example, « about 50 » comprise values of ± 10% of 50, i.e. values in the range between 45 and 55. Preferably, the term « about » refers to a range of values of ± 5% of the specified value.

### Cateslytin peptide

In a first aspect, the invention relates to a cateslytin peptide having an amino acid sequence consisting or consisting essentially in the sequence of SEQ ID NO: 1, wherein at least 60% of the amino acids residues of said cateslytin are D-configured.

The cateslytin peptide according to the invention may have a variant amino acid sequence having no more than 1, 2, 3, 4, 5, 6, preferably no more than 1, 2 or 3, amino acid modifications within the sequences of SEQ ID NO: 1. In particular, the variant amino acid sequence may have 1 or 2 amino acid modifications, preferably 1 amino acid modification.

The amino acids modifications according to the invention may be amino acid additions, deletions, substitutions, or combinations thereof. Preferably, amino acid modifications according to the invention are substitutions. More preferably, the amino acid modifications according to the invention are conservative substitutions.

In a particular embodiment, the cateslytin peptide according to the invention has the following amino acid sequence: X₁SMX₂LSFRX₃X₄X₅YGFR (SEQ ID NO: 7), wherein X₁, X₂, X₃, X₄, and X₅ can be any amino acid, preferably:
- X₁ is R or S;
- X₂ is a positively charged amino acid, preferably R or K;
- X₃ is T, S or A;
- X₄ is P, K or R; and
- X₅ is a very small amino acid, preferably G or A.

Preferably, the cateslytin peptide according to the invention has the following amino acid sequence: X₁SMX₂LSFRARX₅YGFR (SEQ ID NO: 8), wherein X₁, X₂, and X₅ can be any amino acid, preferably:
- X₁ is R or S;
- X₂ is a positively charged amino acid, preferably R or K,
- X₅ is a very small amino acid, preferably G or A.

More preferably, the cateslytin peptide according to the invention has an amino acid sequence selected from the group consisting in the sequences of SEQ ID NO: 1 (RSMRLSFRARGYGFR), SEQ ID NO: 2 (SSMKLSFRARGYGFR), SEQ ID NO: 3 (SS MKLSFRARAYGFR), SEQ ID NO: 4 (RSMKLSFRARAYGFR), SEQ ID NO: 5 (RSMKLSFRTRAYGFR), and SEQ ID NO: 6 (RSMKLSFRAPAYGFR).

Even more preferably, the cateslytin peptide according to the invention has an amino acid sequence consisting in the sequence of SEQ ID NO: 1.

At least 60% of the amino acids residues of the cateslytin peptide according to the invention are D-configured. Preferably, at least 65%, 70%, 75%, 80%, 85%, 90%, 95% of the amino acids residues of the cateslytin peptide according to the invention are D-configured.

In a most preferred embodiment all the amino acids residues of the cateslytin peptide according to the invention are D-configured.

In a particular embodiment, the cateslytin peptide according to the invention has an amino acid sequence consisting in the sequence of SEQ ID NO: 1, wherein all the amino acids residues of said cateslytin peptide are D-configured.

The N- and C-termini of the cateslytin peptides described herein may be optionally protected against proteolysis. In a preferred embodiment, the N-terminus may be in the form of an acetyl group, and/or the C-terminus may be in the form of an amide group. In a preferred embodiment, the peptide has a free C-terminal end.

Alternatively or in addition, internal modifications of the cateslytin peptides to be resistant to proteolysis are also envisioned, e.g. wherein at least a -CONH- peptide bond is modified and replaced by a (CH2NH) reduced bond, a (NHCO) retro-inverso bond, a (CH2-O) methylene-oxy bond, a (CH2-S) thiomethylene bond, a (CH2CH2) carba bond, a (CO-CH2) cetomethylene bond, a (CHOH-CH2) hydroxyethylene bond), a (N-N) bound, a E-alcene bond or also a -CH=CH-bond.

For instance, the cateslytin peptide may be modified by acetylation, acylation, amidation, cross-linking, cyclization, disulfide bond formation, formation of covalent crosslinks, formation of cysteine, formation of pyroglutamate, formylation, gamma-carboxylation, glycosylation, GPI anchor formation, hydroxylation, iodination, methylation, myristylation, oxidation, phosphorylation, and the like.

The cateslytin peptide according to the invention may comprise one or more amino acids which are rare amino acids in particular hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine, aminobutyric acid; or synthetic amino acids in particular ornithine, norleucine, norvaline and cyclohexyl-alanine.

Optionally, the cateslytin peptide according to the invention can be linked to an additional moiety, optionally through a linker or spacer (e.g., diglycine). Optionally, the cateslytin peptide can be part of a protein fusion. The protein fusion results in a heterologous sequence. In particular, it does not comprise the sequence of a catestatin peptide. The additional moiety can be a moiety facilitating its cellular uptake or entry, in particular a PTD (protein transduction domain) or Cell Penetrating Peptide; a homing peptide; a stabilizing agent such as PEG (polyethyleneglycol), oligo-N-methoxy-ethylglycine (NMEG), albumin, an albumin-binding protein or an immunoglobulin Fc domain; an affinity tag such as an immune-tag, biotin, lectin, or chelator; a purification tag such as a His-tag; a detectable label such as an optical tag, a chelated lanthamide, a fluorescent dye, or a FRET acceptor/donor; a targeting moiety; a secretion signal peptide; or a combination thereof. The additional moiety can be added either at the N-terminal end or C-terminal end of the peptide.

In another aspect of the invention, the cateslytin peptide is covalently bound to a polyethylene glycol (PEG) molecule by their C-terminal terminus or a lysine residue, notably a PEG of 1500 or 4000 MW, for a decrease in urinary clearance and in therapeutic doses used and for an increase of the half-life in blood plasma.

In a particular embodiment, the term "cateslytin peptide" also encompass multimeric cateslytin peptides. As used herein, the term "multimeric cateslytin peptide" refers to a structure that comprises two or several monomers of cateslytin peptide according to the invention. As used herein, the term "monomer" refers to a single cateslytin peptide according to the invention when comprised in a structure which comprises at least two of them. The multimeric cateslytin peptide according to the invention can be a homopolymer or a heteropopymer. In a homopolymer, all the monomers are identical. In a heteropolymer, at least two monomers are different. The multimeric cateslytion peptide according to the invention is preferably a homomultimeric cateslyntin peptide. The number of monomers in a multimeric cateslytin peptide is preferably an integer comprised betwenn 2 and 10, more preferably between 2 and 6, even more preferably between 2 and 4. The multimeric cateslytin peptide according to the invention may be a trimeric cateslytin peptide. In a preferred embodiment, the multimeric cateslytin peptide according to the invention is a dimeric cateslytin peptide, preferably a homodimeric cateslytin peptide. Optionally, the monomers of a multimeric cateslytin peptide are linked, preferably covalently linked, through a linker or a spacer.

In still another embodiment, the cateslytin peptide half-life is increased by including the peptide in a biodegradable and biocompatible polymer material for drug delivery system forming microspheres. Polymers and copolymers are, for instance, poly(D,L-lactide-co-glycolide) (PLGA) (as illustrated in US2007/0184015, SoonKap Hahn et al).

The invention also encompasses the pharmaceutically acceptable salts of a cateslytin peptide according to the invention. Pharmaceutically acceptable salts may, for example, be salts of pharmaceutically acceptable mineral acids such as hydrochloric acid, hydrobromic acid, sulphuric acid and phosphoric acid; salts of pharmaceutically acceptable organic acids such as acetic acid, citric acid, maleic acid, malic acid, succinic acid, ascorbic acid and tartaric acid; salts of pharmaceutically acceptable mineral bases such as salts of sodium, potassium, calcium, magnesium or ammonium; or salts of organic bases which contain a salifiable nitrogen, commonly used in pharmaceutical technique. The methods for preparing said salts are well known to one of skill in the art.

In a preferred embodiment, the cateslytin peptide is isolated.

Accordingly, the present invention relates to a molecule comprising a cateslytin peptide according to the present invention as defined above. In particular, the molecule does not comprise the sequence of a catestatin peptide. It also relates to the same uses, kits and methods as disclosed above for the cateslytin peptide according to the present invention but with a molecule comprising a cateslytin peptide according to the present invention.

### Use of the cateslytin peptide

The invention also relates, in a second aspect, to a cateslytin peptide according to the invention for use as a drug.

The invention also relates to the use of a cateslytin peptide according to the invention, for the manufacture of a medecine.

In a preferred embodiment, the invention concerns a cateslytin peptide according to the invention for use in the treatment of an infection.

The invention also concerns a cateslytin peptide according to the invention, for the preparation of a medicament for treating infections in a subject.

The invention further relates to a method for treating in a subject an infection, wherein a therapeutically effective amount of a cateslytin peptide according to the invention, is administered to said subject suffering from an infection.

In still another aspect, the present invention relates to the use of a peptide according to the invention as disinfectant, preservative or pesticide. The term "disinfectant" refers to an antimicrobial activity of the peptide on a surface (for example, walls, doors, medical equipment), a liquid (for example, water) or a gas (for example, an anaesthetic gas). According to one embodiment, the peptide according to the invention is used for elimination of bacterial biofilms. According to a preferred embodiment, the peptide according to the invention is used in particular for disinfecting surgical or prosthetic equipment.

In another aspect, the present invention relates to a medical device or implant comprising a body having at least one surface coated with or including a peptide according to the invention. The present invention also relates to a method for preparing a medical device or implant comprising applying a coating of peptide according to the invention, or placing in contact, with at least one surface of the device or implant.

This type of medical device or implant and the uses and methods of preparation thereof are described for example in patent application WO 2005/006938.

The surface coated with or including a peptide according to the invention may be composed of thermoplastic or polymeric materials such as polyethylene, Dacron, nylon, polyesters, polytetrafluoroethylene, polyurethane, latex, silicone elastomers and the like, or of metallic materials such as gold. In a particular embodiment, the peptide of the invention is covalently attached to a functionalized surface, preferably a metallic surface, via its N-terminal or C-terminal end. Optionally, the peptide may be attached to the surface through a spacer arm.

Preferably, the surface may be coated with a peptide at a density of 0.4 to 300 mg/cm².

Alternatively, the device or implant, in particular bone and joint prosthetic device, may be coated with a cement mixture comprising a peptide according to the invention.

The peptide may be combined with an additionnal active ingredient selected from the group consisting in an antibiotic, an antifungal, an antiviral, an antiparasitic and a combination thereof, preferably an antibiotic or antifungal agent.

The device or implant may be, for example, intravascular, peritoneal, pleural and urological catheters; heart valves; cardiac pacemakers; vascular shunts; coronary stunts; dental implants or orthopaedic or intraocular prosthesis.

Preferably, the infection is selected from the group consisting in bacterial, viral, parasitic and fungal infections. More preferably, the infection is a bacterial or a fungal infection. Even more preferably the infection is a bacterial infection.

The infection according to the invention may be selected from the group consisting in skin infections such as acne, intestinal infections such as esophagitis, gastritis, enteritis, colitis, sigmoiditis, rectitis, and peritonitis, urinary tract infections, vaginal infections, female upper genital tract infections such as salpingitis, endometritis, oophoritis, myometritis, parametritis and infection in the pelvic peritoneum, respiratory tract infections such as pneumonia, intra-amniotic infections, odontogenic infections, endodontic infections, fibrosis, meningitis, bloodstream infections, or a combination thereof.

Preferably, the infection according to the invention is a nosocomial infection. Preferably, the nosocomial infection according to the invention is selected from the group consisting in catheter-related infections, hospital acquired pneumonia such as ventilator associated pneumonia, post-partum infection, hospital acquired gastroenteritis, hospital acquired urinary tract infections, or a combination thereof. Even more preferably the nosocomial infection according to the invention is a catheter-related infection.

Preferably, the infection according to the invention is caused by a microbial agent presenting an antimicrobial resistance. Preferably the antimicrobial resistance is selected from the group consisting in antifungal resistance, antiviral resistance, antiparasitic resistance, antibiotic resistance, and a combination thereof. More preferably, the antimicrobial resistance is an antifungal resistance or an antibiotic resistance. Even more preferably, the antimicrobial resistance is an antibiotic resistance.

The infection according to the invention can be a fungal infection. Preferably, the fungal infection according to the invention is selected from the group consisting in aspergillosis, blastomycosis, candidiasis, coccidioidomycosis, cryptococcosis, histoplasmosis, ucormycosis, paracoccidioidomycosis, sporotrichosis, pneumocystis, and a mixture thereof, more preferably the fungal infection is a candidiasis.

The candidiasis according to the invention may be selected from the group consisting in mucosal, cutaneous, systemic, antibiotic candidiasis, and combination thereof. Preferably, the candidiasis according to the invention is a mucosal candidiasis.

The mucosal candidiasis according to the invention may be selected from the group consisting in oral candidiasis, candidal vulvovaginitis, candidal balanitis, esophageal candidiasis, gastrointestinal candidiasis, respiratory candidiasis, and combination thereof. Preferably, the candidiasis according to the invention is an oral candidiasis.

The oral candidiasis according to the invention may be selected from the group consisting in pseudomembranous candidiasis, erythematous candidiasis, hyperplastic candidiasis, denture-related stomatitis, angular cheilitis, median rhomboid glossitis, and combination thereof.

The fungal infection according to the invention may be caused by a pathogen selected from the group consisting in *Candida* species such as *Candida albicans, Candida parapsilosis, Candida tropicalis, Candida krusei, Candida guillermondii, Candida rugosa, Candida dubliniensis, Candida auris, Candida glabrata, Candida lusitaniae, Candida kefyr, Candida famata, Candida inconspicua,* and *Candida norvegensis*; *Aspergillus species* such as *Aspergillus fumigatus, Aspergillus clavatus,* and *Aspergillus flavus; Cryptococcus* species such as *Cryptococcus neoformans* and *Cryptococcus gattii; Histoplasma* species such as *Histoplasma capsulatu*m*; Pneumocystis* species such as *Pneumocystis jirovecii* and *Pneumocystis carinii*; *Stachybotrys* species such as *Stachybotrys chartarum*; *Blastomyces* species such as *Blastomyces dermatitidis; Coccidioides* species such as *Coccidioides immitis and Coccidioides posadasii; Mucorales* species such as *Rhizopus oryzae, Rhizomucor, Absidia, Lichtheimia corymbifera, Syncephalastrum racemosum, Apophysomyces variabilis* and *Mucor indicus; Paracoccidioides* species such as *Paracoccidioides brasiliensis; Sporothrix* species such as *Sporothrix schenckii*; *Epidermophyton* species such as *Epidermophyton floccosum; Microsporum* species such as *Microsporum canis* and *Microsporum audouinii*; *Trichophyton* species such as *Trichophyton interdigitale* (or *mentagrophytes*), *Trichophyton tonsurans, Trichophyton schoenleini, Trichophyton rubrum,* and *Trichophyton verrucosum*; *Hortaea* species such as *Hortaea werneckii*; *Penicillium* species such as *Penicillium marneffei*; *Piedraia* species such as *Penicillium hortae*; *Malassezia* species such as *Malassezia furfur; Lacazia* species such as *Lacazia loboi*; *Exophiala* species such as *Exophiala jeanselmei*; *Fonsecaea* species such as *Fonsecaea pedrosoi* and *Fonsecaea compacta*; *Phialophora* species such as *Phialophora verrucosa*; *Basidiobolus* species such as *Basidiobolus ranarum*; *Conidiobolus* species such as *Conidiobolus coronatus* and *Conidiobolus incongruus; Enterocytozoon* species such as *Enterocytozoon bieneusi* and *Encephalitozoon intestinalis*; *Rhinosporidium* species such as *Rhinosporidium seeberi; Geotrichum* species such as *Geotrichum candidum*; *Pseudallescheria* species such as *Pseudallescheria boydii; Trichosporon* species; *Torulopsis glabrata,* or a mixture thereof.

Preferably the fungal infection according to the invention is caused by a *Candida species* selected from the group consisting in *Candida albicans, Candida parapsilosis, Candida tropicalis, Candida krusei, Candida guillermondii, Candida rugosa, Candida dubliniensis, Candida auris, Candida glabrata, Candida lusitaniae, Candida kefyr, Candida famata, Candida inconspicua,* and *Candida norvegensis.* Even more preferably the fungal infection is caused by *Candida albicans.*

Preferably, the fungal infection according to the invention is caused by a yeast.

The infection according to the invention is preferably a bacterial infection. Preferably, the bacterial infection according to the invention is caused by a bacteria selected from the group consisting in *Escherichia coli, Escherichia coli Amp^{R}, Escherichia coli Kan^{R}, Escherichia coli Amp^{R} Kan^{R}, Escherichia coli Kan^{R} Chlo^{R}, Escherichia coli Amp^{R} Chlo^{R}, Escherichia coli Amp^{R} Kan^{R} Chlo^{R}, Escherichia coli AmpC, Escherichia coli ESBL, Escherichia coli OXA48, Staphylococcus aureus MSSA, Staphylococcus aureus MRSA, Klebsiella pneumoniae, Klebsiella pneumoniae ESBL, Klebsiella pneumoniae KPC, Enterobacter cloacae, Enterobacter cloacae ESBL, Enterobacter cloacae AmpC, Enterobacter cloacae OXA48, Enterobacter aerogenes, Enterobacter aerogenes ESBL, Enterobacter aerogenes AmpC, Serratia marcescens, Serratia marcescens AmpC, Morganella morganii, Morganella morganii AmpC, Citrobacter freundii, Citrobacter freundii AmpC, Pseudomonas aerigunosa, Pseudomonas aerigunosa AmpC, Pseudomonas aerigunosa VIM, Parvimonas micra, Prevotella intermedia, Fusobacterium nucleatum, Enterococcus faecalis, Prevotella nigrescens, Actinomyces israelii, Porphyromonas endodontalis, Porphyromonas gingivalis Micrococcus luteus, Bacillus megaterium, Actinomyces israelii, Aeromonas hydrophila, Aeromonas caviae, Bacillus anthracis, Bacillus cereus, Bacteroides fragilis, Bartonella henselae, Bartonella Quintana, Bordetella pertussis, Borrelia burgdorferi, Borrelia garinii, Borrelia afzelii, Borrelia recurrentis, Brucella abortus, Brucella canis, Brucella melitensis, Brucella suis, Campylobacter jejuni, Campylobacter coli, Campylobacter fetus, Chlamydia pneumoniae, Chlamydia trachomatis, Chlamydophila psittaci, Clostridium botulinum, Clostridium difficile, Clostridium perfringens, Clostridium tetani, Corynebacterium diphtheria, Ehrlichia canis, Ehrlichia chaffeensis, Enterococcus faecium, Francisella tularensis, Haemophilus influenza, Helicobacter pylori, Legionella pneumophila, Leptospira interrogans, Leptospira santarosai, Leptospira weilii, Leptospira noguchii, Listeria monocytogenes, Mycobacterium leprae, Mycobacterium tuberculosis, Mycobacterium ulcerans, Mycoplasma pneumonia, Neisseria gonorrhoeae, Neisseria meningitides, Nocardia asteroids, Rickettsia rickettsia, Salmonella enteritidis, Salmonella typhi, Salmonella paratyphi, Salmonella typhimurium, Shigella sonnei, Shigella flexnerii, Shigella dysenteriae, Staphylococcus epidermidis, Staphylococcus saprophyticus, Streptococcus agalactiae, Streptococcus pneumoniae, Streptococcus pyogenes, Streptococcus viridans, Treponema pallidum, Ureaplasma urealyticum, Vibrio cholera, Vibrio parahaemolyticus, Yersinia pestis, Yersinia enterocolitica, Yersinia pseudotuberculosis,* and combination thereof.

More preferably, the bacterial infection according to the invention is caused by a bacteria selected from the group consisting in *Escherichia coli, Escherichia coli Amp^{R} Kan^{R} Chlo^{R}, Escherichia coli AmpC, Escherichia coli ESBL, Escherichia coli OXA48, Staphylococcus aureus MSSA, Staphylococcus aureus MRSA, Klebsiella pneumoniae, Klebsiella pneumoniae ESBL, Klebsiella pneumoniae KPC, Enterobacter cloacae, Enterobacter cloacae ESBL, Enterobacter cloacae AmpC, Enterobacter cloacae OXA48, Enterobacter aerogenes, Enterobacter aerogenes ESBL, Enterobacter aerogenes AmpC, Serratia marcescens, Serratia marcescens AmpC, Morganella morganii, Morganella morganii AmpC, Citrobacter freundii, Citrobacter freundii AmpC, Pseudomonas aerigunosa, Pseudomonas aerigunosa AmpC, Pseudomonas aerigunosa VIM, Parvimonas micra, Prevotella intermedia, Fusobacterium nucleatum, Enterococcus faecalis* and combination thereof, preferably the bacteria is selected from the group consisting in *Escherichia coli, Escherichia coli Amp^{R} Kan^{R}, MSSA Staphylococcus aureus, MRSA Staphylococcus aureus, Parvimonas micra, Prevotella intermedia, Fusobacterium nucleatum, Enterococcus faecalis,* and combination thereof.

Even more preferably, the bacterial infection according to the invention is caused by *Escherichia coli or Escherichia coli Amp^{R} Kan^{R} Chlo^{R}.*

In a particular embodiment, the bacterial infection according to the invention is caused by a bacteria presenting an antibiotic resistance, preferably an antibiotic resistance selected from the group consisting in Amp^{R}, Kan^{R} *Chlo^{R},* AmpC, ESBL, methicillin resistance, OXA48, KPC, VIM, and a combination thereof.

### Pharmaceutical composition

In a third aspect, the invention also relates to a pharmaceutical composition comprising a cateslytin peptide according to the invention. The pharmaceutical composition comprises at least one excipient or pharmaceutically acceptable carrier.

Preferably, the pharmaceutical composition further comprises an active ingredient selected from the group consisting in an antibiotic, an antiviral, an antiparasitic, an antifungal and a combination thereof. More preferably, the pharmaceutical composition further comprises an antibiotic and/or an antifungal. Even more preferably, the pharmaceutical composition further comprises an antibiotic.

In a particular embodiment, the pharmaceutical composition according to the invention further comprises an antifungal.

Preferably, the antifungal is selected from the group consisting in polyenes such as amphotericin B, candicidin, filipin, hamycin, natamycin, nystatin and rimocidin; imidazoles such as bifonazole, butoconazole, clotrimazole, econazole, fenticonazole, isoconazole, ketoconazole, luliconazole, miconazole, omoconazole, oxiconazole, sertaconazole, sulconazole, tioconazole, and spectrazole; triazolles such as albaconazole, efinaconazole, epoxiconazole, fluconazole, isavuconazole, itraconazole, posaconazole, propiconazole, ravuconazole, terconazole, and voriconazole; thiazoles such as abafungin; allylamines such as amorolfin, butenafine, naftifine, and terbinafine; echinocandins such as anidulafungin, caspofungin, and micafungin; benzoic acid, ciclopirox, flucytosine, 5-fluorocytosine, griseofulvin, haloprogin, tolnaftate, undecylenic acid, derivatives and combinations thereof.

More preferably, the antifungal of the invention is selected from the group consisting in triazolles such as albaconazole, efinaconazole, epoxiconazole, fluconazole, isavuconazole, itraconazole, posaconazole, propiconazole, ravuconazole, terconazole, voriconazole, or combination thereof.

Still preferably the antifungal according to the invention is the voriconazole or the fluconazole. Even more preferably, the antifungal according to the invention is the voriconazole.

In a preferred embodiment, the pharmaceutical composition further comprise an antibiotic.

Preferably, the antibiotic according to the invention is selected from the group consisting in penicillins such as penicillin G, penicillin K, penicillin N, penicillin O, penicillin V, methicillin, benzylpenicillin, nafcillin, oxacillin, cloxacillin, dicloxacillin, ampicillin, amoxicillin, pivampicillin, hetacillin, bacampicillin, metampicillin, talampicillin, epicillin, carbenicillin, ticarcillin, temocillin, mezlocillin, and piperacillin; cephalosporins such as cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefonicid, cefprozil, cefuroxime, cefuzonam, cefmetazole, cefotetan, cefoxitin, loracarbef, cefbuperazone, cefminox, cefotetan, cefoxitin, cefotiam, cefcapene, cefdaloxime, cefdinir, cefditoren, cefetamet, cefixime, cefmenoxime, cefodizime, cefotaxime, cefovecin, cefpimizole, cefpodoxime, cefteram, ceftamere, ceftibuten, ceftiofur, ceftiolene, ceftizoxime, ceftriaxone, cefoperazone, ceftazidime, latamoxef, cefclidine, cefepime, cefluprenam, cefoselis, cefozopran, cefpirome, cefquinome, flomoxef, ceftobiprole, ceftaroline, ceftolozane, cefaloram, cefaparole, cefcanel, cefedrolor, cefempidone, cefetrizole, cefivitril, cefmatilen, cefmepidium, cefoxazole, cefrotil, cefsumide, ceftioxide, cefuracetime, and nitrocefin; polymyxins such as polysporin, neosporin, polymyxin B, and polymyxin E, rifampicins such as rifampicin, rifapentine, and rifaximin; Fidaxomicin; quinolones such as cinoxacin, nalidixic acid, oxolinic acid, piromidic acid, pipemidic acid, rosoxacin, ciprofloxacin, enoxacin, fleroxacin, lomefloxacin, nadifloxacin, norfloxacin, ofloxacin, pefloxacin, rufloxacin, balofloxacin, grepafloxacin, levofloxacin, pazufloxacin, temafloxacin, tosufloxacin, clinafloxacin, gatifloxacin, gemifloxacin, moxifloxacin, sitafloxacin, trovafloxacin, prulifloxacin, delafloxacin, nemonoxacin, and zabofloxacin; sulfonamides such as sulfafurazole, sulfacetamide, sulfadiazine, sulfadimidine, sulfafurazole, sulfisomidine, sulfadoxine, sulfamethoxazole, sulfamoxole, sulfanitran, sulfadimethoxine, sulfametho-xypyridazine, sulfametoxydiazine, sulfadoxine, sulfametopyrazine, and terephtyl; macrolides such as azithromycin, clarithromycin, erythromycin, fidaxomicin, telithromycin, carbomycin A, josamycin, kitasamycin, midecamycin, oleandomycin, solithromycin, spiramycin, troleandomycin, tylosin, and roxithromycin; ketolides such as telithromycin, and cethromycin; lluoroketolides such as solithromycin; lincosamides such as lincomycin, clindamycin, and pirlimycin; tetracyclines such as demeclocycline, doxycycline, minocycline, oxytetracycline, and tetracycline; aminoglycosides such as amikacin, dibekacin, gentamicin, kanamycin, neomycin, netilmicin, sisomicin, tobramycin, paromomycin, and streptomycin; ansamycins such as geldanamycin, herbimycin, and rifaximin; carbacephems such as loracarbef; carbapenems such as ertapenem, doripenem, imipenem (or cilastatin), and meropenem; glycopeptides such as teicoplanin, vancomycin, telavancin, dalbavancin, and oritavancin; lincosamides such as clindamycin and lincomycin; lipopeptides such as daptomycin; monobactams such as aztreonam; nitrofurans such as furazolidone, and nitrofurantoin; oxazolidinones such as linezolid, posizolid, radezolid, and torezolid; teixobactin, clofazimine, dapsone, capreomycin, cycloserine, ethambutol, ethionamide, isoniazid, pyrazinamide, rifabutin, arsphenamine,chloramphenicol, fosfomycin, fusidic acid, metronidazole, mupirocin, platensimycin, quinupristin (or dalfopristin), thiamphenicol, tigecycline, tinidazole, trimethoprim, alatrofloxacin, fidaxomycin, nalidixice acide, rifampin, derivatives and combination thereof.

More preferably the antibiotic according to the invention is selected from the group consisting in cefotaxim, methicillin, vancomycin, amoxicillin, derivatives and combination thereof.

The invention also concerns the pharmaceutical composition of the invention for use in the treatment of an infection.

The invention also relates to the use of a pharmaceutical composition according to the invention for the manufacture of a medecine for treating infections in a subject.

The invention further relates to a method for treating in a subject an infection, wherein a therapeutically effective amount of a pharmaceutical composition according to the invention is administered to said subject suffering from an infection.

Preferably, the infection is as described above for the use of the cateslytin peptide. Preferably, the infection is selected from the group consisting in bacterial, viral, parasitic and fungal infections. More preferably, the infection is a bacterial or a fungal infection. Even more preferably the infection is a bacterial infection.

The invention also relates to a pharmaceutical composition comprising a cateslytin peptide according to the invention and cefotaxime for use in the treatment of an infection, preferably an infection caused by *Escherichia coli Amp^{R} Kan^{R} Chlo^{R}.*

The invention also relates to the use of a pharmaceutical composition comprising a cateslytin peptide according to the invention and cefotaxime for the manufacture of a medecine for treating infections in a subject, preferably an infection caused by *Escherichia coli Amp^{R} Kan^{R} Chlo^{R}.*

The invention further relates to a method for treating in a subject an infection, preferably an infection caused by *Escherichia coli Amp^{R} Kan^{R} Chlo^{R},* wherein a therapeutically effective amount of a pharmaceutical composition comprising a cateslytin peptide according to the invention and cefotaxime is administered to said subject suffering from an infection, preferably an infection caused by *Escherichia coli Amp^{R} Kan^{R} Chlo^{R}.*

The invention also relates to a pharmaceutical composition comprising a cateslytin peptide according to the invention and methicillin for use in the treatment of an infection, preferably an infection caused by *MSSA Staphylococcus aureus.*

The invention also relates to the use of a pharmaceutical composition comprising a cateslytin peptide according to the invention and methicillin for the manufacture of a medecine for treating infections in a subject, preferably an infection caused by *MSSA Staphylococcus aureus.*

The invention further relates to a method for treating in a subject an infection, preferably an infection caused by *MSSA Staphylococcus aureus,* wherein a therapeutically effective amount of a pharmaceutical composition comprising a cateslytin peptide according to the invention and methicillin is administered to said subject suffering from an infection, preferably an infection caused by *MSSA Staphylococcus aureus.*

The invention also relates to a pharmaceutical composition comprising a cateslytin peptide according to the invention and vancomycin for use in the treatment of an infection, preferably an infection caused by *MRSA Staphylococcus aureus.*

The invention also relates to the use of a pharmaceutical composition comprising a cateslytin peptide according to the invention and vancomycin for the manufacture of a medecine for treating infections in a subject, preferably an infection caused by *MRSA Staphylococcus aureus.*

The invention further relates to a method for treating in a subject an infection, preferably an infection caused by *MRSA Staphylococcus aureus,* wherein a therapeutically effective amount of a pharmaceutical composition comprising a cateslytin peptide according to the invention and vancomycin is administered to said subject suffering from an infection, preferably an infection caused by *MRSA Staphylococcus aureus.*

The invention also relates to a pharmaceutical composition comprising a cateslytin peptide according to the invention and amoxicillin for use in the treatment of an infection, preferably an infection caused by a bacteria selected from the group consisting in *Parvimonas micra*, *Prevotella intermedia, Fusobacterium nucleatum,* and a combination thereof.

The invention also relates to the use of a pharmaceutical composition comprising a cateslytin peptide according to the invention and amoxicillin for the manufacture of a medecine for treating infections in a subject, preferably an infection caused by a bacteria selected from the group consisting in *Parvimonas micra, Prevotella intermedia*, *Fusobacterium nucleatum.*

The invention further relates to a method for treating in a subject an infection, preferably an infection caused by a bacteria selected from the group consisting in *Parvimonas micra, Prevotella intermedia, Fusobacterium nucleatum,* wherein a therapeutically effective amount of a pharmaceutical composition comprising a cateslytin peptide according to the invention and amoxicillin is administered to said subject suffering from an infection, preferably an infection caused by a bacteria selected from the group consisting in *Parvimonas micra, Prevotella intermedia*, *Fusobacterium nucleatum.*

The invention also relates to a pharmaceutical composition comprising a cateslytin peptide according to the invention and calcium hydroxyde for use in the treatment of an infection, preferably an endodontic infection, more preferably an endodontic infection caused by a pathogen selected from the group consisting in *Enterococcus faecalis, Parvimonas micra, Prevotella intermedia, Fusobacterium nucleatum, Candida albicans,* and a combination thereof, even more preferably an endodontic infection caused by *Enterococcus faecalis.*

The invention also relates to the use of a pharmaceutical composition comprising a cateslytin peptide according to the invention and calcium hydroxyde for the manufacture of a medecine for treating infections in a subject, preferably an endodontic infection, more preferably an endodontic infection caused by a pathogen selected from the group consisting in *Enterococcus faecalis, Parvimonas micra, Prevotella intermedia, Fusobacterium nucleatum, Candida albicans,* and a combination thereof, even more preferably an endodontic infection caused by *Enterococcus faecalis.*

The invention further relates to a method for treating in a subject an infection, preferably an endodontic infection, more preferably an endodontic infection caused by a pathogen selected from the group consisting in *Enterococcus faecalis, Parvimonas micra, Prevotella intermedia, Fusobacterium nucleatum, Candida albicans,* and a combination thereof, even more preferably an endodontic infection caused by *Enterococcus faecalis,* wherein a therapeutically effective amount of a pharmaceutical composition comprising a cateslytin peptide according to the invention and calcium hydroxyde is administered to said subject suffering from an infection, preferably an infection caused by a bacteria selected from the group consisting in *Enterococcus faecalis, Parvimonas micra*, *Prevotella intermedia, Fusobacterium nucleatum, Candida albicans.*

The invention also relates to a pharmaceutical composition comprising a cateslytin peptide according to the invention and voriconazole or fluconazole, preferably voriconazole, for use in the treatment of an infection, preferably a fungal infection, more preferably a candidiasis, still preferably a candidiasis caused by *Candida albicans,* even more preferably an oral candidiasis caused by *Candida albicans.*

The invention also relates to the use of a pharmaceutical composition comprising a cateslytin peptide according to the invention and voriconazole or fluconazole, preferably voriconazole, for the manufacture of a medecine for treating infections in a subject, preferably a fungal infection, more preferably a candidiasis, still preferably a candidiasis caused by *Candida albicans,* even more preferably an oral candidiasis caused by *Candida albicans.*

The invention further relates to a method for treating in a subject an infection, preferably a fungal infection, more preferably a candidiasis, still preferably a candidiasis caused by *Candida albicans,* even more preferably an oral candidiasis caused by *Candida albicans,* wherein a therapeutically effective amount of a pharmaceutical composition comprising a cateslytin peptide according to the invention and voriconazole or fluconazole, preferably voriconazole, is administered to said subject suffering from an infection, preferably a fungal infection, more preferably a candidiasis, still preferably a candidiasis caused by *Candida albicans,* even more preferably an oral candidiasis caused by *Candida albicans.*

### Product or kit

In a forth aspect, the invention also concerns a product or kit comprising a) a cateslytin peptide according to the invention and b) an active ingredient selected from the group consisting in an antibiotic, an antifungal, an antiparasitic, an antiviral, and a combination thereof, as a combined preparation for simultaneous, separate or sequential use.

Preferably the active ingredient is an antibiotic or an antifungal. More preferably, the active ingredient is an antibiotic.

Preferably, the antifungal and the antibiotic are as described above for the pharmaceutical composition.

The invention also concerns the product or kit according to the invention for use in the treatment of an infection.

The invention also relates to the use of a product or kit according to the invention for the manufacture of a medecine for treating infections in a subject.

The invention further relates to a method for treating in a subject an infection, wherein a therapeutically effective amount of a product or kit according to the invention is administered to said subject suffering from an infection.

Preferably, the infection is as described above for the use of the cateslytin peptide. Preferably, the infection is selected from the group consisting in bacterial, viral, parasitic and fungal infections. More preferably, the infection is a bacterial or a fungal infection. Even more preferably the infection is a bacterial infection.

The invention also relates to a product or kit comprising a cateslytin peptide according to the invention and cefotaxime for use in the treatment of an infection, preferably an infection caused by *Escherichia coli Amp^{R} Kan^{R} Chlo^{R}.*

The invention also relates to the use of a product or kit comprising a cateslytin peptide according to the invention and cefotaxime for the manufacture of a medecine for treating infections in a subject, preferably an infection caused by *Escherichia coli Amp^{R} Kan^{R} Chlo^{R}.*

The invention further relates to a method for treating in a subject an infection, preferably an infection caused by *Escherichia coli Amp^{R} Kan^{R} Chlo^{R},* wherein a therapeutically effective amount of a product or kit comprising a cateslytin peptide according to the invention and cefotaxime is administered to said subject suffering from an infection, preferably an infection caused by *Escherichia coli Amp^{R} Kan^{R} Chlo^{R}.*

The invention also relates to a product or kit comprising a cateslytin peptide according to the invention and methicillin for use in the treatment of an infection, preferably an infection caused by *MSSA Staphylococcus aureus.*

The invention also relates to the use of a product or kit comprising a cateslytin peptide according to the invention and methicillin for the manufacture of a medecine for treating infections in a subject, preferably an infection caused by *MSSA Staphylococcus aureus.*

The invention further relates to a method for treating in a subject an infection, preferably an infection caused by *MSSA Staphylococcus aureus,* wherein a therapeutically effective amount of a a product or kit comprising a cateslytin peptide according to the invention and methicillin is administered to said subject suffering from an infection, preferably an infection caused by *MSSA Staphylococcus aureus.*

The invention also relates to a product or kit comprising a cateslytin peptide according to the invention and vancomycin for use in the treatment of an infection, preferably an infection caused by *MRSA Staphylococcus aureus.*

The invention also relates to the use of a product or kit comprising a cateslytin peptide according to the invention and vancomycin for the manufacture of a medecine for treating infections in a subject, preferably an infection caused by *MRSA Staphylococcus aureus.*

The invention further relates to a method for treating in a subject an infection, preferably an infection caused by *MRSA Staphylococcus aureus,* wherein a therapeutically effective amount of a product or kit comprising a cateslytin peptide according to the invention and vancomycin is administered to said subject suffering from an infection, preferably an infection caused by *MRSA Staphylococcus aureus.*

The invention also relates to a product or kit comprising a cateslytin peptide according to the invention and amoxicillin for use in the treatment of an infection, preferably an infection caused by a bacteria selected from the group consisting in *Parvimonas micra, Prevotella intermedia, Fusobacterium nucleatum,* and a combination thereof.

The invention also relates to the use of a product or kit comprising a cateslytin peptide according to the invention and amoxicillin for the manufacture of a medecine for treating infections in a subject, preferably an infection caused by a bacteria selected from the group consisting in *Parvimonas micra, Prevotella intermedia*, *Fusobacterium nucleatum,* and a combination thereof.

The invention further relates to a method for treating in a subject an infection, preferably an infection caused by a bacteria selected from the group consisting in *Parvimonas micra*, *Prevotella intermedia, Fusobacterium nucleatum,* and a combination thereof, wherein a therapeutically effective amount of a product or kit comprising a cateslytin peptide according to the invention and amoxicillin is administered to said subject suffering from an infection, preferably an infection caused by preferably an infection caused by a bacteria selected from the group consisting in *Parvimonas micra, Prevotella intermedia*, *Fusobacterium nucleatum,* and a combination thereof.

The invention also relates to a product or kit comprising a cateslytin peptide according to the invention and calcium hydroxyde for use in the treatment of an infection, preferably an endodontic infection, more preferably an endodontic infection caused by a pathogen selected from the group consisting in *Enterococcus faecalis, Parvimonas micra*, *Prevotella intermedia, Fusobacterium nucleatum, Candida albicans,* and a combination thereof, even more preferably an endodontic infection caused by *Enterococcus faecalis.*

The invention also relates to the use of a product or kit comprising a cateslytin peptide according to the invention and calcium hydroxyde for the manufacture of a medecine for treating infections in a subject, preferably an endodontic infection, more preferably an endodontic infection caused by a pathogen selected from the group consisting in *Enterococcus faecalis, Parvimonas micra, Prevotella intermedia, Fusobacterium nucleatum, Candida albicans,* and a combination thereof, even more preferably an endodontic infection caused by *Enterococcus faecalis.*

The invention further relates to a method for treating in a subject an infection, preferably an endodontic infection, more preferably an endodontic infection caused by a pathogen selected from the group consisting in *Enterococcus faecalis, Parvimonas micra, Prevotella intermedia, Fusobacterium nucleatum, Candida albicans,* and a combination thereof, even more preferably an endodontic infection caused by *Enterococcus faecalis,* wherein a therapeutically effective amount of a product or kit comprising a cateslytin peptide according to the invention and calcium hydroxyde is administered to said subject suffering from an infection, preferably an endodontic infection, more preferably an endodontic infection caused by a pathogen selected from the group consisting in *Enterococcus faecalis, Parvimonas micra, Prevotella intermedia, Fusobacterium nucleatum, Candida albicans,* and a combination thereof, even more preferably an endodontic infection caused by *Enterococcus faecalis.*

The invention also relates to a product or kit comprising a cateslytin peptide according to the invention and voriconazole for use in the treatment of an infection, preferably a fungal infection, more preferably a candidiasis, still preferably a candidiasis caused by *Candida albicans,* even more preferably an oral candidiasis caused by *Candida albicans.*

The invention also relates to the use of a product or kit comprising a cateslytin peptide according to the invention and voriconazole for the manufacture of a medecine for treating infections in a subject, preferably a fungal infection, more preferably a candidiasis, still preferably a candidiasis caused by *Candida albicans,* even more preferably an oral candidiasis caused by *Candida albicans*

The invention further relates to a method for treating in a subject an infection, preferably a fungal infection, more preferably a candidiasis, still preferably a candidiasis caused by *Candida albicans,* even more preferably an oral candidiasis caused by *Candida albicans,* wherein a therapeutically effective amount of a product or kit comprising a cateslytin peptide according to the invention and voriconazole is administered to said subject suffering from an infection, preferably a fungal infection, more preferably a candidiasis, still preferably a candidiasis caused by *Candida albicans,* even more preferably an oral candidiasis caused by *Candida albicans.*

### Subject, regimen and administration

The subject according to the invention is an animal, preferably a mammal, even more preferably a human. However, the term "subject" can also refer to non-human animals, in particular mammals such as dogs, cats, horses, cows, pigs, sheep, donkeys, rabbits, ferrets, gerbils, hamsters, chinchillas, rats, mice, guinea pigs and non-human primates, among others, that are in need of treatment.

The human subject according to the invention may be a human at the prenatal stage, a new-born, a child, an infant, an adolescent or an adult, in particular an adult of at least 40 years old, preferably an adult of at least 50 years old, still more preferably an adult of at least 60 years old, even more preferably an adult of at least 70 years old.

In a preferred embodiment, the subject has been diagnosed with an infection. Preferably, the subject has been diagnosed with an infection selected from the group consisting in bacterial, viral, parasitic and fungal infections. More preferably, the subject has been diagnosed with a bacterial or a fungal infection. Even more preferably the subject has been diagnosed with a bacterial infection. Diagnostic method of infections are well known by the man skilled in the art.

In a particular embodiment, the infection present a resistance to treatment. Preferably, the infection present an antibiotic or an antifungal resistance, more preferably an antibiotic resistance.

In another particular embodiment, the subject has a wound in need of cicatrization.

In a particular embodiment, the subject has already received at least one line of treatment, preferably several lines of treatment, prior to the administration of a cateslytin peptide according to the invention, of a pharmaceutical composition according to the invention, or of a product or kit according to the invention.

The cateslytin peptide according to the invention, the pharmaceutical composition according to the invention, or the product or kit according to the invention may be administered by any conventional route of administration.

In particular, cateslytin peptide according to the invention, the pharmaceutical composition according to the invention, or the product or kit according to the invention can be formulated for a topical, enteral, oral, parenteral, intranasal, intravenous, intramuscular, subcutaneous or intraocular administration and the like.

Preferably, the cateslytin peptide according to the invention, the pharmaceutical composition according to the invention, or the product or kit according to the invention may be administered by enteral or parenteral route of administration. When administered parenterally, the cateslytin peptide according to the invention, the pharmaceutical composition according to the invention, or the product or kit according to the invention is preferably administered by intravenous route of administration. When administered enterally, the cateslytin peptide according to the invention, the pharmaceutical composition according to the invention, or the product or kit according to the invention is preferably administered by oral route of administration.

The pharmaceutical composition comprising the molecule is formulated in accordance with standard pharmaceutical practice (Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the invention may be formulated to release the active drug substantially immediately upon administration or at any predetermined time or time period after administration.

The pharmaceutical composition according to the invention may be a cicatrizing composition. Such a cicatrizing composition can be directly applied on a wound, i.e. through a topical route of administration.

The cicatrizing composition according to the invention may be selected from the group consisting in a cream, a gel, an emulsion, a solution, a polymer, an ointment, or a spray.

The cicatrizing composition according to the invention may also be part of a plaster.

The invention also concerns the cateslytin peptide according to the invention, or the cicatrizing composition according to the invention, for use in the treatment of a wound.

The invention also relates to a cateslytin peptide according to the invention, or to a cicatrizing composition according to the invention for the preparation of a medicament or of a medical device for cicatrizing a wound in a subject.

The invention further relates to a method for cicatrizing a wound in a subject, wherein a therapeutically effective amount of a cateslytin peptide according to the invention, or of a therapeutically effective amount of a cicatrizing composition according to the invention is administered to said subject suffering from an infection.

Preferably, the treatment with the cateslytin peptide according to the invention, the pharmaceutical composition according to the invention, or the product or kit according to the invention start no longer than a month, preferably no longer than a week, after the diagnosis of the infection. In a most preferred embodiment, the treatment start the day of the diagnosis.

The cateslytin peptide according to the invention, the pharmaceutical composition according to the invention, or the product or kit according to the invention may be administered as a single dose or in multiple doses.

Preferably, the treatment is administered regularly, preferably between every day and every month, more preferably between every day and every two weeks, more preferably between every day and every week, even more preferably the treatment is administered every day. In a particular embodiment, the treatment is administered several times a day, preferably 2 or 3 times a day, even more preferably 3 times a day.

The duration of treatment with the cateslytin peptide according to the invention, the pharmaceutical composition according to the invention, or the product or kit according to the invention is preferably comprised between 1 day and 20 weeks, more preferably between 1 day and 10 weeks, still more prefererably between 1 day and 4 weeks, even more preferably between 1 day and 2 weeks. In a particular embodiment, the duration of the treatment is of about 1 week. Alternatively, the treatment may last as long as the infection persists, and/or as long as the wound is not fully cicatrized.

The amount of cateslytin peptide according to the invention, of pharmaceutical composition according to the invention, or of the product or kit according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

In a preferred embodiment, the total cateslytin peptide dose for each administration of the cateslytin peptide according to the invention, the pharmaceutical composition according to the invention, or the product or kit according to the invention is comprised between 0.01 µg and 1 g, preferably between 0.1 µg and 10 mg, even more preferably between 1 µg and 1 mg.

When the cateslytin peptide according to the invention is not the only active ingredient administered to the patient, i.e. in the case of a pharmaceutical composition according to the invention which further comprise another active ingredient, or in the case of a product or kit according to the invention, the cateslytin peptide according to the invention and the other active ingredient are preferably administered at doses allowing to obtain a therapeutic effect, preferably a synergistic effect as illustrated in the examples herein after. To obtain such an effect, the cateslytin peptide according to the invention and/or the other active ingredient can be administered at therapeutically effective doses or subtherapeuthic doses.

In a preferred embodiment, the cateslytin peptide according to the invention and the other active ingredient are both administered at therapeutically effective doses.

In another preferred embodiment, the cateslytin peptide according to the invention is administered at a therapeutically effective dose and the other active ingredient is administered at a subtherapeutic dose.

In another preferred embodiment, the cateslytin peptide according to the invention is administered at a subtherapeutic dose and the other active ingredient is administered at a therapeutically effective dose.

In yet another preferred embodiment, the cateslytin peptide according to the invention and the other active ingredient are both administered at subtherapeutic doses.

A therapeutic dose of cateslytin peptide according to the invention is comprised between 0.01 µg and 1 g, preferably between 0.1 µg and 10 mg, even more preferably between 1 µg and 1 mg for each administration.

A subtherapeutic dose may also be defined as 90, 80, 70, 60, 50, 40, 30, 20 or 10% of the conventional dose.

In a product or kit according to the invention, the active ingredients of the combined preparation can be administered simultaneously, separately or sequentially, or a combination thereof when the combined preparation comprises more than two active ingredients.

When the active ingredients of the combined preparation are administered separately or sequentially, the time intervals between the different administrations are preferably selected so as to allow a therapeutic effect, preferably a synergetic effect.

The active ingredients of the combined preparation can be administered to the subject by the same or different routes of administration. Administration routes usually depend on the pharmaceutical compositions used.

The form of the pharmaceutical compositions, the route of administration and the dose of administration of the cateslytin peptide according to the invention, the pharmaceutical composition according to the invention, or the product or kit according to the invention can be adjusted by the man skilled in the art according to the type and severity of the infection, and to the patient, in particular its age, weight, sex, and general physical condition.

### Kit and use of a kit

The invention also concerns a kit for the treatment of an infection in a subject, wherein the kit comprises a cateslytin peptide according to the invention or a pharmaceutical composition according to the invention and optionally a leaflet providing guidelines to use such a kit. Preferably, the kit further comprises another active ingredient selected from the group constiting in an antifungal, an antibiotic, an antiviral, an antiparasitic, and a combination thereof, preferably an antifungal or an antibiotic, more preferably an antibiotic.

The invention also concerns the use of a kit as described above in the treatment of an infection, preferably an infection selected from the group consisting in bacterial, viral, and fungal infections, even more preferably a bacterial infection, in a patient in needs thereof, in a subject in need thereof.

Preferably, the subject is a human.

All the references cited in this application, including scientific articles and summaries, published patent applications, granted patents or any other reference, are entirely incorporated herein by reference, which includes all the results, tables, figures and texts of theses references.

Although having different meanings, the terms "comprising", "having", "consisting in" and "containing" can be replaced one for the other in the entire application.

Further aspects and advantages of the present invention will be described in the following examples, which should be regarded as illustrative and not limiting.

### Examples

### Example 1: D-Cateslytin peptide, a new antibiotic agent and antibiotic potentiator

### Material and Methods

### Peptide synthesis

The chemically synthesized peptides corresponding to L-cateslytin and D-cateslytin (RSMRLSFRARGYGFR, SEQ ID No 1) were commercially obtained (Proteogenix).

### Microorganisms and mammalian cell cultivation

*Escherischia coli* (ATCC® 25922™), *Staphylococcus aureus* (ATCC® 25923™), *Fusobacterium nucleatum* (ATCC® 49256™), *Prevotella intermedia* (ATCC® 49046™), *Parvimonas micra* (ATCC® 33270™) were purchased from ATCC. *E. coli* K-12 mutant E2146 was kindly provided by the Institut Pasteur of Paris. This multi-resistant strain was constructed from *E. coli* MG1655 (*E. coli* genetic stock center CGSC#6300). It is resistant to specific antibiotics, i.e. ampicillin, chloramphenicol, and kanamycin. The *S. aureus* Methicillin Resistant (MRSA) S1 strain was kindly provided by Dr Gilles Prévost (University of Strasbourg) (Aslam et al., PLoS One. 2013;8(7):e68993). Microorganisms were cultured according to the manufacturer's or the owner's instructions in their respective media: Luria Bertani broth (Sigma) was used for *E. coli* strains, Mueller Hinton broth (Difco) for *S. aureus* strains and Anaerobe Basal broth (Oxoid) for *F. nucleatum, P. intermedia* and *P. micra.*

The Caco-2 cell line (ATCC® HTB-37™) was kindly provided by Dr Benoit Frisch (UMR 7199 CNRS University of Strasbourg). All cell lines were cultured at 37°C in a 5% CO₂ humidified incubator in their respective media: Dulbecco's Modified Eagle's Medium (Thermo Fisher Scientific) supplemented with 10% bovine calf serum and 1% penicillin-streptomycin 100X (Thermo Fisher Scientific) for HGF-1, Eagle's Minimum Essential Medium (Thermo Fisher Scientific) supplemented with 20% bovine calf serum and 1% penicillin/streptomycin for Caco-2.

Human Peripheral Blood Mononuclear Cells (PBMC) were obtained from healthy volunteers and isolated by density gradient centrifugation using Lymphoprep™ (Stemcell Technologies). PMBC were then maintained in AIM V® medium (Thermo Fisher Scientific) at 37°C in a 5% CO₂ humidified incubator.

### Minimum inhibitory concentration (MIC) determination

MIC was determined by broth microdilution. An overnight culture of each pathogen was diluted (OD₆₀₀ = 0,001) and microorganisms were plated in 96-well plates in the presence of different concentrations of antibiotics, L-CTL or D-CTL. After 24 hours of incubation, the microorganism growth was assessed by optical density OD₆₀₀ using a Multiskan™ EX microplate spectrophotometer (Thermo Fisher Scientific). The MIC, defined as the lowest concentration of drug able to inhibit 100% of the inoculum, was determined from a modified Gompertz function as described in Lambert et al., 2000 (J Appl Microbiol, 88(5): 784-790). Experiments were performed with biological replicates.

### Cytotoxicity assays

The MMT [3-(4,5-dimethylthiazol-2-yl)-2,5 diphenyl tetrazolium bromide] assay was used to assess the cytotoxicity of D-CTL and L-CTL. Cells in their exponential phase of growth were seeded into a 96-well plate at 1X10⁶ cells/mL prior being treated with a tenfold serial dilution of L-CTL or D-CTL. After 72 hours incubation, MTT (Sigma-Aldrich) was added to each well at a final concentration of 0,25mg/mL. Cells were then incubated for an additional 2 hours at 37°C in a 5% CO₂ humidified incubator and lysed with isopropanol/HCl (96:4, v/v). Cell cytotoxicity was then assessed by optical density OD₅₇₀ using a Multiskan™ EX microplate spectrophotometer (Thermo Fisher Scientific). Experiments were performed with biological replicates.

### Cytokine release assays

The following cytokines IL-1b, IL-2, IL-4, IL-7, IL-8, IL-10, IL12p70, IL-13, IL-17A, G-CSF, IFNg, MCP-1, MIP1b, TNFa were measured using the Bio-Plex® Multiplex Immunoassay system (Bio-Rad). In brief, human PBMCs were treated for 24 hours with 60µg/ml D-CTL, 60µg/ml L-CTL, or 5µg/ml LPS (LPS-EK, InvivoGen). Supernatants were then filtered and assessed for cytokine dosage according to the manufacturer's instructions.

### Resistance acquisition assays

*E. coli* (ATCC® 25922™) strain was sequentially passaged every day in the presence of the different antibacterial compounds: D-CTL, ampicillin or cefotaxime at ½ MIC during 28 days. The changes in the MICs were determined as previously described by broth microdilution at the indicated times.

### Peptide stability assays towards diverse bacterial virulence factors using HPLC

A preculture of each bacterial strain was prepared as follows: a single colony of each strain was incubated in its appropriate media (see above) for 24 hours at 37°C. Bacteria were then centrifuged for 1min at 13000rpm and the supernatant filtered using a 0,2 µm Millex filter (Merck-Millipore) to remove any residual bacteria. 400µl of supernatant was then incubated with or without each peptide of interest at 37°C for 24 hours. As a control, each peptide was incubated in water at 37°C for 24 hours. Samples were then separated using a Dionex HPLC system (Ultimate 3000; Sunnyvale, CA USA) on a Nucleosil reverse-phase 300-5C18-column (46250 mm; particle size: 5 mm; porosity, 300Å) (Macherey Nagel, Hoerdt, France). Absorbance was monitored at 214 nm and the solvent system consisted of 0.1% (v/v) TFA in water (solvent A) and 0.09% (v/v) TFA in 70% (v/v) acetonitrile-water (solvent B). Elution was performed at a flow rate of 700 mL/min with a gradient of solvent B as indicated on the chromatograms.

### Peptide stability assays in saliva using LC-SRM

All samples were centrifuged at 14 000 g during 5 min at +4°C and then diluted 3 times in water with 0.1 % (v/v) HCOOH. For each sample, 2 µL were injected on the LC-SRM system. All separations were carried out on Agilent 1100 Series HPLC system (Agilent Technologies). For each analysis, the sample was loaded into a trapping column ZORBAX 300SB-C18 MicroBore Guard 5 µm, 1.0 x 17 mm (Agilent Technologies) at 50 µL/min with aqueous solution containing 0.1 % (v/v) HCOOH and 2 % CH₃CN. After 3 min trapping, the column was put on-line with a ZORBAX 300SB-C18 3.5 µm, 0.3 x 150 mm column (Agilent Technologies). Peptide elution was performed at 5 µL/min by applying a linear gradient of solvent A (water with 0.1 % (v/v) HCOOH) and B (CH₃CN with 0.1 % (v/v) HCOOH), from 40 to 95 % solvent B over 5 min followed by a washing step (3 min at 95 % solvent B) and an equilibration step (13 min at 40 % solvent B).

For LC-SRM assay, each peptide was targeted with either an oxidized or non-oxidized state [RSMRLSFRARGYGFR]. For each state, three different precursors corresponding to three different charged states (3+, 4+ and 5+) were followed. For each of the 6 precursors, 8 transitions were monitored (48 transitions in total) on a QQQ-6490 triple quadrupole mass spectrometer (Agilent technologies) in unscheduled mode and within a cycle time of 3 000 s. For each transition, the collision energy was experimentally optimized by testing 7 values (step of ± 3 V) centered on the reference value. The reference value was calculated using the equation given by the supplier. The isolation width for both Q1 and Q3 was set to 0.7 m/z unit. Mass data collected during LC-SRM were processed with the Skyline open-source software package 3.6.1. Area intensities of each precursor were manually checked.

### Planktonic E. coli suspensions for physicochemical analysis

The bacterial model used for the physicochemical analysis (AFM, infrared spectroscopy and epifluorescence microscopy) is *E. coli* 2147. Bacteria were cultured in Lysogeny Broth (Miller, Fluka) broth at 25 g/L (LB) or at 6.25 g/L (LB/4) in deionized water (Purelab Option, ELGA). All the cultures were performed in a water bath shaker (Inova 3100, New Brunswick Scientific) at 37 ± 1°C and under continuous agitation at 160 rpm. After an overnight sub-culture (16 hours, with ampicillin + kanamycin), bacteria were cultivated in 200 mL of LB medium (without antibiotics) in 500 mL Erlenmeyer flasks with an initial optical density at 600 nm (OD₆₀₀, measured with a cell density meter Biochrom AG, Fisherbrand) of 0.050 ± 0.005.

The antimicrobial assays against planktonic *E.coli* E2146 were performed in sterile 96-well plates (Nunc) in a final volume of 200 µL. When the optical density of the bacterial culture reached an OD₆₀₀ value between 0.5 and 0.6 (bacteria were at the end of the exponential phase), the suspension was diluted in LB or LB/4 to give an OD₆₀₀ = 0.10 ± 0.01. The necessary volume of the stock solution of the peptide at 1 g/L was spotted in the bacterial suspension. Sterility and growth controls were sterile LB and LB/4, and a bacterial suspension without peptide, respectively. The plate was incubated for 24 h at 22°C.

### Epifluorescence optical microscopy

Planktonic bacteria were analyzed by fluorescence microscopy using the *Bac*Light™ stain kit (L7012, Molecular Probes, Eugene, USA) in order to determine the permeability of the sessile cells in the absence and presence of the AMP. This kit contains two nucleic acids stains: SYTO 9 (excitation/emission maxima: 480/500 nm) that penetrates all the cells, and propidium iodide that penetrates only cells with damaged membranes (excitation/emission maxima: 490/635 nm). Therefore, bacteria with intact membranes fluoresce green, while bacteria with damaged membranes fluoresce red. After 24 hours of incubation, 200µL of the 24 h-old bacterial suspension were mixed with 300 µL of *Bac*Light™ solution (15 µL of the reconstructed Baclight solution as described by the pushaser in 300 µL of sterile water), and stained for 20 min in the dark at 22±1°C. The suspension was then filtrated with 0.2 µm black filters (Millipore, GTBP04700) and rinsed three times with sterile water to eliminate excess *Bac*Light™. The sample was mounted in *Bac*Light™ mounting oil as described by the instructions provided by the manufacturer. Both fluorescences were viewed simultaneously with the 100 x oil immersion objective of an Olympus BX51 microscope equipped with an Olympus XC50 camera.

### ATR-FTIR spectroscopy

ATR-FTIR spectra were recorded between 4000 and 800 cm⁻¹ on a Bruker Vertex 70v spectrometer equipped with a KBr beam splitter and a DTGS detector, and driven by the OPUS 7.5 software. The resolution of the single beam spectra was 4 cm⁻¹. A nine-reflection diamond ATR accessory (Durasamp1*I*R™, SensIR Technologies, incidence angle: 45°) was used for acquiring spectra. The number of bidirectional double-sided interferogram scans was 200, which corresponds to a 2 min accumulation. All interferograms were Fourier processed using the Mertz phase correction mode and a Blackman-Harris three-term apodization function. No ATR correction was performed. Measurements were performed at 21±1°C in an air-conditioned room. 50 µL of the bacterial suspensions in their culture media was put on the ATR crystal. Half of the suspension was centrifuged at 8000 rpm during 5 minutes, and it was used to remove the spectral background. Water vapor subtraction was performed when necessary.

### AFM mechanical properties measurements.

AFM experiments were carried out using a MFP3D-BIO instrument (Asylum Research Technology, Oxford Instruments Company, Mannheim, Germany). Silicon nitride cantilevers of conical shape were purchased from Asylum Research Technology (Olympus TR400 PSA, Mannheim, Germany). The spring constants of the cantilevers measured using the thermal noise method were found to be 0.02-0.03 nN/nm. Experiments were performed in PBS at room temperature. The nanoindentation method was used to determine the Young's modulus from the force vs. indentation curves. Mechanical properties were obtained by recording a grid map of 50-by-50 force curves on several bacterial clusters containing at least 10 bacteria electrostatically immobilized onto PEI coated glass substrate. The maximal loading force was 4 nN, the piezodrive was fixed to 2 µm and the approach rate was 2 µm/s. The histograms corresponding to the statistic distribution of the Young modulus were estimated from the analysis of the approach curves according to the Sneddon model (Sneddon IN, 1965; Gavara N., 2012) where δ is the indentation depth, ν the Poisson coefficient, *R* is the curvature radius of AFM-tip apex and *f_{BECC}* the bottom effect correction described by Gavara et Chadwick (Gavara N., 2012). All the FVI were analysed by mean of an automatic Matlab algorithm described elsewhere (Polyakov P., 2011). Bacteria were then exposed to various L-CTL concentrations (8 µg/mL, 150 µg/mL and 750 µg/mL) and also to various D-CTL concentrations (8 µg/mL, 40 µg/mL and 80 µg/mL) in PBS buffer at 22°C for 16 hours. Mechanical properties were measured by AFM in force mapping mode at indentation rate of 2µm/s and the average values correspond to at least 500 force curves taken from at least 10 bacteria. For bars labelled with * and ** the corresponding values were obtained after only 3 and 0.8h of peptide exposure, respectively. Noticed that beyond these exposure periods all bacteria were too much damaged and not enough numerous for relevant measurements because of their lysis.

### Results

### D-CTL is an efficient antibacterial agent

The inventors synthesized D-Cateslytin and compared its antibacterial efficiency with L-CTL. To this aim, a panel of Gram-negative versus Gram-positive bacteria, as well as facultative versus strict anaerobes, were used: *Escherischia coli* wild type and multi-resistant, *Staphylococcus aureus* Met^{S} (MSSA) and Met^{R} (MRSA), *Parvimonas micra, Prevotella intermedia* and *Fusobacterium nucleatum* (cf. Table 1). Depending on the bacterial species, the antibacterial Minimum Inhibitory Concentration (MIC) of D-CTL was 2 to 15 times lower than L-CTL and ranged between 8 and 24µg/ml (cf. Table 1 and Figure 6). D-CTL was specifically effective against *P. intermedia* with a MIC of 10µg/ml and *E*. *coli* with a MIC of 8µg/ml for *E. coli* wild type strain and 8.4µg/ml for the multi-resistant strain of *E. coli.* Interestingly, the antibiotic activity of D-CTL on wild-type *E. coli* strain was comparable to that of ampicillin and kanamycin and could therefore constitute an alternative treatment for *E. coli* infections (cf. Table 1 and Figure 9). Regarding the others species tested, the antibiotics of reference were having lower MICs than D-CTL (cf. Table 1 and Figure 7).

**Table 1: Comparison of the MICs of D-CTL, L-CTL and reference antibiotics on different bacteria strains**

| | | | **MIC (peptide)** | | **Antibiotic of reference** | |
|---|---|---|---|---|---|---|
| **Pathogens** | **Gram** | **Respiratory type** | **L-CTL (µg/mL)** | **D-CTL (µg/mL)** | **Name** | **MIC (µg/mL)** |
| ***Escherichia coli*** | - | Facultative anaerobe | 75 | 8 | Ampicillin Kanamycin | 7.0 21.6 |
| ***Escherichia coli Amp^{R} Kan^{R}Chlo^{R}*** | + | Facultative anaerobe | 150 | 8,4 | Cefotaxim | 0.09 |
| ***Staphylococcus aureus (MSSA)*** | + | Facultative anaerobe | 40 | 24 | Methicillin | 1.2 |
| ***Staphylococcus aureus (MRSA)*** | + | Facultative anaerobe | 37 | 18 | Vancomycin | 0.8 |
| ***Parvimonas micra*** | + | Obligate anaerobe | 120 | 23 | Amoxicillin | 0.45 |
| ***Prevotella intermedia*** | - | Obligate anaerobe | 148.6 | 10 | Amoxicillin | 0.5 |
| ***Fusobacterium nucleatum*** | - | Obligate anaerobe | 125.3 | 22.4 | Amoxicillin | 0.6 |

### D-CTL is an antibiotic potentiator for numerous currently used antibiotics

The inventors then investigated whether D-CTL could potentiate the antibacterial effect of several antibiotics of reference, specifically methicillin (MET) and vancomycin (VCN) extensively prescribed to treat *S. aureus* infections, amoxicillin (AMX) recommended in numerous infections including periodontal infections and cefotaxime (CFT) often used as second intention treatment against *E. coli* resistant strains. To this aim, they determined the Fractional Inhibitory Concentration Index (FICI) of the combination between D-CTL and each antibiotic of reference. The FICI consists of the sum of the FICs of both antibacterial agents: FICI = FIC_{antibiotic} + FIC_{D-CTL}. For each compound, the FIC was determined as the ratio between the MIC of the compound in combination (MIC_{combination}) and the MIC of the compound acting alone (MICₐₗₒₙₑ). On the basis of their FICI, each combination was categorized as synergistic (≤0.5), additive (>0.5 to 1), indifferent (>1 to <2) or antagonistic (≥4) (European committee on antimicrobial susceptibility testing). Interestingly, a synergistic effect between cefotaxime and D-CTL was observed for the multidrug resistant *E. coli* Amp^{R} Kan^{R} but also between amoxicillin and D-CTL for *P. micra* and *P. intermedia* (cf. Table 2 and figure 8). Moreover, an additive effect was depicted for vancomycin and D-CTL on MSSA but also for amoxicillin and D-CTL on *F. nucleatum* (cf. Table 2 and figure 8). Altogether, D-CTL acts as a robust potentiator for the main antibiotics currently prescribed in clinic.

**Table 2: FICIs of combinations of D-CTL and reference antibiotics on different bacteria strains**

| **Pathogens** | **Combination** | **FICI** | **Effect** |
|---|---|---|---|
| ***Escherichia coli Amp^{R} Kan^{R}Chlo^{R}*** | D-CTL Cefotaxim | 0.3 | Synergistic |
| ***Staphylococcus aureus (MSSA)*** | D-CTL Methicillin | 0.75 | Additive |
| ***Staphylococcus aureus (MRSA)*** | D-CTL Vancomycin | 2.0 | Indifferent |
| ***Parvimonas micra*** | D-CTL Amoxicillin | 0.5 | Synergistic |
| ***Prevotella intermedia*** | D-CTL Amoxicillin | 0.5 | Synergistic |
| ***Fusobacterium nucleatum*** | D-CTL Amoxicillin | 0.75 | Additive |

### D-CTL, unlike ampicilin and cefotaxime, does not trigger resistance in E. coli

To assess whether D-CTL was able to trigger the development of resistance in a bacteria, the inventors cultured *E. coli* wild type strain in the presence of sub-MIC concentrations of D-CTL for 28 days. Interestingly, *E. coli* failed to generate mutants of resistance as its MIC remained stable for the whole duration of the culture (cf. Figure 1). In contrast, the MICs of ampicillin and cefotaxim, two antibiotics of reference used to treat *E*. *coli* infections, rapidly increase over the course of the culture to reach 3 times the MIC at day 28 (cf. Figure 1).

### D-CTL is not cytotoxic and does not elicit cytokine release

In order to investigate whether D-CTL would be a good lead compound for the development of a new antibiotic, the inventors assessed several safety issues such as haemolytic activity, cytotoxicity and immunogenicity through cytokine release.

For haemolytic assays, D-CTL was incubated with human erythrocytes at concentrations ranging from 0 to 100µg/ml. No cell lysis was observed at all concentrations, demonstrating that D-CTL is not haemolytic, even at concentrations higher than its MICs (cf. Figure 2A).

Cytotoxicity was also verified by MTT assays on human PBMCs (cf. Figure 2B), but also on Caco-2 cells (cf. Figure 2C), a human intestinal epithelial cell line. No cytotoxicity was detected for up to 100µg/ml on any of the cellular models tested after 72 hours of incubation.

In order to be used as an antibiotic, D-CTL should not trigger immunogenicity. To verify whether D-CTL influences the immune system, the inventors performed a cytokine release assay. Human PBMCs were treated with D-CTL and cytokines were quantified after 24h in the cell supernatant using the Bio-Plex® technology (Bio-Rad). As indicated in Figure 3A and 3B, no significant cytokine release was observed following D-CTL or L-CTL treatment. As a control, PBMCs treated with LPS in the same conditions resulted in a drastic increase in pro-inflammatory cytokines TNFa, G-CSF and IFNg but also the antiinflammatory cytokine IL-10 (cf. Figure 3C). These results indicate that D-CTL is not associated with cytokine release.

### D-CTL is more stable than L-CTL in body fluids and more resistant to degradation by bacterial virulence factors

One of the weaknesses of the use of peptide as a drug is their lack of stability in the body fluids but also towards the numerous proteases secreted by the pathogens. Therefore, the stability of D-CTL was also tested in saliva, a body fluid highly enriched in proteases using mass spectrometry. Remarkably, unlike L-CTL, D-CTL was stable in saliva for all the healthy volunteers tested (Figure 4).

The sensitivity to secreted virulence factors was assessed by HPLC after 24 hours incubation of D-CTL with different bacterial supernatants. As depicted in Figure 5, D-CTL was not degraded in either of the bacterial supernatants tested. However, L-CTL was degraded in the presence of the virulence factors of *E. coli* wild type strain (cf. Figures 5A and 5B) and multiresistant strain (cf. Figure 5C and 5D) but not *P. micra* (cf. Figure 5E and 5F), *F. nucleatum* (cf. Figure 5G and 5H), *P. intermedia* (cf. Figure 5I and 5J), MSSA (cf. Figure 5K) and MRSA (cf. Figure 5L). Therefore, the change in conformation between L-CTL and D-CTL did not modify their sensitivity towards bacterial virulence factors, except for *E. coli* for which the sensitivity was decreased.

### Example 2: Combination of D-Cateslytin peptide and calcium hydroxide eradicates root canal pathogens

### Material and Methods

### Antimicrobial agents

The following peptides were purchased from Proteogenix (Strasbourg): bovine chromofungin bCHR (bCgA₄₇₋₇₀: RILSILRHQNLLKELQDLAL), bovine catestatin bCAT (bCgA₃₄₄₋₃₆₄: RSMRLSFRARGYGFRGPGLQL), bovine L- cateslytin or bovine D-cateslytin: bL-CTL or bD-CTL (bCgA₃₄₄₋₃₅₈: RSMRLSFRARGYGFR).

### Preparation of Ca(OH)₂ solutions

Ca(OH)₂ was purchased from Sigma-Aldrich (USA). A saturated solution of Ca(OH)₂ was obtained by mixing 1.7mg of Ca(OH)₂ per milliliter of solution. The saturated solution was then diluted to one half (0.85mg/mL) or one quarter (0.425mg/mL).

### Microorganisms and mammalian cell line

*Fusobacterium nucleatum* (ATCC© 49256™), *Prevotella intermedia* (ATCC© 49046™), and *Parvimonas micra* (ATCC© 33270™) were purchased by ATCC (Manassas, USA). *Enterococcus faecalis* (CCM 2541) was obtained from the Czechoslovac Collection of Microorganisms (Czech Republic) Bacteria were cultured in Anaerobe Basal Broth (Oxoid, France) at 37°C in anaerobic conditions. *Candida albicans* (ATCC© 10231™) was cultured in Sabouraud medium (BD, France) supplemented with tetracycline (10µg/mL) and cefotaxime (10µg/mL).

The mammalian cell line HGF-1 (ATCC® CRL-2014™) was commercially obtained by ATCC and cultured at 37°C and 5% CO₂ in Dulbecco's Modified Eagle's Medium (Thermo Fisher Scientific, France) supplemented with 10% (v/v) bovine calf serum (Dutscher, France) and 1% (v/v) penicillin-streptomycin (Thermo Fisher Scientific, France).

### Broth dilution assays

An overnight culture of each pathogen was diluted (OD₆₀₀ₙₘ= 0.001) and incubated at 37°C in 96-plates in the presence of different concentrations of antimicrobial agents. When testing Ca(OH)₂, the pH of the solution was measured with by pH meter. (CyberScan pH 510©, Eutech, France). After 24h incubation, the OD₆₂₀ₙₘ was evaluated with a spectrophotometer (Multikan EX, ThermoScientific, France). Each assay was done at least in triplicate. For each set of assay, the standard deviation was determined.

### Determination of the Minimal Inhibitory Concentration (MIC)

When the peptide was efficient against the bacteria, the MIC, defined as the lowest concentration of peptide able to inhibit 100% of the inoculum, was calculated using a modified Gompertz model as described in Lambert et Pearson (Lambert and Pearson 2000).

### Cytotoxicity assays

The cytotoxicity of the antimicrobial agents was examined by MTT [3(4,5-diméthylthiazol-2-yl)-2,5 diphenyl tetrazolium bromide] assays (Scientific Life,, France) against HGF-1 cells. Cells were incubated for 24h in a 96-wells plate before being treated with various concentrations of Ca(OH)₂ alone or supplemented with the peptide. As a control, cells were plated in each 96-wells without being treated. After 24h, 48h and 72h incubation, cells were carefully washed with PBS and treated with MTT at a final concentration of 0.25mg/mL. HGF-1 cells were then incubated for 2h at 37°C before being lysed with isopropanol/HCL (48:2, v/v). Cell viability was assessed by reading the OD₅₄₀ₙₘ with a Multiskan EX microplate spectrophotometer (Thermo Fisher Scientific, France). The OD₅₄₀ₙₘ of each well was then compared to the OD₅₁₄ₙₘ of the control. For each set of experiments, standard deviations were determined.

### Stability assays

The stability of D-CTL was assessed either in the supernatant of *E. faecalis* or in Ca(OH)₂. The supernatant of *E. faecalis* was prepared as follows: a single colony of the bacteria was suspended in 5mL of Anaerobe Basal Broth and incubated at 37°C overnight. The culture was then centrifuged at 10000g for 1min and filtered (0.2µm). The supernatant was then incubated with D-CTL at a final concentration of 300µg/mL 37°C for 24h. A negative control was obtained after incubation of the supernatant without D-CTL in similar conditions.

Regarding the stability of D-CTL in Ca(OH)₂, the peptide was incubated in a buffered Ca(OH)₂ solution at 37°C for 24h. The pH of the Ca(OH)₂ solution was buffered at the same pH than the one determined during the broth dilution assays. As negative controls, D-CTL in water was also incubated at 37°C for 24h.

The samples were analyzed by HPLC (Dionex, Ultimate 3000, CA USA) using a Nucleosil reverse-phase 300-5C18-colum (4,6x250mm; particle size: 5µm; porosity, 300Å) (Macherey Nagel, France). Two solvents used were: 0.1% (v/v) TFA in water (Solvent A) and 0.09% (v/v) TFA in 70% (v/v) acetonitrile-water (solvent B). Absorbance was measured at 214nm (A₂₁₄ₙₘ). The flow rate for elution was 0,7mL/min with a gradient of solvent B as indicated on the chromatograms.

### Statistical analysis

Each assay was done in triplicate. For each set of assay, standard deviations were determined.

### Results

### A saturated solution of Ca(OH)₂ does not inhibit E. faecalis growth

The efficiency of a saturated solution of Ca(OH)₂ (1.7mg/mL, pH 9) was assessed by microdilution assays on *E. faecalis,* but also *F. nucleatum* as a control. These results confirmed previous studies showing that a saturated solution of Ca(OH)₂ was unable to completely inhibit the growth of *E. faecalis.* In our hands, the inhibition rate was only 58% (± 5%). In addition, the inventors confirmed that a saturated solution of Ca(OH)₂ was able to inhibit 100% (± 1%) of the growth of *F. nucleatum* (Figure 9).

### D-CTL displays antimicrobial properties against E. faecalis

The antibacterial efficiency of several CgA-derived peptides was then tested against *E*. *faecalis* by broth dilution assays: chromofungin (CHR), catestatin (CAT) and cateslytin (L-CTL or D-CTL). All peptides were tested at 200µg/mL. All natural peptides derived from CgA (CHR, CAT and L-CTL) displayed no antimicrobial activity against *E. faecalis.* The only peptide able to achieve 96% ± 4% bacterial growth inhibition at 200µg/mL was D-CTL (Figure 10A). D-CTL was therefore chosen as the best candidate to combine with Ca(OH)₂. Its MCB on *E. faecalis,* determined by broth dilution assays, was 156µg/mL (Figure 10B). The MIC was 156µg/mL.

### D-CTL is stable in a saturated solution of Ca(OH)₂

In order to appreciate the feasibility of the combination, the inventors tested the stability of D-CTL in a saturated solution of Ca(OH)₂ (1.7mg/mL, pH 9) by HPLC (Figure 11A). D-CTL was incubated at the MIC in a saturated solution Ca(OH)₂. Under these experimental conditions, D-CTL was eluted after 38min (Figure 11A, chromatogram 1). This peak was clearly identified in a saturated solution of Ca(OH)₂ at the same retention time (Figure 11A, chromatogram 2). In conclusion, these data confirmed the stability of D-CTL, at the MIC, in a saturated solution of Ca(OH)₂.

### D-CTL remains stable in the supernatant of E. faecalis

*E. faecalis* can overcome the innate immune system response and trigger persistent infections. One of its mechanisms of resistance is the degradation of antimicrobial peptides (Schmidtchen et al. 2002). In order to remain efficient towards an endodontic infection, D-CTL should therefore not be degraded by the proteases secreted by *E. faecalis.* To this aim, the stability of the D-CTL in the supernatant of *E. faecalis* was assessed by HPLC (Figure 11B). In the experimental conditions, D-CTL was eluted at 38min (Figure 13B, chromatogram 1). This peak was still present when D-CTL was incubated with the bacterial supernatant (Figure 11B, chromatogram 2). Of note, the other peaks of the chromatogram correspond to the proteins of the media but also the proteases secreted by the bacteria (Figure 11B, chromatogram 3). In conclusion, D-CTL is not degraded by the virulence factor of *E. faecalis,* allowing a prolonged action against this pathogen.

### Ca(OH)₂ at high concentration is cytotoxic for HGF-1

The cytotoxicity of a saturated solution of Ca(OH)₂ (1.7mg/mL), but also diluted solutions (0.85mg/mL and 0.425mg/mL) was assessed by MTT assays on human gingival fibroblasts (HGF-1) during 72h. Cell viability was assessed and expressed as a percentage of the control. At 0.85g/L and 0.425g/L, Ca(OH)₂ showed a toxicity below 4% (±7%) even after three days incubation meanwhile a saturated solution of Ca(OH)₂ showed a toxicity of 20% (±7%) after 24h and 14% (±11%) and 50% (±6%), respectively after 72h incubation (Figure 12). Of interest, in a previous study D-CTL showed no toxicity towards HGF-1 even at high concentrations (100µg/mL). According to these results, 0.85mg/mL or 0.425mg/mL of Ca(OH)₂ constitute a better choice than a saturated solution in the quest of a new efficient endodontic treatment.

### Combination between D-CTL and Ca(OH)₂ inhibits the main endodontic pathogens

In order to identify the most efficient combination of Ca(OH)₂ and D-CTL against several endodontic pathogens, microdilution assays were performed with different concentrations of Ca(OH)₂ and D-CTL against *E. faecalis.* Specifically, a saturated solution of Ca(OH)₂ (1.7mg/mL, pH 8.5) as well as diluted solutions of Ca(OH)₂ (0.85mg/mL and 0.425mg/mL) were combined with the MIC, ½ MIC and ¼ MIC of D-CTL (Figure 13A). As depicted, the combination using the lowest concentration of Ca(OH)₂ and D-CTL able to kill an inoculum of *E. faecalis* was a solution of Ca(OH)₂ 0.85mg/mL with ½ MIC of D-CTL.
The stability of that combination was confirmed by HPLC (Figure 13B). D-CTL eluted at 38 min (Figure 13B, chromatogram 1) and was stable in a buffered solution of Ca(OH)₂ (0,85g/L pH 8,5) (Figure 13B, chromatogram 2).

The cytotoxicity of the combination was also assessed (Ca(OH)₂ 0.85g/L and ½ MIC of D-CTL) towards HGF-1 by MTT assays. (Figure 13C). The combination showed a mild toxicity (80% ± 10% of cell viability) over 72 hours.

Finally, the inventors verified that the combination was also efficient on other endodontic pathogens. To this aim, microdilution assays were performed using *Parvimonas micra, Prevotella intermedia, Fusobacterium nucleatum* and *Candida albicans* (Figure 13D). Results show that Ca(OH)₂ (0.85mg/mL) was able to inhibit 87%±2% of C. *albicans* growth; 15%±6% of *P. micra* growth, 77%±6% of *P. intermedia* growth and 30%±10% of *F. nucleatum* whereas the combination inhibited respectively 97%±1%, 98±2%, 98±3 and 97%±5% of the pathogens growth. Therefore the combination of Ca(OH)₂ is not only efficient against *E. faecalis,* but also C. *albicans, P. micra, P. intermedia* and *F. nucleatum.*

### Discussion

Several combinational therapies have been developed between Ca(OH)₂ and other intracanal medications such as chlorhexidine (Gomes et al., 2003, Int Endod J., 36(4):267-275; Saatchi et al., 2014, J Appl Oral Sci., 22(5):356-365) or liquorice (Badr et al., 2011, Int Endod J., 44(1):51-58) to eradicate microorganisms from the root canal including *E. faecalis.* Chlorhexidine is an endodontic irrigant well known for its antimicrobial properties, particularly against *E. faeacalis.* Even tough chlorhexidine used as an irrigant is very effective against endodontic pathogens, clinical trials failed to prove its effectiveness as an intracanalar medication *in vivo* (Malkhassian et al., 2009, J Endod., 35(11):1483-1490; Plaquette et al., 2007, J Endod., 33(7):788-795). Moreover, a recent review concluded that mixing Ca(OH)₂ with chlorhexidine does not have a synergistic or additive effect (Saatchi et al., 2014, J Appl Oral Sci., 22(5):356-365). Similarly, liquorice extract alone or mixed with Ca(OH)₂ had a better antimicrobial effect than Ca(OH)₂ alone against *E. faecalis,* but its antimicrobial properties were not increased in combination ((Badr et al., 2011, Int Endod J., 44(1):51-58). This study provides encouraging results as the combination of Ca(OH)₂ 0.85mg/mL and ½ MIC of D-CTL was able to inhibit *E. faecalis* growth.

The absence of cytotoxicity is also an important characteristic for an intracanal dressing. Here, neither Ca(OH)₂ 0.85mg/mL alone, nor its combination with D-CTL was toxic for human gingival fibroblasts. This result is in accordance with a previous study that demonstrated that Ca(OH)₂ was not toxic for human dental pulp cells (Labban et al., 2014, Dent Traumatol.30(6):429-434). In addition, another study conducted with human periodontal ligament fibroblasts, also concluded that the effect of Ca(OH)₂ on cell viability and cytokine expression was minimal (Yadlapati et al., 2014, Int Endod J. 47(8):769-775). Finally, Ruparel et al demonstrated that Ca(OH)₂ (1mg/mL) promoted survival of stem cells of the apical papilla (Ruparel et al., 2012, J Endod., 38(10):1372-1375). This body evidence seems to confirm the mild cytotoxicity of Ca(OH)₂ at low concentrations.

The stability of the peptide in Ca(OH)₂ was also an unavoidable condition to allow the combination of both antimicrobial agents. Our results confirmed the high stability of D-CTL, as it was not degraded in a buffered solution of Ca(OH)₂ (pH 9). Furthermore, the peptide showed great stability in the bacterial supernatant of *E. faecalis.* This result contrasts with LL-37, a HDP known for its antimicrobial effectiveness, which was completely degraded by the virulence factors of *E. faecalis* (Schmidtchen et al., 2002, Mol Microbiol., 46(1):157-168). An interesting finding of this study is that this combination is efficient in a buffered environment. Several studies found that the buffering effect of the dentin, maintaining the pH in the tubules around 8 even in the presence of Ca(OH)₂, might explain its low efficiency in an extracted tooth model (Haapasalo et al., 2000, Int Endod J., 33(2):126-131).

Even though *E. faecalis* is strongly correlated with endodontic failure, several other pathogens are involved in endodontic infection. Our results showed that the combination of D-CTL and Ca(OH)₂ is efficient against four other endodontic pathogens: *Parvimonas micra, Prevotella intermedia, Fusobacterium nucleatum* and *Candida albicans.* These results demonstrate that combining Ca(OH)₂ with D-CTL is able to eradicate the endodontic biofilm.

### Example 3: D-Cateslytin peptide, a new antifungal agent

### Material and Methods

### Peptide synthesis

Bovine L-CTL (bCGA₃₄₄₋₃₅₈, RSMRLSFRARGYGFR) and its derivate D-CTL, as well as the rhodamined peptides Rho-L-CTL and Rho-D-CTL were synthesized purified to >95% by Proteogenix SAS. The rhodamine added to the peptides used for the fluorescence imaging study (L-CTL and D-CTL) is located on the N-terminal end of the polypeptidic chain.

### Antifungal tests

*Candida albicans* (ATCC© 10231™ distributed by LGC Standards, Molsheim, France) was cultured in Sabouraud medium (Sigma-Aldrich) supplemented with tetracycline (10 µg/mL) and cefotaxime (10 µg/mL) at 37°C for 24 h. *Candida albicans* (OD₆₀₀ₙₘ=0,001) were plated in 96-well plates and treated either with different concentrations of the peptides of interest, and/or with different concentrations of voriconazole (VCZ) (Sigma-Aldrich). As a positive control, cells were treated with 10 µg/mL VCZ. After 24 h incubation, yeast growth was assessed by optical density OD₆₀₀nm using a spectrophotometer (Multiscan EX, Shanghai, China).

### Minimal fungicidal concentration (MFC)

The MFC is defined as the lowest concentration of peptide able to inhibit 100% of yeasts, and determined using a modified Gompertz model as described in Lambert et Pearson (Lambert and Pearson 2000, J Appl Microbiol, 88(5): 784-790).

### Cytotoxicity assays

Peptide cytotoxicity was assessed by a MMT [3-(4,5-dimethylthiazol-2-yl)-2,5 diphenyl tetrazolium bromide] assay (Sigma-Aldrich, Lyon, France) using a human gingival fibroblasts (HGF-1) cell line (ATCC® CRL-2014™ distributed by LGC Standards, Molsheim, France) as a model. HGF-1 cells were maintained in DMEM (Sigma-Aldrich, Lyon, France) supplemented with 10% foetal bovine serum (Dutscher, Brumath, France) and 1% penicillin/streptomycin (Sigma-Aldrich, Lyon, France) at 37°C and 5% CO2.

HGF-1 cells (10⁶ cellules/mL) were plated in 96-well plates for 24 h, prior being treated with different concentrations of the peptides of interest for 24, 48 or 72 h. The culture media was then removed and replaced by MTT (Sigma-Aldrich, Lyon, France) diluted in culture media (0.25 mg/mL). Cells were then incubated for an additional 3 h at 37°C, 5% CO₂ and lysed by isopropanol/HCl (v/v). After 15 min incubation at room temperature under agitation, cell toxicity was finally assessed by optical density OD₅₅₀ₙₘ using a Multiskan™ EX microplate spectrophotometer (Thermo Fisher Scientific).

### Time-lapse videomicroscopy

Diluted precultures (1/100, 1/1000) of *Candida albicans* were incubated for 1 hour at 37°C without agitation in µ-Dish^{35mm,high} glass bottom (Ibidi) previously treated with poly-L-lysine. The rhodamined peptides (Rho-L-CTL or Rho-D-CTL) were then directly added to the culture at a concentration of 2-10× MFC, and incubated for 10-30 min at 37°C under minimum agitation. To perform time-lapse videomicroscopy, the culture was replaced by Sabouraud medium alone. Yeasts were replated at 1×10⁴ per cm² on filmcoated coverslips and mounted in a Ludin Chamber (Life Imaging Services, Basel, Switzerland) at 37°C, 5% CO₂, on a Nikon Eclipse Ti inverted microscope equipped with an Andor Zyla 3-tap sCMOS camera and a 60x objective. Images (fluorescence and phase contrast) were captured every 10 min for 20h using Nikon NIS-Elements AR software, and processed with ImageJ.

### Peptide stability assays

*Candida albicans* supernatant aliquots were prepared as follows: for each aliquot, a single colony of *Candida albicans* was resuspended in 5 mL of Sabouraud medium and incubated at 37°C overnight. The culture was then centrifuged at 10000 g for 1 min and the supernatant was filtered using a 0.22 µm MillexH-GV (Millipore, Carrigtwohill, Ireland). In order to check sterility, an aliquot of each supernatant was incubated at 37°C for 48 h. Absence of growth was interpreted as lack of viable microorganism.

100 µL of supernatant was then directly incubated with or without each peptide of interest (19 µg; 10 µL) at 37°C for 24 h. As a control, each peptide (19 µg; 10 µL) was incubated in water (100 µL) at 37°C for 24 h. Samples were then separated using a Dionex HPLC system (Ultimate 3000; Sunnyvale, CA USA) on a Nucleosil reverse-phase 300-5C18-column (4.6x250 mm; particle size: 5 µm; porosity, 300 Å) (Macherey Nagel, Hoerdt, France). Absorbance was monitored at 214 nm and the solvent system consisted of 0.1% (v/v) TFA in water (solvent A) and 0.09% (v/v) TFA in 70% (v/v) acetonitrile-water (solvent B). Elution was performed at a flow rate of 700 µL/min with a gradient of solvent B as indicated on the chromatograms. Each peak was manually collected.

### Fractional inhibitory concentration (FIC) index

The efficiency of the combination D-CTL/VCZ was determined by the calculation of the Fractional inhibitory concentration (FIC) index according to the following formula: FICI = FIC _{D-CTL}+ FIC vcz. According to EUCAST (*European Committee on Antimicrobial Suscpetibility Testing*), the effect of the combination is synergistic if FICI ≤ 0,5, additive if 0,5 < FICI ≤ 1, indifferent if 1 < FICI < 2, antagonistic if FICI ≥ 2⁴⁰⁻⁴¹.

### Results

### D-CTL inhibits the groth of C. albicans

To test the inhibitory potential of D-CTL on *Candida albicans* growth, antifungal assays were conducted with different concentrations of D-CTL and compared with L-CTL assays at the same concentration. L-CTL was able to kill 100% of *Candida albicans* at concentrations ranging from 1 µg/mL to 10 µg/mL. Results show that D-CTL displays a better inhibition activity than L-CTL. Indeed, D-CTL has a minimal fungicidal concentration (MFC) of 5,5 µg/mL (2,9 µM) (cf. Figure 14B), compared to a MFC of 7,9 µg/mL (4,2 µM) for L-CTL (cf. Figure 14A).

Time-lapse videomicroscopy was then used to examine the interaction between *Candida albicans* and L-CTL or D-CTL (cf. Figures 15-16). Two types of colonies of *Candida albicans* were observed: colonies totally invaded by the rhodamined peptide (Rho-L-CTL or Rho-D-CTL) (cf. Figures 15A-B) and colonies partially invaded by the peptide (cf. Figures 16A-B). Among 46 colonies observed for Rho-L-CTL, 34 of them were totally invaded, 12 of them were partially invaded. Among 39 colonies observed for Rho-D-CTL, 23 of them were totally invaded, 16 of them were partially invaded.

All 34 colonies totally invaded by Rho-L-CTL show a reduction of their fluorescence over time starting from 5h (cf. Figure 15A), in contrary to all 23 colonies totally invaded by Rho-D-CTL, that show a stability of their fluorescence over time until 17h (cf. Figure 15B). Moreover, the images recorded for colonies totally invaded by the rhodamined peptide (Rho-L-CTL: 34 colonies, Rho-D-CTL: 23 colonies) reveal a systematic arrest of fungal growth and ongoing cellular divisions (cf. Figures 15A-B).

Among 12 colonies partially invaded by Rho-L-CTL, all of them grew from the non-invaded cell. Among 16 colonies partially invaded by Rho-D-CTL, 6 of them revealed that the division of invaded cell induce the passage of the rhodamined peptide in the nascent cell (Figure 16A). The other 10 colonies partially invaded by Rho-D-CTL grew by division of the non-invaded cell (cf. Figure 16B).

Of interest is to note the absence of fluorescence on the septum separating two yeasts (cf. Figures 16-16).

Time-lapse videomicroscopy was also used to examine 11 colonies of *Candida albicans* alone, without any previous treatment. In each case, a growth progressing from every cell of the colony was observed (cf. Figure 16C).

Altoghether, these results attest the ability of D-CTL to inhibit the growth of C. *albicans.*

### D-CTL is not toxic for human gingival fibroblasts

To assess the cytotoxicity of both peptides, MTT assays were performed using human gingival fibroblasts (HGF-1) as a cellular model. Thus, each peptide was incubated with the cells at different concentrations for 24 hours, 48 hours and 72 hours (cf. Figure 14C-D). As expected for a host defense peptide, L-CTL was not toxic at 100 µg/ml on a period of time ranging from 24 to 72 hours (cf. Figure 14C). Interestingly, D-CTL was not cytotoxic either on HGF-1 after 72 hours for concentrations up to 100 µg/ml (cf. Figure 14D). As a result, neither L-CTL nor D-CTL showed cytotoxicity at their respective minimal inhibitory concentration.

### In contrast to L-CTL, D-CTL is stable in the supernatant of Candida albicans

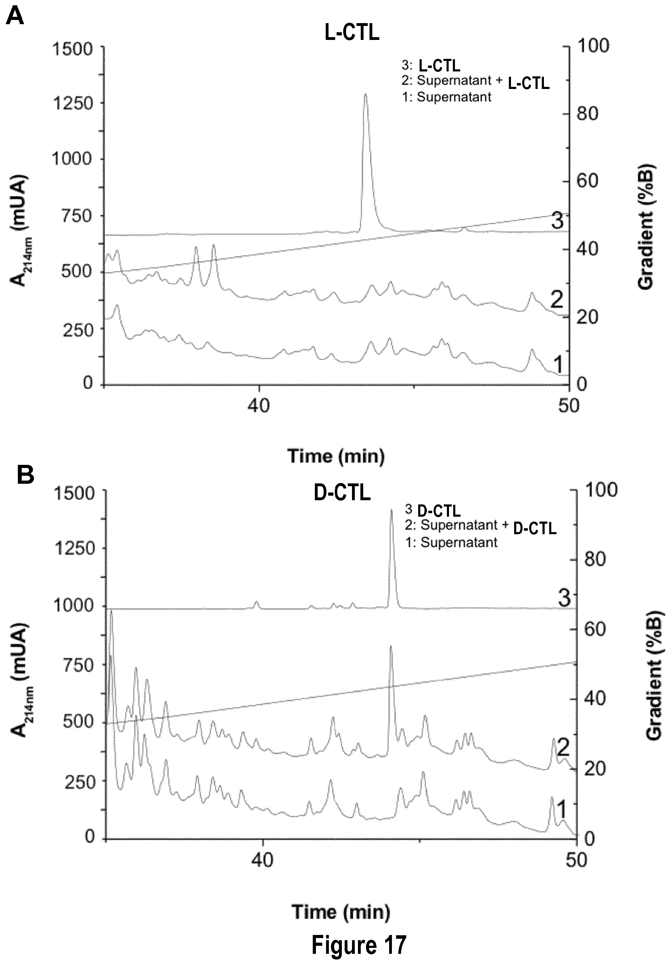

In order to use L-CTL or D-CTL as therapeutic agents, these peptides should not be degraded by virulence factors released by *Candida albicans.* Subsequently, the inventors tested whether L-CTL and D-CTL were stable in the supernatant of *Candida albicans* by HPLC. To this aim, L-CTL or D-CTL were incubated with the supernatant of *Candida albicans* for 24 hours at 37°C prior being analysed by HPLC (cf. Figure 17). As a control, the peptides and supernatant were incubated separately.

Under experimental conditions, L-CTL and D-CTL had a retention time of 44 min (cf. Figure 17A and 17B, chromatograms 3). In addition, the profiles obtained for the supernatant of *Candida albicans* displayed numerous peaks corresponding to the peptides and proteins secreted by the pathogen (cf. Figures 17A and 17B, chromatograms 1). When L-CTL was incubated with *Candida albicans* supernatant, the peak of L-CTL disappeared from the chromatogram, suggesting that L-CTL was degraded by components within the supernatant (cf. Figure 17A, chromatogram 2). However, D-CTL was still present after an incubation of 24h, implying that it remains stable in the supernatant of *Candida albicans* (Figure 17B, chromatogram 2).

### Combination of D-CTL and voriconazole (VCZ), the antifungal agent of reference to treat Candida albicans-related infections

To compare the efficiency of D-CTL with voriconazole (VCZ) against *Candida albicans,* antifungal assays were performed with decreasing concentrations of VCZ. The MFC of VCZ on *Candida albicans* was 0.07 µg/mL (cf. Figure 18A).

As VCZ was still more efficient than D-CTL, the inventors combined both antifungal agents to see whether it would be possible to decrease the administrated doses of VCZ (cf. Figure 18B). The combination using the least amount of antifungal agents able to kill 100% of *Candida albicans* was (1/2 MFC_{D}-_{CTL} + 1/4 MFCvcz).
According to EUCAST (*European Committee on Antimicrobial Suscpetibility Testing*)*,* FIC (VCZ) = 0.25; FIC (D-CTL) = 0.5; FICI = 0.75, which indicates an additive effect of the combination on *Candida albicans.*

Consequently, D-CTL potentiates VCZ, the antifungal of reference for C. *albicans,* and consequently allows to reduce the doses of VCZ commonly prescribed to fight a fungal infection and therefore prevent the emergence of fungal resistance.

### Discussion

In the dental field, the most common pathology involving a fungal biofilm is oral candidosis. Its prevalence is particularly strong in the increased risk populations such as elderly patients, diabetic or immunodepressed. By 2060, in France, a third of the population will be aged over 60 years. These epidemiological data confirm that oral candidosis will certainly be a growing concern for the coming years.

Oral candidosis manifests itself in an inflammatory process of the oral mucosa, due to the colonization by a fungal biofilm of tooth surface and oral mucosa. *Candida albicans* is described as the main agent causing oral candidosis.

The most important aspect of treatment is improving oral hygiene. The other aspect of treatment involves resolution of the mucosal infection, for which topical antifungal medications (as an oral suspension, or a gel) are used. Systemic antifungal drugs are almost exclusively reserved for patients with systemic factors that condition the development and persistence of oral candidosis, such as immunosuppression or diabetes.

However, increased resistance phenomena to antifungal agents commonly prescribed motivate the search for new ways of fighting against oral candidosis. All in all, the development of new molecules alternatives to conventional antifungal agents, such as peptides, is urgent to prevent the emergence of new phenomena of resistance.

In this context, the results obtained show that D-CTL displays a better therapeutic index than L-CTL, and potentiates antifungal agents of reference, suggesting that it constitutes a new potent antifungal agent active against *Candida albicans.* To validate the therapeutic potential of D-CTL over L-CTL, the inventors verified whether these peptides were not degraded by the factors of virulence secreted by *Candida albicans.* Indeed, as a mechanism of defence against the host, *Candida albicans* is able to release virulence factors such as the mannoprotein Mp65, the Seoul imipenemase Sim1 and the secreted aspartic protease Sap6. Only D-CTL remained stable when incubated with the supernatant of

### Candida albicans.

Moreover, time-lapse videomicroscopy revealed a quick invasion of both peptides within *Candida albicans,* suggesting that the pathogen is able to recognize both configurations of CTL.

Even though all images recorded suggested the absence of lysis of yeasts invaded by a peptide, a variability of the fluorescence inside yeasts was observed between L-CTL and D-CTL. Indeed, results showed a stability of the fluorescence inside yeasts invaded by the rhodamined peptide D-CTL that also seems to be able to spread from one yeast to another, therefore stopping the fungal growth and ongoing celluar divisions. This last observation was based on the condition that the ongoing division starts from a cell colonized by the fluorescent peptide.

On the other hand, the decreasing fluorescence observed for yeast formations colonized by L-CTL suggest a lack of stability of L-CTL inside the yeast.

Moreover time-lapse videomicroscopy showed a notable difference between the growth of a colony of *Candida albicans* alone, without any previous treatment, and the growth of a colony partially invaded by a rhodamined peptide. Indeed, among 11 colonies of *Candida albicans* alone, a growth progressing from every cell of the colony was observed in each case. On the other hand the images of colonies partially invaded by a rhodamined peptide show an arrest of the fungal growth at the extremity invaded by the peptide. Actually the fungal growth progresses from the yeast non-invaded by the peptide.

Altogether, the data obtained suggest that D-CTL could constitute an excellent candidate for the development of new antifungal agents against *Candida albicans.* The antifungal protection of the mucosa is an indication of the first order, as a possible prevention of oral fungal infections. A topical application (as a suspension or a gel) of such an antifungal agent, with minor resistance, therefore constitutes an interesting pathway for the protection of oral mucosa.

## Claims

1. A cateslytin peptide having an amino acid sequence consisting or consisting essentially in the sequence of SEQ ID NO: 1, wherein at least 60%, preferably at least 80%, of the amino acids residues of said cateslytin are D-configured.

2. The cateslytin peptide according to claim 1, wherein all the amino acids residues of said cateslytin are D-configured.

3. The cateslytin peptide according to claim 1 or 2 for use as a drug.

4. A pharmaceutical composition comprising a cateslytin peptide according to claim 1 or 2.

5. The pharmaceutical composition according to claim 4, wherein the pharmaceutical composition further comprises an additionnal active ingredient selected from the group consisting in an antibiotic, an antifungal, an antiviral, an antiparasitic and a combination thereof.

6. A product or kit comprising a) a cateslytin peptide according to claim 1 or 2 and b) an active ingredient selected from the group consisting in an antibiotic, an antifungal, an antiviral, an antiparasitic and a combination thereof, as a combined preparation for simultaneous, separate or sequential use.

7. The cateslytin peptide according to claim 1 or 2, the pharmaceutical composition according to claim 4 or 5, or the product or kit according to claim 6 for use in the treatment of an infection, preferably an infection selected from the group consisting in bacterial, viral, parasite, and fungal infections, even more preferably a bacterial infection.

8. The cateslytin peptide for use according to claim 7, the pharmaceutical composition for use according to claim 7, or the product or kit for use according to claim 7, wherein the infection is selected from the group consisting in fibrosis, meningitis, skin infections such as acne, intestinal infections such as esophagitis, gastritis, enteritis, colitis, sigmoiditis, rectitis, and peritonitis, urinary tract infections, vaginal infections, female upper genital tract infections such as salpingitis, endometritis, oophoritis, myometritis, parametritis and infection in the pelvic peritoneum, respiratory tract infections such as pneumonia, intra-amniotic infections, odontogenic infections, endodontic infections, bloodstream infections, or a combination thereof.

9. The cateslytin peptide for use according to claim 7 or 8, the pharmaceutical composition for use according to claim 7 or 8, or the product or kit for use according to claim 7 or 8, wherein the infection is a nosocomial infection, preferably a nosocomial infection selected from the group consisting in catheter-related infections, hospital acquired pneumonia such as ventilator associated pneumonia, post-partum infection, hospital acquired gastroenteritis, hospital acquired urinary tract infections, or a combination thereof, even more preferably the nosocomial infection is a catheter-related infection.

10. The cateslytin peptide for use according to anyone of claims 7 to 9, the pharmaceutical composition for use according to anyone of claims 7 to 9, or the product or kit for use according to anyone of claims 7 to 9, wherein the infection is a fungal infection, preferably a fungal infection selected from the group consisting in aspergillosis, blastomycosis, candidiasis, coccidioidomycosis, cryptococcosis, histoplasmosis, ucormycosis, paracoccidioidomycosis, sporotrichosis, pneumocystis, and a mixture thereof, more preferably the fungal infection is a candidiasis, even more preferably an oral candidiasis.

11. The cateslytin peptide for use according to claim 10, the pharmaceutical composition for use according to claim 10, or the product or kit for use according to claim 10, wherein the fungal infection is caused by a pathogen selected from the group consisting in *Candida* species such as *Candida albicans, Candida parapsilosis, Candida tropicalis, Candida krusei, Candida guillermondii, Candida rugosa, Candida dubliniensis, Candida auris, Candida glabrata, Candida lusitaniae, Candida kefyr, Candida famata, Candida inconspicua,* and *Candida norvegensis; Aspergillus species* such as *Aspergillus fumigatus, Aspergillus clavatus,* and *Aspergillus flavus; Cryptococcus* species such as *Cryptococcus neoformans* and *Cryptococcus gattii; Histoplasma* species such as *Histoplasma capsulatum; Pneumocystis* species such as *Pneumocystis jirovecii* and *Pneumocystis carinii; Stachybotrys* species such as *Stachybotrys chartarum; Blastomyces* species such as *Blastomyces dermatitidis; Coccidioides* species such as *Coccidioides immitis and Coccidioides posadasii; Mucorales* species such as *Rhizopus oryzae, Rhizomucor, Absidia, Lichtheimia corymbifera, Syncephalastrum racemosum, Apophysomyces variabilis* and *Mucor indicus; Paracoccidioides* species such as *Paracoccidioides brasiliensis; Sporothrix* species such as *Sporothrix schenckii; Epidermophyton* species such as *Epidermophyton floccosum; Microsporum* species such as *Microsporum canis* and *Microsporum audouinii; Trichophyton* species such as *Trichophyton interdigitale* (or *mentagrophytes*), *Trichophyton tonsurans, Trichophyton schoenleini, Trichophyton rubrum,* and *Trichophyton verrucosum; Hortaea* species such as *Hortaea werneckii; Penicillium* species such as *Penicillium marneffei; Piedraia* species such as *Penicillium hortae; Malassezia* species such as *Malassezia furfur; Lacazia* species such as *Lacazia loboi; Exophiala* species such as *Exophiala jeanselmei; Fonsecaea* species such as *Fonsecaea pedrosoi* and *Fonsecaea compacta; Phialophora* species such as *Phialophora verrucosa; Basidiobolus* species such as *Basidiobolus ranarum; Conidiobolus* species such as *Conidiobolus coronatus* and *Conidiobolus incongruus; Enterocytozoon* species such as *Enterocytozoon bieneusi* and *Encephalitozoon intestinalis; Rhinosporidium* species such as *Rhinosporidium seeberi; Geotrichum* species such as *Geotrichum candidum ; Pseudallescheria* species such as *Pseudallescheria boydii; Trichosporon* species; *Torulopsis glabrata,* or a mixture thereof, preferably the fungal infection is caused by a *Candida species* selected from the group consisting in *Candida albicans, Candida parapsilosis, Candida tropicalis, Candida krusei, Candida guillermondii, Candida rugosa, Candida dubliniensis, Candida auris, Candida glabrata, Candida lusitaniae, Candida kefyr, Candida famata, Candida inconspicua,* and *Candida norvegensis;* even more preferably the fungal infection is caused by *Candida albicans.*

12. The cateslytin peptide for use according to anyone of claims 7 to 9, the pharmaceutical composition for use according to anyone of claims 7 to 9, or the product or kit for use according to anyone of claims 7 to 9, wherein the infection is a bacterial infection, preferably a bacterial infection caused by a bacteria selected from the group consisting in *Escherichia coli, Escherichia coli Amp^{R} Kan^{R} Chlo^{R}, Escherichia coli AmpC, Escherichia coli ESBL, Escherichia coli OXA48, Staphylococcus aureus MSSA, Staphylococcus aureus MRSA, Klebsiella pneumoniae, Klebsiella pneumoniae ESBL, Klebsiella pneumoniae KPC, Enterobacter cloacae, Enterobacter cloacae ESBL, Enterobacter cloacae AmpC, Enterobacter cloacae OXA48, Enterobacter aerogenes, Enterobacter aerogenes ESBL, Enterobacter aerogenes AmpC, Serratia marcescens, Serratia marcescens AmpC, Morganella morganii, Morganella morganii AmpC, Citrobacter freundii, Citrobacter freundii AmpC, Pseudomonas aerigunosa, Pseudomonas aerigunosa AmpC, Pseudomonas aerigunosa VIM, Parvimonas micra, Prevotella intermedia, Fusobacterium nucleatum, Enterococcus faecalis, Prevotella nigrescens, Actinomyces israelii, Porphyromonas endodontalis, Porphyromonas gingivalis Micrococcus luteus, Bacillus megaterium, Actinomyces israelii, Aeromonas hydrophila, Aeromonas caviae, Bacillus anthracis, Bacillus cereus, Bacteroides fragilis, Bartonella henselae, Bartonella Quintana, Bordetella pertussis, Borrelia burgdorferi, Borrelia garinii, Borrelia afzelii, Borrelia recurrentis, Brucella abortus, Brucella canis, Brucella melitensis, Brucella suis, Campylobacter jejuni, Campylobacter coli, Campylobacter fetus, Chlamydia pneumoniae, Chlamydia trachomatis, Chlamydophila psittaci, Clostridium botulinum, Clostridium difficile, Clostridium perfringens, Clostridium tetani, Corynebacterium diphtheria, Ehrlichia canis, Ehrlichia chaffeensis, Enterococcus faecium, Francisella tularensis, Haemophilus influenza, Helicobacter pylori, Legionella pneumophila, Leptospira interrogans, Leptospira santarosai, Leptospira weilii, Leptospira noguchii, Listeria monocytogenes, Mycobacterium leprae, Mycobacterium tuberculosis, Mycobacterium ulcerans, Mycoplasma pneumonia, Neisseria gonorrhoeae, Neisseria meningitides, Nocardia asteroids, Rickettsia rickettsia, Salmonella enteritidis, Salmonella typhi, Salmonella paratyphi, Salmonella typhimurium, Shigella sonnei, Shigella flexnerii, Shigella dysenteriae, Staphylococcus epidermidis, Staphylococcus saprophyticus, Streptococcus agalactiae, Streptococcus pneumoniae, Streptococcus pyogenes, Streptococcus viridans, Treponema pallidum, Ureaplasma urealyticum, Vibrio cholera, Vibrio parahaemolyticus, Yersinia pestis, Yersinia enterocolitica, Yersinia pseudotuberculosis,* and combination thereof.

13. The cateslytin peptide for use according to claim 12, the pharmaceutical composition for use according to claim 12, or the product or kit for use according to claim 12, wherein the bacterial infection is caused by a bacteria selected from the group consisting in *Escherichia coli, Escherichia coli Amp^{R} Kan^{R} Chlo^{R}, Escherichia coli AmpC, Escherichia coli ESBL, Escherichia coli OXA48, Staphylococcus aureus MSSA, Staphylococcus aureus MRSA, Klebsiella pneumoniae, Klebsiella pneumoniae ESBL, Klebsiella pneumoniae KPC, Enterobacter cloacae, Enterobacter cloacae ESBL, Enterobacter cloacae AmpC, Enterobacter cloacae OXA48, Enterobacter aerogenes, Enterobacter aerogenes ESBL, Enterobacter aerogenes AmpC, Serratia marcescens, Serratia marcescens AmpC, Morganella morganii, Morganella morganii AmpC, Citrobacter freundii, Citrobacter freundii AmpC, Pseudomonas aerigunosa, Pseudomonas aerigunosa AmpC, Pseudomonas aerigunosa VIM, Parvimonas micra, Prevotella intermedia, Fusobacterium nucleatum, Enterococcus faecalis* and combination thereof, preferably the bacteria is selected from the group consisting in *Escherichia coli, Escherichia coli Amp^{R} Kan^{R} Chlo^{R}, MSSA Staphylococcus aureus, MRSA Staphylococcus aureus, Parvimonas micra, Prevotella intermedia, Fusobacterium nucleatum, Enterococcus faecalis,* and combination thereof, even more preferably the bacteria is *Escherichia coli or Escherichia coli Amp^{R} Kan^{R} Chlo^{R}.*

14. The pharmaceutical composition according to claim 5, the pharmaceutical composition for use according to anyone of claims 7 to 11, or the product or kit for use according to anyone of claims 6 to 11, wherein the antifungal is selected from the group consisting in polyenes such as amphotericin B, candicidin, filipin, hamycin, natamycin, nystatin and rimocidin; imidazoles such as bifonazole, butoconazole, clotrimazole, econazole, fenticonazole, isoconazole, ketoconazole, luliconazole, miconazole, omoconazole, oxiconazole, sertaconazole, sulconazole, tioconazole, and spectrazole; triazolles such as albaconazole, efinaconazole, epoxiconazole, fluconazole, isavuconazole, itraconazole, posaconazole, propiconazole, ravuconazole, terconazole, and voriconazole; thiazoles such as abafungin; allylamines such as amorolfin, butenafine, naftifine, and terbinafine; echinocandins such as anidulafungin, caspofungin, and micafungin; benzoic acid, ciclopirox, flucytosine, 5-fluorocytosine, griseofulvin, haloprogin, tolnaftate, undecylenic acid, derivatives and combinations thereof, preferably the antifungal is selected from the group consisting in triazolles such as albaconazole, efinaconazole, epoxiconazole, fluconazole, isavuconazole, itraconazole, posaconazole, propiconazole, ravuconazole, terconazole, voriconazole, or combination thereof, even more preferably the antifungal is the voriconazole.

15. The pharmaceutical composition according to claim 5, the pharmaceutical composition for use according to anyone of claims 7 to 9, 12 and 13, or the product or kit for use according to anyone of claims 6 to 9, 12 and 13 wherein the antibiotic is selected from the group consisting in penicillins such as penicillin G, penicillin K, penicillin N, penicillin O, penicillin V, methicillin, benzylpenicillin, nafcillin, oxacillin, cloxacillin, dicloxacillin, ampicillin, amoxicillin, pivampicillin, hetacillin, bacampicillin, metampicillin, talampicillin, epicillin, carbenicillin, ticarcillin, temocillin, mezlocillin, and piperacillin; cephalosporins such as cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefonicid, cefprozil, cefuroxime, cefuzonam, cefmetazole, cefotetan, cefoxitin, loracarbef, cefbuperazone, cefminox, cefotetan, cefoxitin, cefotiam, cefcapene, cefdaloxime, cefdinir, cefditoren, cefetamet, cefixime, cefmenoxime, cefodizime, cefotaxime, cefovecin, cefpimizole, cefpodoxime, cefteram, ceftamere, ceftibuten, ceftiofur, ceftiolene, ceftizoxime, ceftriaxone, cefoperazone, ceftazidime, latamoxef, cefclidine, cefepime, cefluprenam, cefoselis, cefozopran, cefpirome, cefquinome, flomoxef, ceftobiprole, ceftaroline, ceftolozane, cefaloram, cefaparole, cefcanel, cefedrolor, cefempidone, cefetrizole, cefivitril, cefmatilen, cefmepidium, cefoxazole, cefrotil, cefsumide, ceftioxide, cefuracetime, and nitrocefin; polymyxins such as polysporin, Neosporin,polymyxin B, and polymyxin E, rifampicins such as rifampicin, rifapentine, and rifaximin; Fidaxomicin; quinolones such as cinoxacin, nalidixic acid, oxolinic acid, piromidic acid, pipemidic acid, rosoxacin, ciprofloxacin, enoxacin, fleroxacin, lomefloxacin, nadifloxacin, norfloxacin, ofloxacin, pefloxacin, rufloxacin, balofloxacin, grepafloxacin, levofloxacin, pazufloxacin, temafloxacin, tosufloxacin, clinafloxacin, gatifloxacin, gemifloxacin, moxifloxacin, sitafloxacin, trovafloxacin, prulifloxacin, delafloxacin, nemonoxacin, and zabofloxacin; sulfonamides such as sulfafurazole, sulfacetamide, sulfadiazine, sulfadimidine, sulfafurazole, sulfisomidine, sulfadoxine, sulfamethoxazole, sulfamoxole, sulfanitran, sulfadimethoxine, sulfamethoxypyridazine, sulfametoxydiazine, sulfadoxine, sulfametopyrazine, and terephtyl; macrolides such as azithromycin, clarithromycin, erythromycin, fidaxomicin, telithromycin, carbomycin A, josamycin, kitasamycin, midecamycin, oleandomycin, solithromycin, spiramycin, troleandomycin, tylosin, and roxithromycin; ketolides such as telithromycin, and cethromycin; Iluoroketolides such as solithromycin; lincosamides such as lincomycin, clindamycin, and pirlimycin; tetracyclines such as demeclocycline, doxycycline, minocycline, oxytetracycline, and tetracycline; aminoglycosides such as amikacin, dibekacin, gentamicin, kanamycin, neomycin, netilmicin, sisomicin, tobramycin, paromomycin, and streptomycin; ansamycins such as geldanamycin, herbimycin, and rifaximin; carbacephems such as loracarbef; carbapenems such as ertapenem, doripenem, imipenem (or cilastatin), and meropenem; glycopeptides such as teicoplanin, vancomycin, telavancin, dalbavancin, and oritavancin; lincosamides such as clindamycin and lincomycin; lipopeptides such as daptomycin; monobactams such as aztreonam; nitrofurans such as furazolidone, and nitrofurantoin; oxazolidinones such as linezolid, posizolid, radezolid, and torezolid; teixobactin, clofazimine, dapsone, capreomycin, cycloserine, ethambutol, ethionamide, isoniazid, pyrazinamide, rifabutin, arsphenamine, chloramphenicol, fosfomycin, fusidic acid, metronidazole, mupirocin, platensimycin, quinupristin (or dalfopristin), thiamphenicol, tigecycline, tinidazole, trimethoprim, alatrofloxacin, fidaxomycin, nalidixice acide, rifampin, derivatives and combination thereof, preferably the antibiotic is selected from the group consisting in cefotaxim, methicillin, vancomycin, amoxicillin, derivatives and combination thereof.
